(19) Europäisches Patentamt / European Patent Office / Office européen des brevets

(11) EP 3 441 399 A1

(12) **EUROPEAN PATENT APPLICATION**

(43) Date of publication:
**13.02.2019 Bulletin 2019/07**

(21) Application number: **18194117.0**

(22) Date of filing: **13.06.2013**

(51) Int Cl.:
***C07K 11/02*** *(2006.01)* ***A01N 43/72*** *(2006.01)*
***A01P 5/00*** *(2006.01)* ***A01P 7/02*** *(2006.01)*
***A01P 7/04*** *(2006.01)* ***C07C 231/12*** *(2006.01)*
***C07C 235/12*** *(2006.01)* ***C07C 269/06*** *(2006.01)*
***C07C 271/22*** *(2006.01)* ***C07D 273/08*** *(2006.01)*
***C07B 51/00*** *(2006.01)* ***C07B 61/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.06.2012 JP 2012134304**

(62) Document number(s) of the earlier application(s) in accordance with Art. 76 EPC:
**13804000.1 / 2 862 872**

(71) Applicants:
- **Meiji Seika Pharma Co., Ltd.**
  **Tokyo 104-8002 (JP)**
- **The Kitasato Institute**
  **Tokyo 108-8641 (JP)**

(72) Inventors:
- **MITOMI, Masaaki**
  **Yokohama-shi, Kanagawa 222-8567 (JP)**
- **SAKAI, Masayo**
  **Yokohama-shi, Kanagawa 222-8567 (JP)**
- **HORIKOSHI, Ryo**
  **Yokohama-shi, Kanagawa 222-8567 (JP)**
- **ONOZAKI, Yasumichi**
  **Yokohama-shi, Kanagawa 222-8567 (JP)**
- **NAKAMURA, Satoshi**
  **Yokohama-shi, Kanagawa 222-8567 (JP)**
- **OMURA, Satoshi**
  **TOKYO, Tokyo 108-86421 (JP)**
- **SUNAZUKA, Toshiaki**
  **TOKYO, Tokyo 108-8641 (JP)**
- **HIROSE, Tomoyasu**
  **TOKYO, Tokyo 108-8641 (JP)**
- **SHIOMI, Kazuro**
  **TOKYO, Tokyo 108-8641 (JP)**
- **MASUMA, Rokuro**
  **TOKYO, Tokyo 108-8641 (JP)**

(74) Representative: **Regimbeau**
**20, rue de Chazelles**
**75847 Paris Cedex 17 (FR)**

Remarks:
This application was filed on 12-09-2018 as a divisional application to the application mentioned under INID code 62.

# (54) NOVEL CYCLIC DEPSIPEPTIDE DERIVATIVES AND HARMFUL ORGANISM CONTROL AGENTS COMPRISING THE SAME

(57) An objective of the present invention is to provide novel cyclic depsipeptide derivatives and harmful organism control agents including the same as each other. Specifically, the present invention provides compounds represented by formula (1) or stereoisomers thereof, harmful organism control agents containing them, and a process for producing them.

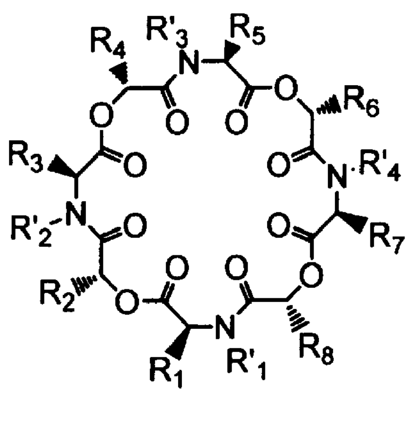

(1)

EP 3 441 399 A1

## Description

CROSS-REFERENCE TO RELATED APPLICATION

The application is based upon and claims the benefit of priority from the prior Japanese Patent Application No. 134304/2012, filed on June 13, 2012; the entire contents of which are incorporated herein by reference.

BACKGROUND OF THE INVENTION

Field of Invention

The present invention relates to novel cyclic depsipeptide derivatives and novel harmful organism control agents using the same.

Background Art

Many harmful organism control agents have hitherto been found. However, novel drugs have still been desired, for example, from the viewpoint of a problem of lowered drug sensitivity, persistence of effect, and safety in use.

Non-patent documents 1 and 2 report cyclic octadepsipeptides. These reports, however, do not disclose the compounds of the present invention and thus do not suggest the harmful organism control effect of the compounds of the present invention.

Non-patent documents 3, 4, and 5 disclose the structure of cyclic octadepsipeptides and derivatives thereof, and the insecticidal activity thereof against silkworms. These documents, however, do not disclose the compounds of the present invention and thus do not suggest the harmful organism control effect of the compounds of the present invention.

Patent documents 1, 2, and 3 disclose cyclic octadepsipeptides and their endoparasite control effect. These documents, however, do not disclose the compounds of the present invention and thus do not suggest harmful organism control effect of the compounds of the present invention.

Patent documents 4, 5, and 6 disclose cyclic octadepsipeptides as endoparasite control agents. These documents, however, do not disclose the compounds of the present invention and thus do not suggest harmful organism control effect of the compounds of the present invention.

Patent document 7 discloses FKI-1033 substances as cyclic octadepsipeptides and discloses inhibitory effect of ryanodine receptors. This document, however, does not disclose the compounds of the present invention and thus does not suggest harmful organism control effect of the compounds of the present invention.

Patent document 8 discloses cyclic octadepsipeptides and insecticidal activity thereof against silkworms, Spodoptera litura, and Bursaphelenchus xylophilus. This document, however, does not disclose the compounds of the present invention and thus does not suggest harmful organism control effect of the compounds of the present invention.

PRIOR ART DOCUMENTS

Patent Documents

Patent document 1: European Patent Application Laid-Open No. 0382173
Patent document 2: Japanese Patent Application Laid-Open No. 229997/1993
Patent document 3: WO 93/19053
Patent document 4: European Patent Application Laid-Open No. 0626376
Patent document 5: European Patent Application Laid-Open No. 0626375
Patent document 6: WO 2011/069995
Patent document 7: WO 2004/44214
Patent document 8: Japanese Patent Application Laid-Open No. 271013/1993

Non-patent documents

Non-patent document 1: Tetrahedron Letters (1963), 4(6), 351

Non-patent document 2: Tetrahedron Letters(1963), 4(14), 885
Non-patent document 3: Tetrahedron Letters(1977), 18(25), 2167
Non-patent document 4: Agricultural and Biological Chemistry (1978), 42(3), 629
Non-patent document 5: Peptide Chemistry (1978) 16th, 165

SUMMARY OF THE INVENTION

The present inventors have found novel cyclic depsipeptide derivatives and have also found that the cyclic depsipeptide derivatives have an excellent activity as harmful organism control agents. The present invention has been made based on such finding.

An object of the present invention is to provide novel harmful organism control agents.

Thus, the following inventions are provided.

(1) A compound represented by formula (1) or a stereoisomer thereof:

[Chemical formula 1]

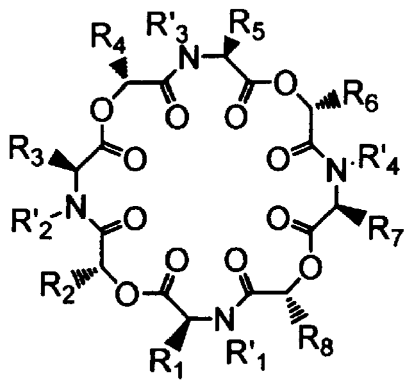

(1)

wherein

$R_1$, $R_2$, $R_3$, $R_4$, $R_5$, $R_6$, $R_7$, and $R_8$, which may be the same as or different from each other, represent a hydrogen atom, optionally substituted straight-chain, branched, or cyclic alkyl having 1 to 9 carbon atoms, or optionally substituted alkoxyalkyl having 2 to 9 carbon atoms and

$R'_1$, $R'_2$, $R'_3$, and $R'_4$, which may be the same as or different from each other, represent a hydrogen atom, methyl or ethyl, excluding compounds wherein $R_1$, $R_3$, $R_5$, and $R_7$ represent isopropyl or isobutyl when all of $R_2$, $R_4$, $R_6$, and $R_8$ represent isopropyl; compounds wherein all of $R_1$, $R_3$, $R_5$, and $R_7$ represent methyl when all of $R_2$, $R_4$, $R_6$, and $R_8$ represent n-pentyl; and compounds wherein all of $R_1$, $R_2$, $R_3$, $R_4$, $R_5$, $R_6$, $R_7$, and $R_8$ represent methyl.

(2) The compound according to (1) or a strereoisomer thereof, wherein $R_1$, $R_3$, $R_5$, and $R_7$, which may be the same as or different from each other, represent a hydrogen atom or optionally substituted straight-chain, branched, or cyclic alkyl having 1 to 7 carbon atoms and $R_2$, $R_4$, $R_6$, and $R_8$, which may be the same as or different from each other, represent straight-chain alkyl having 1 to 9 carbon atoms or branched or cyclic alkyl having 4 to 8 carbon atoms.

(3) The compound according to (1) or a stereoisomer thereof, wherein $R_1$, $R_3$, $R_5$, and $R_7$, which may be the same as or different from each other, represent a hydrogen atom or optionally substituted straight-chain or branched alkyl having 1 to 4 carbon atoms and $R_2$, $R_4$, $R_6$, and $R_8$, which may be the same as or different from each other, represent straight-chain alkyl having 4 to 9 carbon atoms.

(4) The compound according to (1) or a strereoisomer thereof, wherein $R_1$, $R_3$, $R_5$, and $R_7$ each represent a hydrogen atom or optionally substituted straight-chain, branched, or cyclic alkyl having 1 to 7 carbon atoms, provided that $R_1$, $R_3$, and $R_5$ are the same as each other while $R_7$ is different from $R_1$, $R_3$, and $R_5$; $R_2$, $R_4$, $R_6$, and $R_8$ each represent straight-chain, branched, or cyclic alkyl having 4 to 9 carbon atoms, provided that $R_2$, $R_4$, and $R_6$ are the same as

each other while $R_8$ may be the same as or different from $R_2$, $R_4$, and $R_6$; and $R'_1$, $R'_2$, $R'_3$, and $R'_4$ each represent a hydrogen atom, methyl or ethyl, provided that $R'_1$, $R'_2$, $R'_3$, and $R'_4$ are the same as each other,

$R_1$, $R_3$, $R_5$, and $R_7$ each represent a hydrogen atom or optionally substituted straight-chain, branched, or cyclic alkyl having 1 to 7 carbon atoms, provided that $R_1$, $R_3$, and $R_5$ are the same as each other while $R_7$ may be the same as or different from $R_1$, $R_3$, and $R_5$; $R_2$, $R_4$, $R_6$, and $R_8$ each represent straight-chain, branched, or cyclic alkyl having 4 to 9 carbon atoms, provided that $R_2$, $R_4$, and $R_6$ are the same as each other while $R_8$ is different from $R_2$, $R_4$, and $R_6$; and $R'_1$, $R'_2$, $R'_3$, and $R'_4$ each represent a hydrogen atom, methyl or ethyl, provided that $R'_1$, $R'_2$, $R'_3$, and $R'_4$ are the same as each other, or

$R_1$, $R_3$, $R_5$, and $R_7$ each represent a hydrogen atom or optionally substituted straight-chain, branched, or cyclic alkyl having 1 to 7 carbon atoms, provided that $R_1$, $R_3$, $R_5$, and $R_7$ are the same as each other; $R_2$, $R_4$, $R_6$, and $R_8$ each represent straight-chain, branched, or cyclic alkyl having 4 to 9 carbon atoms, provided that $R_2$, $R_4$, $R_6$, and $R_8$ are the same as each other; and $R'_1$, $R'_2$, $R'_3$, and $R'_4$ each represent a hydrogen atom, methyl or ethyl, provided that $R'_1$, $R'_2$, and $R'_3$ are the same as each other while $R'_4$ is different from $R'_1$, $R'_2$, and $R'_3$.

(5) The compound according to (1) or a stereoisomer thereof, wherein $R_1$, $R_3$, $R_5$, and $R_7$ each represent a hydrogen atom or straight-chain or branched alkyl having 1 to 4 carbon atoms, provided that $R_1$ and $R_3$ are the same as each other and $R_5$ and $R_7$ are the same as each other while $R_1$ and $R_3$ are different from $R_5$ and $R_7$; $R_2$, $R_4$, $R_6$, and $R_8$ each represent straight-chain alkyl having 4 to 8 carbon atoms, provided that $R_2$ and $R_4$ are the same as each other and $R_6$ and $R_8$ are the same as each other while $R_2$ and $R_4$ may be the same as or different from $R_6$ and $R_8$; and $R'_1$, $R'_2$, $R'_3$, and $R'_4$ each represent a hydrogen atom, methyl or ethyl, provided that $R'_1$ and $R'_2$ are the same as each other and $R'_3$ and $R'_4$ are the same as each other while $R'_1$ and $R'_2$ may be the same as or different from $R'_3$ and $R'_4$,

$R_1$, $R_3$, $R_5$, and $R_7$ each represent a hydrogen atom or straight-chain or branched alkyl having 1 to 4 carbon atoms, provided that $R_1$ and $R_3$ are the same as each other and $R_5$ and $R_7$ are the same as each other while $R_1$ and $R_3$ may be the same as or different from $R_5$ and $R_7$; $R_2$, $R_4$, $R_6$, and $R_8$ each represent straight-chain alkyl having 4 to 8 carbon atoms, provided that $R_2$ and $R_4$ are the same as each other and $R_6$ and $R_8$ are the same as each other while $R_2$ and $R_4$ are different from $R_6$ and $R_8$; and $R'_1$, $R'_2$, $R'_3$, and $R'_4$ each represent a hydrogen atom, methyl or ethyl, provided that $R'_1$ and $R'_2$ are the same as each other and $R'_3$ and $R'_4$ are the same as each other while $R'_1$ and $R'_2$ may be the same as or different from $R'_3$ and $R'_4$, or

$R_1$, $R_3$, $R_5$, and $R_7$ each represent a hydrogen atom or straight-chain or branched alkyl having 1 to 4 carbon atoms, provided that $R_1$ and $R_3$ are the same and $R_5$ and $R_7$ are the same while $R_1$ and $R_3$ may be the same as or different from $R_5$ and $R_7$; $R_2$, $R_4$, $R_6$, and $R_8$ each represent straight-chain alkyl having 4 to 8 carbon atoms, provided that $R_2$ and $R_4$ are the same and $R_6$ and $R_8$ are the same as each other while $R_2$ and $R_4$ may be the same as or different from $R_6$ and $R_8$; and $R'_1$, $R'_2$, $R'_3$, and $R'_4$ each represent a hydrogen atom, methyl or ethyl, provided that $R'_1$ and $R'_2$ are the same and $R'_3$ and $R'_4$ are the same as each other while $R'_1$ and $R'_2$ are different from $R'_3$ and $R'_4$.

(6) The compound according to (1) or a stereoisomer thereof, wherein $R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ are different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ may be the same as or different from $R_4$ and $R_8$; and $R'_1$, $R'_2$, $R'_3$, and $R'_4$ each represent a hydrogen atom, methyl or ethyl, provided that $R'_1$ and $R'_3$ are the same as each other and $R'_2$ and $R'_4$ are the same as each other while $R'_1$ and $R'_3$ may be the same as or different from $R'_2$ and $R'_4$,

$R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ are different from $R_4$ and $R_8$; and $R'_1$, $R'_2$, $R'_3$, and $R'_4$ each represent a hydrogen atom, methyl or ethyl, provided that $R'_1$ and $R'_3$ are the same as each other and $R'_2$ and $R'_4$ are the same as each other while $R'_1$ and $R'_3$ may be the same as or different from $R'_2$ and $R'_4$, or

$R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ may be the same as or different from $R_4$ and $R_8$; and $R'_1$, $R'_2$, $R'_3$, and $R'_4$ each represent a hydrogen atom, methyl or ethyl, provided that $R'_1$ and $R'_3$ are the same as each other and $R'_2$ and $R'_4$ are the same as each other while $R'_1$ and $R'_3$ are different from $R'_2$ and $R'_4$.

(7) The compound according to (1) or a stereoisomer thereof, wherein $R_1$, $R_3$, $R_5$, and $R_7$, which are the same as each other, represent a hydrogen atom or straight-chain or branched alkyl having 1 to 4 carbon atoms; $R_2$, $R_4$, $R_6$

and $R_8$, which are the same as each other, represent straight-chain alkyl having 1 to 9 carbon atoms; and $R'_1$, $R'_2$, $R'_3$, and $R'_4$, which are the same as each other, represent a hydrogen atom, methyl or ethyl.

(8) The compound according to (1) or a strereoisomer thereof, wherein $R_1$, $R_3$, $R_5$, and $R_7$ each represent a hydrogen atom or straight-chain, branched, or cyclic alkyl having 3 to 7 carbon atoms, provided that $R_1$ and $R_3$ are the same as each other, $R_5$ is different from $R_1$ and $R_3$, and $R_7$ is different from $R_1$, $R_3$, and $R_5$; and $R_2$, $R_4$, $R_6$, and $R_8$, which are the same as each other, represent straight-chain alkyl having 5 to 7 carbon atoms; and $R'_1$, $R'_2$, $R'_3$, and $R'_4$, which are the same as each other, represent methyl, or

$R_1$, $R_3$, $R_5$, and $R_7$ each represent a hydrogen atom or straight-chain, branched, or cyclic alkyl having 3 to 7 carbon atoms, provided that $R_1$ is different from $R_3$, $R_5$, and $R_7$ and $R_3$, $R_5$ and $R_7$ are the same as each other; $R_2$, $R_4$, $R_6$, and $R_8$ each represent straight-chain alkyl having 5 to 7 carbon atoms, provided that $R_4$ is different from $R_2$, $R_6$, and $R_8$ which are the same as each other, or $R_8$ is different from $R_2$, $R_4$, and $R_6$ which are the same as each other; and $R'_1$, $R'_2$, $R'_3$, and $R'_4$, which are the same as each other, represent methyl.

(9) The compound according to (1) or a strereoisomer thereof, wherein $R_1$, $R_3$, $R_5$, and $R_7$ each represent a hydrogen atom or straight-chain, branched, or cyclic alkyl having 1 to 7 carbon atoms, provided that $R_1$ and $R_5$ are the same as each other, $R_3$ is different from $R_1$ and $R_5$, and $R_7$ is different from $R_1$, $R_3$, $R_5$; $R_2$, $R_4$, $R_6$, and $R_8$, which are the same as each other, represent straight-chain alkyl having 5 to 7 carbon atoms; and $R'_1$, $R'_2$, $R'_3$, and $R'_4$, which are the same as each other, represent methyl, or

$R_1$, $R_3$, $R_5$, and $R_7$ each represent a hydrogen atom or straight-chain, branched, or cyclic alkyl having 1 to 7 carbon atoms, provided that $R_1$ is different from $R_3$, $R_5$, and $R_7$; $R_3$, $R_5$, and $R_7$ are the same as each other; $R_2$, $R_4$, $R_6$, and $R_8$ each represent straight-chain alkyl having 5 to 7 carbon atoms, provided that $R_6$ is different from $R_2$, $R_4$, and $R_8$ and $R_2$, $R_4$, and $R_8$ are the same as each other; and $R'_1$, $R'_2$, $R'_3$, and $R'_4$, which are the same as each other, represent methyl.

(10) The compound according to (8) or (9) or a strereoisomer thereof, wherein $R_1$ represents straight-chain, branched, or cyclic alkyl having 3 to 7 carbon atoms.

(11) An agricultural and horticultural harmful organism (pest) control agent, comprising at least one of compounds according to any one of (1) to (10) or stereoisomers thereof.

(12) A veterinary parasite control agent comprising at least one of compounds according to (4) or stereoisomers thereof.

(13) A method for controlling an agricultural and horticultural harmful organism, the method comprising applying an effective amount of a compound according to any one of (1) to (10) or a stereoisomer thereof to a harmful organism or a habitat thereof.

(14) A method for controlling a veterinary parasite, the method comprising applying an effective amount of a compound according to (4) or a stereoisomer to a veterinary parasite or a habitat thereof.

(15) Use of a compound according to any one of (1) to (10) or a stereoisomer thereof, for producing an agricultural and horticultural harmful organism control agent.

(16) Use of a compound according to (4) or a stereoisomer thereof, for producing a veterinary parasite control agent.

(17) A compound according to any one of (1) to (10) or a stereoisomer thereof, for use as an agricultural and horticultural harmful organism control agent.

(18) A compound according to (4) or a stereoisomer thereof, for use as a veterinary parasite control agent.

(19) A process for producing a compound represented by formula (1):

[Chemical formula 2]

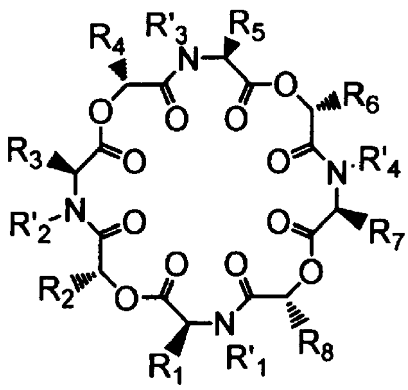

(1)

wherein $R_1$, $R_2$, $R_3$, $R_4$, $R_5$, $R_6$, $R_7$, $R_8$, $R'_1$, $R'_2$, $R'_3$, and $R'_4$ are as defined in (1), the process comprising: cyclizing a compound represented by formula (2):

[Chemical formula 3]

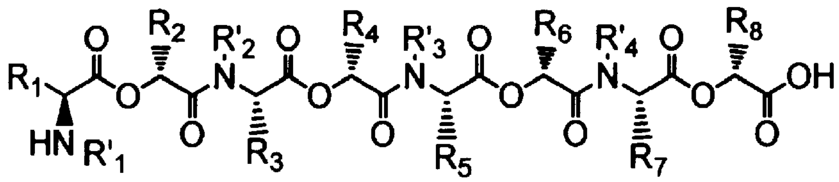

(2)

wherein $R_1$, $R_2$, $R_3$, $R_4$, $R_5$, $R_6$, $R_7$, $R_8$, $R'_1$, $R'_2$, $R'_3$, and $R'_4$ are as defined in (1).

(20) The process according to (19), which further comprises obtaining the compound of formula (2) by removing group X and group Y of a compound represented by formula (3):

[Chemical formula 4]

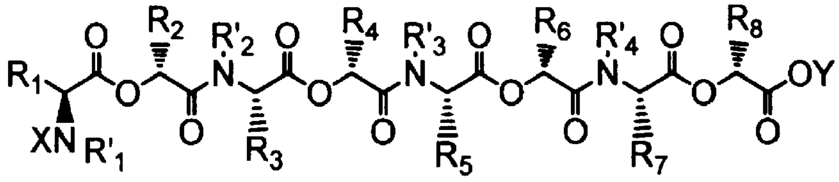

(3)

wherein $R_1$, $R_2$, $R_3$, $R_4$, $R_5$, $R_6$, $R_7$, $R_8$, $R'_1$, $R'_2$, $R'_3$, and $R'_4$ are as defined in (1); X represents an N-terminal protecting group selected from the group consisting of acetyl (Ac), allyloxycarbonyl (Alloc), benzyloxycarbonyl (CbZ), tert-butyloxycarbonyl (Boc), and 9-fluorenylmethyloxycarbonyl (Fmoc); and Y represents a C-terminal protecting group selected from the group consisting of benzyl (OBn), p-nitrobenzyl (ONb), and tert-butyl ($OBu^t$), or a support of a polymer with a selectively removable fixing group.

(21) The process according to (20), which further comprises obtaining a compound of formula (3) by reacting a compound represented by formula (4):

[Chemical formula 5]

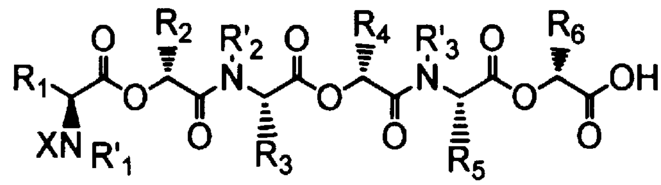

(4)

wherein $R_1$, $R_2$, $R_3$, $R_4$, $R_5$, $R_6$, $R'_1$, $R'_2$, $R'_3$, and X are as defined in (20), with a compound represented by formula (5):

[Chemical formula 6]

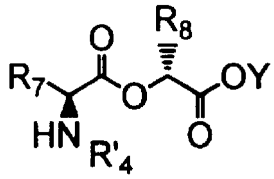

(5)

wherein $R_7$, $R_8$, $R'_4$, and Y are as defined in (20).

Derivatives of the present invention can effectively control Plutella xylostella, Spodoptera litura, Aphis gossypii, Aleyrodidae, Deltocephalidae, Pentatomidae, Thysanoptera, Tetranychidae, rust mites, and other harmful organisms. In particular, Tetranychidae can be advantageously effectively controlled even when Tetranychidae are resistant to drugs.

DETAILED DESCRIPTION OF THE INVENTION

The term "halogen atom" as used herein means an atom selected from fluoro, chloro, bromo, and iodo.

In compounds of formula (1), the "straight-chain, branched, or cyclic alkyl having 1 to 9 carbon atoms" represented by $R_1$, $R_2$, $R_3$, $R_4$, $R_5$, $R_6$, $R_7$, and $R_8$ means alkyl that may contain a straight chain, branched or cyclic structure having 1 to 9 carbon atoms. The Alkyl is saturated or unsaturated alkyl, that is, alkyl that may contain one or more unsaturated bonds. When the branched or cyclic structure is contained or when an unsaturated bond is contained, it is apparent that the number of carbon atoms is 3 or more. Specific examples thereof include methyl, ethyl, n-propyl, isopropyl, cyclopropyl, n-butyl, isobutyl, sec-butyl, cyclobutyl, n-pentyl, neopentyl, cyclopentyl, n-hexyl, 2-methylpentyl, n-heptyl, 2-methylhexyl, n-octyl, and n-nonyl.

Here the "optionally substituted" means that any carbon atom in straight-chain, branched, or cyclic alkyl having 1 to 9 carbon atoms may have 1 to 3, preferably one substituent, and examples of the "substituent" include halogen atoms, hydroxyl, cyano, and nitro.

In the compounds of formula (1), "alkoxyalkyl having 2 to 9 carbon atoms" represented by $R_1$, $R_2$, $R_3$, $R_4$, $R_5$, $R_6$, $R_7$, and $R_8$ means alkyl substituted by alkoxy, wherein the total number of carbon atoms in alkoxy moiety and alkyl moiety is 2 to 9. Examples of the "alkoxy" include methoxy, ethoxy, n-propoxy, i-propoxy, n-butoxy, i-butoxy, s-butoxy, t-butoxy, n-pentyloxy, neopentyloxy, i-pentyloxy, t-pentyloxy, n-hexyloxy, and i-hexyloxy. Specific examples thereof include methoxymethyl and ethoxymethyl.

Here the "optionally substituted" means that any carbon atom (preferably a carbon atom in alkoxy moiety) in alkoxyalkyl having 2 to 9 carbon atoms may have 1 to 3, preferably one substituent. Examples of the "substituent" include halogen atoms, cyano, and nitro.

In the compounds of formula (1), $R_1$, $R_3$, $R_5$, and $R_7$ are preferably a hydrogen atom or optionally substituted straight-chain, branched, or cyclic alkyl having 1 to 7 carbon atoms, more preferably a hydrogen atom or optionally substituted straight-chain or branched alkyl having 1 to 4 carbon atoms, still more preferably optionally substituted straight-chain or branched alkyl having 1 to 4 carbon atoms, and specific examples thereof include methyl, ethyl, n-propyl, isopropyl, n-butyl, and isobutyl.

In compounds of formula (1), $R_2$, $R_4$, $R_6$, and $R_8$ are preferably straight-chain alkyl having 1 to 9 carbon atoms or branched or cyclic alkyl having 4 to 8 carbon atoms, more preferably straight-chain alkyl having 4 to 9 carbon atoms or branched or cyclic alkyl having 4 to 8 carbon atoms, still more preferably straight-chain alkyl having 4 to 9 carbon atoms, even more preferably straight-chain alkyl having 4 to 8 carbon atoms, and specific examples thereof include n-butyl, n-pentyl, n-hexyl, n-heptyl, and n-octyl.

In compounds of formula (1), $R'_1$, $R'_2$, $R'_3$, and $R'_4$ are a hydrogen atom, methyl or ethyl, preferably methyl.

The compounds of the present invention are preferably compounds represented by formula (I) wherein $R_1$, $R_3$, $R_5$, and $R_7$, which may be the same as or different from each other, represent a hydrogen atom or optionally substituted straight-chain, branched, or cyclic alkyl having 1 to 7 carbon atoms; $R_2$, $R_4$, $R_6$, and $R_8$, which may be the same as or different from each other, represent straight-chain alkyl having 1 to 9 carbon atoms or branched or cyclic alkyl having 4 to 8 carbon atoms; $R'_1$, $R'_2$, $R'_3$, and $R'_4$, which may be the same as or different from each other, represent a hydrogen atom, methyl, or ethyl.

The compounds of the present invention are more preferably compounds represented by formula (I) wherein $R_1$, $R_3$, $R_5$, and $R_7$, which may be the same as or different from each other, represent a hydrogen atom or optionally substituted straight-chain, branched or cyclic alkyl having 1 to 7 carbon atoms; $R_2$, $R_4$, $R_6$, and $R_8$, which may be the same as or different from each other, represent straight-chain alkyl having 4 to 9 carbon atoms or branched or cyclic alkyl having 4 to 8 carbon atoms; $R'_1$, $R'_2$, $R'_3$, and $R'_4$, which may be the same as or different from each other, represent a hydrogen atom, methyl, or ethyl.

The compounds of the present invention are still more preferably compounds represented by formula (I) wherein $R_1$, $R_3$, $R_5$, and $R_7$, which may be the same as or different from each other, represent a hydrogen atom or optionally substituted straight-chain or branched alkyl having 1 to 4 carbon atoms; $R_2$, $R_4$, $R_6$, and $R_8$, which may be the same as or different from each other, represent straight-chain alkyl having 1 to 9 carbon atoms; $R'_1$, $R'_2$, $R'_3$, and $R'_4$, which may be the same as or different from each other, represent a hydrogen atom, methyl, or ethyl.

The compounds of the present invention are even more preferably compounds represented by formula (I) wherein $R_1$, $R_3$, $R_5$, and $R_7$, which may be the same as or different from each other, represent a hydrogen atom or optionally substituted straight-chain or branched alkyl having 1 to 4 carbon atoms; $R_2$, $R_4$, $R_6$, and $R_8$, which may be the same as or different from each other, represent straight-chain alkyl having 4 to 9 carbon atoms; $R'_1$, $R'_2$, $R'_3$, and $R'_4$, which may be the same as or different from each other, represent a hydrogen atom, methyl, or ethyl.

The compounds of the present invention are even more preferably compounds represented by formula (I) wherein $R_1$, $R_3$, $R_5$, and $R_7$, which may be the same as or different from each other, represent a hydrogen atom or optionally substituted straight-chain or branched alkyl having 1 to 4 carbon atoms; $R_2$, $R_4$, $R_6$, and $R_8$, which may be the same as or different from each other, represent straight-chain alkyl having 4 to 8 carbon atoms; $R'_1$, $R'_2$, $R'_3$, and $R'_4$, which may be the same as or different from each other, represent a hydrogen atom, methyl, or ethyl.

Compounds of formula (1) wherein the octadepsipeptide chain constituting the ring is of -A-A-A-B- type may be mentioned as compounds in a first embodiment of compounds of formula (I) (compounds in the first embodiment).

Specific examples thereof include compounds of formula (1) wherein $R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$, $R_3$, and $R_5$ are the same as each other while $R_7$ is different from $R_1$, $R_3$, and $R_5$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$, $R_4$, and $R_6$ are the same as each other while $R_8$ may be the same as or different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other; $R_1$, $R_3$, $R_5$, and $R_7$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_1$, $R_3$, and $R_5$ are the same as each other while $R_7$ may be the same as or different from $R_1$, $R_3$, and $R_5$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$, $R_4$, and $R_6$ are the same as each other while $R_8$ is different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other; or

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$, $R_3$, $R_5$, and $R_7$ are the same as each other; $R_2$, $R_4$, $R_6$, and $R_8$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_2$, $R_4$, $R_6$, and $R_8$ are the same as each other; and $R'_1$, $R'_2$, $R'_3$, and $R'_4$ each represent a hydrogen atom, methyl or ethyl, provided that $R'_1$, $R'_2$, and $R'_3$ are the same as each other while $R'_4$ is different from $R'_1$, $R'_2$, and $R'_3$.

Preferred examples thereof include compounds of formula (1) wherein $R_1$, $R_3$, $R_5$, and $R_7$ each represent a hydrogen atom or optionally substituted straight-chain, branched, or cyclic alkyl having 1 to 7 carbon atoms, provided

that $R_1$, $R_3$, and $R_5$ are the same as each other while $R_7$ is different from $R_1$, $R_3$, and $R_5$; $R_2$, $R_4$, $R_6$, and $R_8$ each represent straight-chain, branched, or cyclic alkyl having 4 to 9 carbon atoms, provided that $R_2$, $R_4$, and $R_6$ are the same as each other while $R_8$ may be the same as or different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other;

$R_1$, $R_3$, $R_5$, and $R_7$ each represent a hydrogen atom or optionally substituted straight-chain, branched, or cyclic alkyl having 1 to 7 carbon atoms, provided that $R_1$, $R_3$, and $R_5$ are the same as each other while $R_7$ is the same as or different from $R_1$, $R_3$, and $R_5$; $R_2$, $R_4$, $R_6$, and $R_8$ each represent, straight-chain, branched, or cyclic alkyl having 4 to 9 carbon atoms, provided that $R_2$, $R_4$, and $R_6$ are the same as each other while $R_8$ is different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other; or

$R_1$, $R_3$, $R_5$, and $R_7$ each represent a hydrogen atom or optionally substituted straight-chain, branched, or cyclic alkyl having 1 to 7 carbon atoms, provided that $R_1$, $R_3$, $R_5$, and $R_7$ are the same as each other; $R_2$, $R_4$, $R_6$, and $R_8$ each represent, straight-chain, branched, or cyclic alkyl having 4 to 9 carbon atoms, provided that $R_2$, $R_4$, $R_6$, and $R_8$ are the same as each other; and $R'_1$, $R'_2$, $R'_3$, and $R'_4$ each represent a hydrogen atom, methyl, or ethyl, provided that $R'_1$, $R'_2$, and $R'_3$ are the same as each other while $R'_4$ is different from $R'_1$, $R'_2$, and $R'_3$.

The compounds in the first embodiment are preferably compounds of formula (I) wherein $R_1$, $R_3$, and $R_5$ represent methyl, $R_2$, $R_4$, and $R_6$ represent n-pentyl, and $R'_1$, $R'_2$, $R'_3$, and $R'_4$ represent methyl; compounds of formula (I) wherein $R_1$, $R_3$, and $R_5$ represent a hydrogen atom, $R_2$, $R_4$, and $R_6$ represent n-pentyl, $R'_1$, $R'_2$, $R'_3$, and $R'_4$ represent methyl; compounds of formula (I) wherein $R_1$, $R_3$, and $R_5$ represent isobutyl, $R_2$, $R_4$, and $R_6$ represent n-pentyl, $R'_1$, $R'_2$, $R'_3$, and $R'_4$ represent methyl; or compounds of formula (I) wherein $R_1$, $R_3$, and $R_5$ represent n-propyl, $R_2$, $R_4$, and $R_6$ represent n-pentyl, $R'_1$, $R'_2$, $R'_3$, and $R'_4$ repersent methyl. In these compounds, preferably, $R_7$ represents a hydrogen atom, methyl, ethyl, n-propyl, hydroxy n-propyl, isopropyl, n-butyl, i-butyl, neopentyl, or cyclohexylmethyl, $R_8$ represents n-butyl, i-butyl, n-pentyl, n-hexyl, n-heptyl, n-octyl, n-nonyl, or cyclopropylmethyl.

The compounds in the first embodiment are more preferably compounds of formula (1) wherein $R_1$, $R_3$, and $R_5$ represent methyl, $R_2$, $R_4$, and $R_6$ represent n-pentyl, $R_7$ represents a hydrogen atom, ethyl, n-propyl, isopropyl, n-butyl, i-butyl, or hydroxy n-propyl, $R_8$ represents n-pentyl, n-hexyl, n-heptyl, n-octyl, or n-nonyl, $R'_1$, $R'_2$, $R'_3$, and $R'_4$ represent methyl; compounds of formula (1) wherein $R_1$, $R_3$, $R_5$, and $R_7$ represent methyl, $R_2$, $R_4$, and $R_6$ represent n-pentyl, $R_8$ represents n-butyl, isobutyl, n-hexyl, n-octyl, n-nonyl, or cyclopropylmethyl, $R'_1$, $R'_2$, $R'_3$, and $R'_4$ represent methyl; compounds of formula (1) wherein $R_1$, $R_3$, and $R_5$ represent methyl, $R_2$, $R_4$, and $R_6$ represent n-pentyl, $R_7$ represents a hydrogen atom, $R_8$ represents isobutyl or cyclopropylmethyl, $R'_1$, $R'_2$, $R'_3$, and $R'_4$ represent methyl; compounds of formula (1) wherein $R_1$, $R_3$, and $R_5$ represent methyl, $R_2$, $R_4$, and $R_6$ represent n-pentyl, $R_7$ represents i-butyl, $R_8$ represents isobutyl or cyclopropylmethyl, $R'_1$, $R'_2$, $R'_3$, and $R'_4$ represent methyl; compounds of formula (1) wherein $R_1$, $R_3$, and $R_5$ represent a hydrogen atom, $R_2$, $R_4$, and $R_6$ represent n-pentyl, $R_7$ represents isobutyl, $R_8$ represents n-pentyl, n-hexyl, n-heptyl, n-octyl, or n-nonyl, $R'_1$, $R'_2$, $R'_3$, and $R'_4$ represent methyl; compounds of formula (1) wherein $R_1$, $R_3$, and $R_5$ represent isobutyl, $R_2$, $R_4$, and $R_6$ represent n-pentyl, $R_7$ represents a hydrogen atom or n-propyl, $R_8$ represents n-pentyl, and $R'_1$, $R'_2$, $R'_3$, and $R'_4$ represent methyl; or compounds of formula (1) wherein $R_1$, $R_3$, and $R_5$ represent n-propyl, $R_2$, $R_4$, and $R_6$ represent n-pentyl, $R_7$ represents a hydrogen atom, isobutyl, neopentyl, or cyclohexylmethyl, $R_8$ represents n-pentyl, $R'_1$, $R'_2$, $R'_3$, and $R'_4$ represent methyl. The compounds in the first embodiment are more preferably compounds of formula (1) wherein $R_1$, $R_3$, $R_5$ and $R_7$ represent methyl, $R_2$, $R_4$, $R_6$, and $R_8$ represent n-pentyl, $R'_1$, $R'_2$, and $R'_3$ represent a hydrogen atom, and $R'_4$ represents methyl.

The compounds in the first embodiment are more preferably compounds of formula (1) wherein $R_1$, $R_3$, and $R_5$ represent methyl, $R_2$, $R_4$, and $R_6$ represent n-pentyl, $R_7$ represents a hydrogen atom, n-propyl, isopropyl, or n-butyl, $R_8$ represents n-pentyl, n-hexyl, or n-heptyl, and $R'_1$, $R'_2$, $R'_3$, and $R'_4$ represent methyl; compounds of formula (1) wherein $R_1$, $R_3$, and $R_5$ represent isobutyl, $R_2$, $R_4$, and $R_6$ represent n-pentyl, $R_7$ represents a hydrogen atom, $R_8$ represents n-pentyl, $R'_1$, $R'_2$, $R'_3$, and $R'_4$ represent methyl; or compounds of formula (1) wherein $R_1$, $R_3$, and $R_5$ represent n-propyl, $R_2$, $R_4$, and $R_6$ represent n-pentyl, $R_7$ represents isobutyl or neopentyl, $R_8$ represents n-pentyl, and $R'_1$, $R'_2$, $R'_3$, and $R'_4$ represent methyl.

Compounds of formula (1) wherein the octadepsipeptide chain constituting the ring is of -A-A-B-B- type may be mentioned as compounds in a second embodiment of the compounds of formula (1) (compounds in the second embodiment).

Specific compounds are compounds of formula (1) wherein $R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ and $R_3$ are the same as each other and $R_5$ and $R_7$ are the same as each other while $R_1$ and $R_3$ are different from $R_5$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_4$ and $R_6$ is the same as each other as $R_8$ while $R_2$ and $R_4$ may be the same as or different from $R_6$ and $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ may be the same as

or different from $R'_3$ and $R'_4$;

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is the same as $R_3$ and $R_5$ is the same as $R_7$ while $R_1$ and $R_3$ may be the same as or different from $R_5$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_4$ and $R_6$ is the same as $R_8$ while $R_2$ and $R_4$ are different from $R_6$ and $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ may be the same as or different from $R'_3$ and $R'_4$; or

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is the same as $R_3$ and $R_5$ is the same as $R_7$ while $R_1$ and $R_3$ may be the same as or different from $R_5$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_4$ and $R_6$ is the same as $R_8$ while $R_2$ and $R_4$ may be the same as or different from $R_6$ and $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ are different from $R'_3$ and $R'_4$.

Preferred compounds are compounds of formula (1) wherein $R_1$, $R_3$, $R_5$, and $R_7$ each represent a hydrogen atom or straight-chain or branched alkyl having 1 to 4 carbon atoms, provided that $R_1$ is the same as $R_3$ and $R_5$ is the same as $R_7$ while $R_1$ and $R_3$ are different from $R_5$ and $R_7$; $R_2$, $R_4$, $R_6$, and $R_8$ each represent straight-chain alkyl having 4 to 8 carbon atoms, provided that wherein $R_2$ is the same as $R_4$ and $R_6$ is the same as $R_8$ while $R_2$ may be the same as or different from $R_4$ and $R_6$ may be the same as or different from $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ may be the same as or different from $R'_3$ and $R'_4$;

$R_1$, $R_3$, $R_5$, and $R_7$ each represent a hydrogen atom or straight-chain or branched alkyl having 1 to 4 carbon atoms, provided that $R_1$ is the same as $R_3$ and $R_5$ is the same as $R_7$ while $R_1$ and $R_3$ may be the same as or different from $R_5$ and $R_7$; $R_2$, $R_4$, $R_6$, and $R_8$ each represent straight-chain alkyl having 4 to 8 carbon atoms, provided that $R_2$ is the same as $R_4$ and $R_6$ is the same as $R_8$ while $R_2$ and $R_4$ are different from $R_6$ and $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ may be the same as or different from $R'_3$ and $R'_4$; or

$R_1$, $R_3$, $R_5$, and $R_7$ each represent a hydrogen atom or straight-chain or branched alkyl having 1 to 4 carbon atoms, provided that $R_1$ is the same as $R_3$ and $R_5$ is the same as $R_7$ while $R_1$ and $R_3$ may be the same as or different from $R_5$ and $R_7$; $R_2$, $R_4$, $R_6$, and $R_8$ each represent straight-chain alkyl having 4 to 8 carbon atoms, provided that $R_2$ is the same as $R_4$ and $R_6$ is the same as $R_8$ while $R_2$ and $R_4$ may be the same as or different from $R_6$ and $R_8$; and $R'_1$, $R'_2$, $R'_3$, and $R'_4$ each represent a hydrogen atom, methyl, or ethyl, provided that $R'_1$ is the same as $R'_2$ and $R'_3$ is the same as $R'_4$ while $R'_1$ and $R'_2$ are different from $R'_3$ and $R'_4$.

The compounds in the second embodiment are preferably compounds of formula (1) wherein $R_1$ and $R_3$ represent a hydrogen atom or isobutyl, $R_5$ and $R_7$ represent a hydrogen atom, isobutyl, or n-propyl; and $R_2$ and $R_4$ and/or $R_6$ and $R_8$ represent n-pentyl or n-heptyl, provided that, when $R_1$ and $R_3$ are the same as $R_5$ and $R_7$, $R_2$ and $R_4$ are different from $R_6$ and $R_8$ while, when $R_1$ and $R_3$ are different from $R_5$ and $R_7$, $R_2$ and $R_4$ may be the same as or different from $R_6$ and $R_8$, and $R'_1$, $R'_2$, $R'_3$, and $R'_4$ represent methyl.

The compounds in the second embodiment are preferably compounds of formula (1) wherein $R_1$ and $R_3$ represent isobutyl, $R_5$ and $R_7$ represent a hydrogen atom, $R_2$, $R_4$, $R_6$, and $R_8$ represent n-pentyl, $R'_1$, $R'_2$, $R'_3$, and $R'_4$ represent methyl; compounds of formula (1) wherein $R_1$ and $R_3$ represent isobutyl, $R_5$ and $R_7$ represent n-propyl, $R_2$, $R_4$, $R_6$, and $R_8$ represent n-pentyl, $R'_1$, $R'_2$, $R'_3$, and $R'_4$ represent methyl; or compounds of formula (1) wherein $R_1$ and $R_3$ represent a hydrogen atom, $R_5$ and $R_7$ represent isobutyl, $R_2$ and $R_4$ represent n-pentyl, $R_6$ and $R_8$ represent n-heptyl, and $R'_1$, $R'_2$, $R'_3$, and $R'_4$ represent methyl.

The compounds in the second embodiment are more preferably compounds of formula (1) wherein $R_1$ and $R_3$ represent isobutyl, $R_5$ and $R_7$ represent a hydrogen atom or n-propyl, $R_2$, $R_4$, $R_6$, and $R_8$ represent n-pentyl, and $R'_1$, $R'_2$, $R'_3$, and $R'_4$ represent methyl.

Compounds of formula (1) wherein the octadepsipeptide chain constituting the ring is of -A-B-A-B- type may be mentioned as compounds in a third embodiment of the compounds of formula (1) (compounds in the third embodiment).

Specific compounds are compounds of formula (1) wherein $R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is the same as $R_5$ and $R_3$ is the same as $R_7$ while $R_1$ and $R_5$ are different from $R_3$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_6$ and $R_4$ is the same as $R_8$ while $R_2$ and $R_6$ may be the same as or different from $R_4$

and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ may be the same as or different from $R'_2$ and $R'_4$;

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is the same as $R_5$ and $R_3$ is the same as $R_7$ while $R_1$ and $R_5$ may be the same as or different from $R_3$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_6$ and $R_4$ is the same as $R_8$ while $R_2$ and $R_6$ are different from $R_4$ and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ may be the same as or different from $R'_2$ and $R'_4$; or

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is the same as $R_5$ and $R_3$ is the same as $R_7$ while $R_1$ and $R_5$ may be the same as or different from $R_3$ and $R_7$; $R_2$, $R_4$, $R_6$, and $R_8$ each represent a hydrogen atom, optionally substituted straight-chain, branched, or cyclic alkyl having 1 to 9 carbon atoms or optionally substituted alkoxyalkyl having 2 to 9 carbon atoms, provided that $R_2$ is the same as $R_6$ and $R_4$ is the same as $R_8$ while $R_2$ and $R_6$ may be the same as or different from $R_4$ and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ are different from $R'_2$ and $R'_4$.

Preferred compounds are compounds of formula (1) wherein $R_1$, $R_3$, $R_5$, and $R_7$ each represent a hydrogen atom or straight-chain or branched alkyl having 1 to 4 carbon atoms, provided that $R_1$ is the same as $R_5$ and $R_3$ is the same as $R_7$ while $R_1$ and $R_5$ are different from $R_3$ and $R_7$; $R_2$, $R_4$, $R_6$, and $R_8$ each represent straight-chain alkyl having 4 to 8 carbon atoms, provided that $R_2$ is the same as $R_6$ and $R_4$ is the same as $R_8$ while $R_2$ and $R_6$ may be the same as or different from $R_4$ and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ may be the same as or different from $R'_2$ and $R'_4$;

$R_1$, $R_3$, $R_5$, and $R_7$ each represent a hydrogen atom or straight-chain or branched alkyl having 1 to 4 carbon atoms, provided that $R_1$ is the same as $R_5$ and $R_3$ is the same as $R_7$ while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ is the same as $R_6$ and $R_4$ is the same as $R_8$ while $R_2$ and $R_6$ are different from $R_4$ and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ may be the same as or different from $R'_2$ and $R'_4$; or

$R_1$, $R_3$, $R_5$, and $R_7$ each represent a hydrogen atom or straight-chain or branched alkyl having 1 to 4 carbon atoms, provided that $R_1$ is the same as $R_5$ and $R_3$ is the same as $R_7$ while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ is the same as $R_6$ and $R_4$ is the same as $R_8$ while $R_2$ and $R_6$ may be the same as or different from $R_4$ and $R_8$; and $R'_1$, $R'_2$, $R'_3$, and $R'_4$ each represent a hydrogen atom, methyl, or ethyl, provided that $R'_1$ is the same as $R'_3$ and $R'_2$ is the same as $R'_4$ while $R'_1$ and $R'_3$ are different from $R'_2$ and $R'_4$.

The compounds in the third embodiment are preferably compounds of formula (1) wherein $R_1$ and $R_5$ represent a hydrogen atom, $R_3$ and $R_7$ represent n-propyl or isobutyl, $R_2$, $R_4$, $R_6$, and $R_8$ represent n-pentyl, $R'_1$, $R'_2$, $R'_3$, and $R'_4$ represent methyl, $R_1$ and $R_5$ represent methyl, $R_3$ and $R_7$ represent a hydrogen atom, n-propyl, or n-butyl, $R_2$, $R_4$, $R_6$, and $R_8$ represent n-pentyl, $R'_1$, $R'_2$, $R'_3$, and $R'_4$ represent methyl; compounds of formula (1) wherein $R_1$ and $R_5$ represent isobutyl, $R_3$ and $R_7$ represent methyl or n-propyl, $R_2$, $R_4$, $R_6$, and $R_8$ represent n-pentyl, $R'_1$, $R'_2$, $R'_3$, and $R'_4$ represent methyl; or compounds of formula (1) wherein $R_1$, $R_3$, $R_5$, and $R_7$ represent methyl, $R_2$, $R_4$, $R_6$, and $R_8$ represent n-pentyl, $R'_1$ and $R'_3$ represent methyl, and $R'_2$, and $R'_4$ represent a hydrogen atom or ethyl.

The compounds in the third embodiment are more preferably compounds of formula (1) wherein $R_1$ and $R_5$ represent a hydrogen atom, $R_3$ and $R_7$ represent isobutyl, $R_2$, $R_4$, $R_6$, and $R_8$ represent n-pentyl, $R'_1$, $R'_2$, $R'_3$, and $R'_4$ represent methyl; and compounds of formula (1) wherein $R_1$ and $R_5$ represent isobutyl, $R_3$ and $R_7$ represent n-propyl, $R_2$, $R_4$, $R_6$, and $R_8$ represent n-pentyl, and $R'_1$, $R'_2$, $R'_3$, and $R'_4$ represent methyl.

Compounds of formula (1) wherein the octadepsipeptide chain constituting the ring is of -A-A-A-A- type may be mentioned as compounds in a fourth embodiment of the compounds of formula (1) (compounds in the fourth embodiment).

Specific compounds are compounds of formula (1) wherein $R_1$, $R_3$, $R_5$, and $R_7$ are the same as each other and represent a hydrogen atom, optionally substituted straight-chain, branched, or cyclic alkyl having 1 to 9 carbon atoms, or optionally substituted alkoxyalkyl having 2 to 9 carbon atoms, $R_2$, $R_4$, $R_6$, and $R_8$ are the same as each other and represent a hydrogen atom, optionally substituted straight-chain, branched, or cyclic alkyl having 1 to 9 carbon atoms, or optionally substituted alkoxyalkyl having 2 to 9 carbon atoms, and $R'_1$, $R'_2$, $R'_3$, and $R'_4$ are the same as each other and represent a hydrogen atom, methyl, or ethyl.

Preferred compounds are compounds of formula (1) wherein $R_1$, $R_3$, $R_5$, and $R_7$ are the same as each other and represent a hydrogen atom or straight-chain or branched alkyl having 1 to 4 carbon atoms, $R_2$, $R_4$, $R_6$, and $R_8$ are

the same as each other and represent straight-chain alkyl having 1 to 9 carbon atoms and $R'_1$, $R'_2$, $R'_3$, and $R'_4$ are the same as each other and represent a hydrogen atom, methyl, or ethyl.

The compounds in the fourth embodiment are preferably compounds of formula (1) wherein $R_1$, $R_3$, $R_5$, and $R_7$ represent methyl, n-propyl, or isobutyl, $R_2$, $R_4$, $R_6$, and $R_8$ represent n-pentyl or n-heptyl, $R'_1$, $R'_2$, $R'_3$, and $R'_4$ represent a hydrogen atom or methyl, more preferably methyl.

Compounds of formula (1) wherein the octadepsipeptide chain constituting the ring is of -A-A-B-C- type or -B-A-A-C- type may be mentioned as compounds in a fifth embodiment of the compounds of formula (1) (compounds in the fifth embodiment).

Specific compounds are compounds of formula (1) wherein $R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ and $R_3$ are the same as each other, $R_5$ is different from $R_1$ and $R_3$ while $R_7$ is different from $R_1$, $R_3$, and $R_5$; $R_2$, $R_4$, $R_6$, and $R_8$ are the same as each other and represent a hydrogen atom, optionally substituted straight-chain, branched, or cyclic alkyl having 1 to 9 carbon atoms, or optionally substituted alkoxyalkyl having 2 to 9 carbon atoms; and $R'_1$, $R'_2$, $R'_3$, and $R'_4$ are the same as each other and represent a hydrogen atom, methyl, or ethyl; or

$R_1$, $R_3$, $R_5$, and $R_7$ each represent a hydrogen atom, optionally substituted straight-chain, branched, or cyclic alkyl having 1 to 9 carbon atoms or optionally substituted alkoxyalkyl having 2 to 9 carbon atoms, provided that $R_1$ is different from $R_3$, $R_5$, and $R_7$ while $R_3$, $R_5$, and $R_7$ are the same as each other; $R_2$, $R_4$, $R_6$, and $R_8$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_4$ is different from $R_2$, $R_6$, and $R_8$ while $R_2$, $R_6$, and $R_8$ are the same as each other, or $R_8$ is different from $R_2$, $R_4$, and $R_6$ while $R_2$, $R_4$, and $R_6$ are the same as each other; and $R'_1$, $R'_2$, $R'_3$, and $R'_4$ are the same as each other and represent a hydrogen atom, methyl, or ethyl.

Preferred compounds are compounds of formula (1) wherein $R_1$, $R_3$, $R_5$, and $R_7$ each represent a hydrogen atom or straight-chain, branched, or cyclic alkyl having 3 to 7 carbon atoms, provided that $R_1$ and $R_3$ are the same as each other and $R_5$ is different from $R_1$ and $R_3$ while $R_7$ is different from $R_1$, $R_3$, and $R_5$; $R_2$, $R_4$, $R_6$ and Rs are the same as each other and represent straight-chain alkyl having 5 to 7 carbon atoms; and $R'_1$, $R'_2$, $R'_3$, and $R'_4$ are the same as each other and represent methyl; or

$R_1$, $R_3$, $R_5$, and $R_7$ each represent a hydrogen atom or straight-chain, branched, or cyclic alkyl having 3 to 7 carbon atoms, provided that $R_1$ is different from $R_3$, $R_5$, and $R_7$ while $R_3$, $R_5$ and $R_7$ are the same as each other; $R_2$, $R_4$, $R_6$, and $R_8$ each represent straight-chain alkyl having 5 to 7 carbon atoms, provided that $R_4$ is different from $R_2$, $R_6$, and $R_8$ while $R_2$, $R_6$, and $R_8$ are the same as each other, or $R_8$ is different from $R_2$, $R_4$, and $R_6$ while $R_2$, $R_4$, and $R_6$ are the same as each other; and $R'_1$, $R'_2$, $R'_3$, and $R'_4$ are the same as each other and represent methyl.

More preferred compounds are compounds of formula (1) wherein $R_1$, $R_3$, $R_5$, and $R_7$ each represent a hydrogen atom, n-propyl, i-butyl, or neopentyl, provided that $R_1$ and $R_3$ are the same while $R_5$ is different from $R_1$ and $R_3$ and $R_7$ is different from $R_1$, $R_3$, and $R_5$; $R_2$, $R_4$, $R_6$, and $R_8$ are the same as each other and represent straight-chain alkyl having 5 to 7 carbon atoms; and $R'_1$, $R'_2$, $R'_3$, and $R'_4$ are the same as each other and represent methyl; or

$R_1$, $R_3$, $R_5$, and $R_7$ each represent a hydrogen atom, n-propyl, i-butyl, or neopentyl, provided that $R_1$ is different from $R_3$, $R_5$, and $R_7$ while $R_3$, $R_5$, and $R_7$ are the same as each other; $R_2$, $R_4$, $R_6$, and $R_8$ each represent straight-chain alkyl having 5 to 7 carbon atoms, provided that $R_4$ is different from $R_2$, $R_6$, and $R_8$ while $R_2$, $R_6$, and $R_8$ are the same as each other, or $R_8$ is different from $R_2$, $R_4$, and $R_6$ while $R_2$, $R_4$, and $R_6$ are the same as each other; and $R'_1$, $R'_2$, $R'_3$, and $R'_4$ are the same as each other and represent methyl.

The compounds in the fifth embodiment are preferably compounds of formula (1) wherein $R_1$ and $R_3$ represent isobutyl and $R_5$ and $R_7$ are different and represent a hydrogen atom or n-propyl, $R_2$, $R_4$, $R_6$, and $R_8$ represent n-pentyl, and $R'_1$, $R'_2$, $R'_3$, and $R'_4$ represent methyl; compounds of formula (1) wherein $R_1$ and $R_3$ represent n-propyl and $R_5$ and $R_7$ are different and represent a hydrogen atom or isobutyl, $R_2$, $R_4$, $R_6$, and $R_8$ represent n-pentyl, and $R'_1$, $R'_2$, $R'_3$, and $R'_4$ represent methyl.

The compounds in the fifth embodiment are preferably compounds of formula (1) wherein $R_1$ represents isobutyl and $R_3$, $R_5$, and $R_7$ represent a hydrogen atom; $R_2$, $R_4$, and $R_6$ represent n-penty and $R_8$ represents n-heptyl; and $R'_1$, $R'_2$, $R'_3$, and $R'_4$ represent methyl.

The compounds in the fifth embodiment are more preferably compounds of formula (1) wherein $R_1$ and $R_3$ represent isobutyl, $R_5$ and $R_7$ are different and represent a hydrogen atom or n-propyl, $R_2$, $R_4$, $R_6$, and $R_8$ represent n-pentyl, and $R'_1$, $R'_2$, $R'_3$, and $R'_4$ represent methyl; or compounds of formula (1) wherein $R_1$ and $R_3$ represent n-propyl, $R_5$ and $R_7$ are different and represent a hydrogen atom or isobutyl, $R_2$, $R_4$, $R_6$, and $R_8$ represent n-pentyl, and $R'_1$, $R'_2$, $R'_3$, and $R'_4$ represent methyl.

Compounds of formula (1) wherein the octadepsipeptide chain constituting the ring is of -A-B-A-C- type or -B-A-C-A- type may be mentioned as compounds in a sixth embodiment of the compounds of formula (1) (compounds in the sixth embodiment).

Specific compounds are compounds of formula (1) wherein $R_1$, $R_3$, $R_5$, and $R_7$ each represent a hydrogen

atom, optionally substituted straight-chain, branched, or cyclic alkyl having 1 to 9 carbon atoms, or optionally substituted alkoxyalkyl having 2 to 9 carbon atoms, provided that $R_1$ is the same as $R_5$ and $R_3$ is different from $R_1$ and $R_5$ while $R_7$ is different from $R_1$, $R_3$, and $R_5$; $R_2$, $R_4$, $R_6$, and $R_8$ are the same as each other and represent a hydrogen atom, optionally substituted straight-chain, branched, or cyclic alkyl having 1 to 9 carbon atoms, or optionally substituted alkoxyalkyl having 2 to 9 carbon atoms; and $R'_1$, $R'_2$, $R'_3$, and $R'_4$ are the same as each other and represent a hydrogen atom, methyl, or ethyl; or

$R_1$, $R_3$, $R_5$, and $R_7$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_1$ is different from $R_3$, $R_5$ and $R_7$ while $R_3$, $R_5$, and $R_7$ are the same as each other; $R_2$, $R_4$, $R_6$, and $R_8$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_6$ is different from $R_2$, $R_4$, and $R_8$ while $R_2$, $R_4$, and $R_8$ are the same as each other; and $R'_1$, $R'_2$, $R'_3$, and $R'_4$ are the same as each other and represent a hydrogen atom, methyl, or ethyl.

Preferred compounds are compounds of formula (1) wherein $R_1$, $R_3$, $R_5$, and $R_7$ each represent a hydrogen atom or straight-chain, branched, or cyclic alkyl having 1 to 7 carbon atoms, provided that $R_1$ is the same as $R_5$ and $R_3$ is different from $R_1$ and $R_5$ while $R_7$ is different from $R_1$, $R_3$, and $R_5$; $R_2$, $R_4$, $R_6$, and $R_8$ are the same as each other and represent straight-chain alkyl having 5 to 7 carbon atoms; and $R'_1$, $R'_2$, $R'_3$, and $R'_4$ are the same as each other and represent methyl; or

$R_1$, $R_3$, $R_5$, and $R_7$ each represent a hydrogen atom or straight-chain, branched, or cyclic alkyl having 1 to 7 carbon atoms, provided that $R_1$ is different from $R_3$, $R_5$, and $R_7$ and $R_3$, $R_5$, and $R_7$ are the same as each other; $R_2$, $R_4$, $R_6$, and $R_8$ each represent straight chain alkyl having 5 to 7 carbon atoms, provided that $R_6$ is different from $R_2$, $R_4$, and $R_8$ and $R_2$, $R_4$, and $R_8$ are the same as each other; and $R'_1$, $R'_2$, $R'_3$, and $R'_4$ are the same as each other and represent methyl.

More preferred compounds are compounds of formula (1) wherein $R_1$, $R_3$, $R_5$, and $R_7$ each represent a hydrogen atom, methyl, n-propyl, i-butyl, or neopentyl, provided that $R_1$ is the same as $R_5$ and $R_3$ is different from $R_1$ and $R_5$ while $R_7$ is different from $R_1$, $R_3$, and $R_5$; $R_2$, $R_4$, $R_6$, and $R_8$ are the same as each other and represent straight-chain alkyl having 5 to 7 carbon atoms; and $R'_1$, $R'_2$, $R'_3$, and $R'_4$ are the same as each other and represent methyl; or

$R_1$, $R_3$, $R_5$, and $R_7$ each represent a hydrogen atom, methyl, n-propyl, i-butyl, or neopentyl, provided that $R_1$ is different from $R_3$, $R_5$ and $R_7$ while $R_3$, $R_5$, and $R_7$ are the same as each other; $R_2$, $R_4$, $R_6$, and $R_8$ each represent straight-chain alkyl having 5 to 7 carbon atoms, provided that $R_6$ is different from $R_2$, $R_4$, and $R_8$ while $R_2$, $R_4$, and $R_8$ are the same as each other; and $R'_1$, $R'_2$, $R'_3$, and $R'_4$ are the same as each other and represent methyl.

The compounds in the sixth embodiment are preferably compounds of formula (1) wherein $R_1$ and $R_5$ represent isobutyl, $R_3$ is different from $R_7$ and represent a hydrogen atom or methyl, $R_2$, $R_4$, $R_6$, and $R_8$ represent n-pentyl, and $R'_1$, $R'_2$, $R'_3$, and $R'_4$ represent methyl; and compounds of formula (1) wherein $R_1$ and $R_5$ represent n-propyl, $R_3$ and $R_7$ are different and represent a hydrogen atom or isobutyl, $R_2$, $R_4$, $R_6$, and $R_8$ represent n-pentyl, and $R'_1$, $R'_2$, $R'_3$, and $R'_4$ represent methyl.

Specific examples of compounds represented by formula (1) are described in Table 1.

[Table 1]

| Compound No. | N (R'1) | Amino acid (R1) | Hydroxy acid (R2) | N(R'2) | Amino acid (R3) | Hydroxy acid (R4) | N (R'3) | Amino acid (R5) | Hydroxy acid (R6) Hydroxy | N(R'4) | Amino acid (R7) | Hydroxy acid (R8) |
|---|---|---|---|---|---|---|---|---|---|---|---|---|
| 1 | $CH_3$ | $CH_3$ | C5 | $CH_3$ | $CH_3$ | C5 | $CH_3$ | $CH_3$ | C5 | $CH_3$ | H | C5 |
| 2 | $CH_3$ | $CH_3$ | C5 | $CH_3$ | $CH_3$ | C5 | $CH_3$ | $CH_3$ | C5 | $CH_3$ | H | C6 |
| 3 | $CH_3$ | $CH_3$ | C5 | $CH_3$ | $CH_3$ | C5 | $CH_3$ | $CH_3$ | C5 | $CH_3$ | H | C7 |
| 4 | $CH_3$ | $CH_3$ | C5 | $CH_3$ | $CH_3$ | C5 | $CH_3$ | $CH_3$ | C5 | $CH_3$ | H | C8 |
| 5 | $CH_3$ | $CH_3$ | C5 | $CH_3$ | $CH_3$ | C5 | $CH_3$ | $CH_3$ | C5 | $CH_3$ | H | C9 |
| 6 | $CH_3$ | $CH_3$ | C5 | $CH_3$ | $CH_3$ | C5 | $CH_3$ | $CH_3$ | C5 | $CH_3$ | $CH_3$ | C4 |
| 7 | $CH_3$ | $CH_3$ | C5 | $CH_3$ | $CH_3$ | C5 | $CH_3$ | $CH_3$ | C5 | $CH_3$ | $CH_3$ | C6 |
| 8 | $CH_3$ | $CH_3$ | C5 | $CH_3$ | $CH_3$ | C5 | $CH_3$ | $CH_3$ | C5 | $CH_3$ | $CH_3$ | C8 |
| 9 | $CH_3$ | $CH_3$ | C5 | $CH_3$ | $CH_3$ | C5 | $CH_3$ | $CH_3$ | C5 | $CH_3$ | $CH_3$ | C9 |
| 10 | $CH_3$ | $CH_3$ | C5 | $CH_3$ | $CH_3$ | C5 | $CH_3$ | $CH_3$ | C5 | $CH_3$ | $CH_2CH_3$ | C5 |
| 11 | $CH_3$ | $CH_3$ | C5 | $CH_3$ | $CH_3$ | C5 | $CH_3$ | $CH_3$ | C5 | $CH_3$ | $CH_2CH_3$ | C6 |
| 12 | $CH_3$ | $CH_3$ | C5 | $CH_3$ | $CH_3$ | C5 | $CH_3$ | $CH_3$ | C5 | $CH_3$ | $CH_2CH_3$ | C7 |
| 13 | $CH_3$ | $CH_3$ | C5 | $CH_3$ | $CH_3$ | C5 | $CH_3$ | $CH_3$ | C5 | $CH_3$ | $CH_2CH_3$ | C8 |
| 14 | $CH_3$ | $CH_3$ | C5 | $CH_3$ | $CH_3$ | C5 | $CH_3$ | $CH_3$ | C5 | $CH_3$ | $CH_2CH_3$ | C9 |
| 15 | $CH_3$ | $CH_3$ | C5 | $CH_3$ | $CH_3$ | C5 | $CH_3$ | $CH_3$ | C5 | $CH_3$ | $(CH_2)_3CH_3$ | C5 |
| 16 | $CH_3$ | $CH_3$ | C5 | $CH_3$ | $CH_3$ | C5 | $CH_3$ | $CH_3$ | C5 | $CH_3$ | $(CH_2)_3CH_3$ | C7 |
| 17 | $CH_3$ | $CH_3$ | C5 | $CH_3$ | $CH_3$ | C5 | $CH_3$ | $CH_3$ | C5 | $CH_3$ | $(CH_2)_3CH_3$ | C8 |
| 18 | $CH_3$ | $CH_3$ | C5 | $CH_3$ | $CH_3$ | C5 | $CH_3$ | $CH_3$ | C5 | $CH_3$ | $(CH_2)_3CH_3$ | C9 |
| 19 | $CH_3$ | $CH_3$ | C5 | $CH_3$ | $CH_3$ | C5 | $CH_3$ | $CH_3$ | C5 | $CH_3$ | $(CH_2)_2CH_3$ | C5 |
| 20 | $CH_3$ | $CH_3$ | C5 | $CH_3$ | $CH_3$ | C5 | $CH_3$ | $CH_3$ | C5 | $CH_3$ | $(CH_2)_2CH_3$ | C6 |
| 21 | $CH_3$ | $CH_3$ | C5 | $CH_3$ | $CH_3$ | C5 | $CH_3$ | $CH_3$ | C5 | $CH_3$ | $(CH_2)_2CH_3$ | C7 |
| 22 | $CH_3$ | $CH_3$ | C5 | $CH_3$ | $CH_3$ | C5 | $CH_3$ | $CH_3$ | C5 | $CH_3$ | $(CH_2)_2CH_3$ | C8 |
| 23 | $CH_3$ | $CH_3$ | C5 | $CH_3$ | $CH_3$ | C5 | $CH_3$ | $CH_3$ | C5 | $CH_3$ | $(CH_2)_2CH_3$ | C9 |
| 24 | $CH_3$ | $CH_3$ | C5 | $CH_3$ | $CH_3$ | C5 | $CH_3$ | $CH_3$ | C5 | $CH_3$ | $CH(CH_3)_2$ | C5 |

(continued)

| Compound No. | N (R'1) | Amino acid (R1) | Hydroxy acid (R2) | N(R'2) | Amino acid (R3) | Hydroxy acid (R4) | N (R'3) | Amino acid (R5) | Hydroxy acid (R6) Hydroxy | N(R'4) | Amino acid (R7) | Hydroxy acid (R8) |
|---|---|---|---|---|---|---|---|---|---|---|---|---|
| 25 | $CH_3$ | $CH_3$ | C5 | $CH_3$ | $CH_3$ | C5 | $CH_3$ | $CH_3$ | C5 | $CH_3$ | $CH(CH_3)_2$ | C7 |
| 26 | $CH_3$ | $CH_3$ | C5 | $CH_3$ | $CH_3$ | C5 | $CH_3$ | $CH_3$ | C5 | $CH_3$ | $CH(CH_3)_2$ | C9 |
| 27 | $CH_3$ | $CH_3$ | C5 | $CH_3$ | $CH_3$ | C5 | $CH_3$ | $CH_3$ | C5 | $CH_3$ | $CH_2CH(CH_3)_2$ | C5 |
| 28 | $CH_3$ | $CH_3$ | C5 | $CH_3$ | $CH_3$ | C5 | $CH_3$ | $CH_3$ | C5 | $CH_3$ | $CH_2CH(CH_3)_2$ | C7 |
| 29 | $CH_3$ | $CH_3$ | C5 | $CH_3$ | $CH_3$ | C5 | $CH_3$ | $CH_3$ | C5 | $CH_3$ | $CH_2CH(CH_3)_2$ | C8 |
| 30 | $CH_3$ | $CH_3$ | C5 | $CH_3$ | $CH_3$ | C5 | $CH_3$ | $CH_3$ | C5 | $CH_3$ | $CH_2CH(CH_3)_2$ | C9 |
| 31 | $CH_3$ | $CH_3$ | C5 | $CH_3$ | $CH_3$ | C5 | $CH_3$ | $CH_3$ | C5 | $CH_3$ | $(CH_2)_3OH$ | C5 |
| 32 | $CH_3$ | H | C5 | $CH_3$ | H | C5 | $CH_3$ | H | C5 | $CH_3$ | $CH_2CH(CH_3)_2$ | C7 |
| 33 | $CH_3$ | $CH_2CH(CH_3)_2$ | C5 | $CH_3$ | $CH_2CH(CH_3)_2$ | C5 | $CH_3$ | $CH_2CH(CH_3)_2$ | C5 | $CH_3$ | H | C5 |
| 34 | $CH_3$ | H | C5 | $CH_3$ | H | C5 | $CH_3$ | H | C5 | $CH_3$ | $CH_2CH(CH_3)_2$ | C5 |
| 35 | $CH_3$ | H | C5 | $CH_3$ | $CCH_2)_2CH_3$ | C5 | $CH_3$ | H | C5 | $CH_3$ | $(CH_2)_2CH_3$ | C5 |
| 36 | $CH_3$ | H | C5 | $CH_3$ | $CH_2CH(CH_3)_2$ | C5 | $CH_3$ | H | C5 | $CH_3$ | $CH_2CH(CH_3)_2$ | C5 |
| 37 | $CH_3$ | $CH_3$ | C5 | $CH_3$ | H | C5 | $CH_3$ | $CH_3$ | C5 | $CH_3$ | H | C5 |
| 38 | $CH_3$ | $CH_3$ | C5 | $CH_3$ | $(CH_2)_2CH_3$ | C5 | $CH_3$ | $CH_3$ | C5 | $CH_3$ | $(CH_2)_2CH_3$ | C5 |
| 39 | $CH_3$ | $CH_3$ | C5 | $CH_3$ | $(CH_2)_3CH_3$ | C5 | $CH_3$ | $CH_3$ | C5 | $CH_3$ | $(CH_2)_3CH_3$ | C5 |
| 40 | $CH_3$ | $CH_2CH(CH_3)_2$ | C5 | $CH_3$ | $CH_3$ | C5 | $CH_3$ | $CH_2CH(CH_3)_2$ | C5 | $CH_3$ | $CH_3$ | C5 |
| 41 | $CH_3$ | $CH_2CH(CH_3)_2$ | C5 | $CH_3$ | $(CH_2)_2CH_3$ | C5 | $CH_3$ | $CH_2CH(CH_3)_2$ | C5 | $CH_3$ | $(CH_2)_2CH_3$ | C5 |

(continued)

| Compound No. | N (R'1) | Amino acid (R1) | Hydroxy acid (R2) | N(R'2) | Amino acid (R3) | Hydroxy acid (R4) | N (R'3) | Amino acid (R5) | Hydroxy acid (R6) Hydroxy | N(R'4) | Amino acid (R7) | Hydroxy acid (R8) |
|---|---|---|---|---|---|---|---|---|---|---|---|---|
| 42 | $CH_3$ | $CH_3$ | C5 | H | $CH_3$ | C5 | $CH_3$ | $CH_3$ | C5 | H | $CH_3$ | C5 |
| 43 | $CH_3$ | $CH_3$ | C5 | $CH_2CH_3$ | $CH_3$ | C5 | $CH_3$ | $CH_3$ | C5 | $CH_2CH_3$ | $CH_3$ | C5 |
| 44 | H | $CH_3$ | C5 | H | $CH_3$ | C5 | H | $CH_3$ | C5 | H | $CH_3$ | C5 |
| 45 | $CH_3$ | $(CH_2)_2CH_3$ | | $CH_3$ | $(CH_2)_2CH_3$ | C5 | $CH_3$ | $(CH_2)_2CH_3$ | C5 | $CH_3$ | $(CH_2)_2CH_3$ | C5 |
| 46 | $CH_3$ | $CH_2CH(CH_3)_2$ | C5 | $CH_3$ | $CH_2CH(CH_3)_2$ | C5 | $CH_3$ | $CH_2CH(CH_3)_2$ | C5 | $CH_3$ | $CH_2CH(CH_3)_2$ | C5 |
| 47 | $CH_3$ | $CH_2CH(CH_3)_2$ | C5 | $CH_3$ | $CH_2CH(CH_3)_2$ | C5 | $CH_3$ | H | C5 | $CH_3$ | H | C5 |
| 48 | $CH_3$ | H | C5 | $CH_3$ | H | C5 | $CH_3$ | $CH_2CH(CH_3)_2$ | C7 | $CH_3$ | $CH_2CH(CH_3)_2$ | C7 |
| 49 | $CH_3$ | $CH_2CH(CH_3)_2$ | C5 | $CH_3$ | H | C5 | $CH_3$ | H | C5 | $CH_3$ | H | C7 |
| 50 | H | $CH_3$ | C5 | H | $CH_3$ | C5 | H | $CH_3$ | C5 | $CH_3$ | $CH_3$ | C5 |
| 51 | $CH_3$ | $CH_3$ | C7 | $CH_3$ | $CH_3$ | C7 | $CH_3$ | $CH_3$ | C7 | $CH_3$ | $CH_3$ | C7 |
| 52 | $CH_3$ | $CH_2CH(CH_3)_2$ | C5 | $CH_3$ | H | C5 | $CH_3$ | $CH_2CH(CH_3)_2$ | C5 | $CH_3$ | $CH_3$ | C5 |
| 53 | $CH_3$ | $CH_2CH(CH_3)_2$ | C5 | $CH_3$ | $CH_2CH(CH_3)_2$ | C5 | $CH_3$ | $(CH_2)_2CH_3$ | C5 | $CH_3$ | $(CH_2)_2CH_3$ | C5 |
| 54 | $CH_3$ | $CH_3$ | C5 | $CH_3$ | $CH_3$ | C5 | $CH_3$ | $CH_3$ | C5 | $CH_3$ | H | $CH_2CH(CH_3)_2$ |
| 55 | $CH_3$ | $(CH_2)_2CH_3$ | C5 | $CH_3$ | $(CH_2)_2CH_3$ | C5 | $CH_3$ | $(CH_2)_2CH_3$ | C5 | $CH_3$ | $CH_2CH(CH_3)_2$ | C5 |
| 56 | $CH_3$ | $CH_2CH(CH_3)_2$ | C5 | $CH_3$ | $CH_2CH(CH_3)_2$ | C5 | $CH_3$ | $CH_2CH(CH_3)_2$ | C5 | $CH_3$ | $(CH_2)_2CH_3$ | C5 |
| 57 | $CH_3$ | $CH_2CH(CH_3)_2$ | C5 | $CH_3$ | $CH_2CH(CH_3)_2$ | C5 | $CH_3$ | H | C5 | $CH_3$ | $(CH_2)_2CH_3$ | C5 |

(continued)

| Compound No. | N (R'1) | Amino acid (R1) | Hydroxy acid (R2) | N(R'2) | Amino acid (R3) | Hydroxy acid (R4) | N (R'3) | Amino acid (R5) | Hydroxy acid (R6) Hydroxy | N(R'4) | Amino acid (R7) | Hydroxy acid (R8) |
|---|---|---|---|---|---|---|---|---|---|---|---|---|
| 58 | $CH_3$ | $CH_2CH(CH_3)_2$ | C5 | $CH_3$ | $CH_2CH(CH_3)_2$ | C5 | $CH_3$ | $(CH_2)_2CH_3$ | C5 | $CH_3$ | H | C5 |
| 59 | $CH_3$ | $(CH_2)_2CH_3$ | C5 | $CH_3$ | $(CH_2)_2CH_3$ | C5 | $CH_3$ | H | C5 | $CH_3$ | $CH_2CH(CH_3)_2$ | C5 |
| 60 | $CH_3$ | $(CH_2)_2CH_3$ | C5 | $CH_3$ | $(CH_2)_2CH_3$ | C5 | $CH_3$ | $CH_2CH(CH_3)_2$ | C5 | $CH_3$ | H | C5 |
| 61 | $CH_3$ | $CH_3$ | C5 | $CH_3$ | $CH_3$ | C5 | $CH_3$ | $CH_3$ | C5 | $CH_3$ | $CH_3$ | $CH_2CH(CH_3)_2$ |
| 62 | $CH_3$ | $CH_3$ | C5 | $CH_3$ | $CH_3$ | C5 | $CH_3$ | $CH_3$ | C5 | $CH_3$ | $CH_2CH(CH_3)_2$ | $CH_2CH(CH_3)_2$ |
| 63 | $CH_3$ | $CH_3$ | C5 | $CH_3$ | $CH_3$ | C5 | $CH_3$ | $CH_3$ | C5 | $CH_3$ | H | $CH_2$cPro |
| 64 | $CH_3$ | $CH_3$ | C5 | $CH_3$ | $CH_3$ | C5 | $CH_3$ | $CH_3$ | C5 | $CH_3$ | $CH_2CH(CH_3)_2$ | $CH_2$cPro |
| 65 | $CH_3$ | $CH_3$ | C5 | $CH_3$ | $CH_3$ | C5 | $CH_3$ | $CH_3$ | C5 | $CH_3$ | $CH_3$ | $CH_2$cPro |
| 66 | $CH_3$ | $(CH_2)_2CH_3$ | C5 | $CH_3$ | $CH_2CH(CH_3)_2$ | C5 | $CH_3$ | $(CH_2)_2CH_3$ | C5 | $CH_3$ | H | C5 |
| 67 | $CH_3$ | $(CH_2)_2CH_3$ | C5 | $CH_3$ | $(CH_2)_2CH_3$ | C5 | $CH_3$ | $(CH_2)_2CH_3$ | C5 | $CH_3$ | H | C5 |
| 68 | $CH_3$ | $(CH_2)_2CH_3$ | C5 | $CH_3$ | $(CH_2)_2CH_3$ | C5 | $CH_3$ | $(CH_l)_2CH_3$ | C5 | $CH_3$ | $CH_2$tBu | C5 |
| 69 | $CH_3$ | $(CH_2)_2CH_3$ | C5 | $CH_3$ | $(CH_2)_2CH_3$ | C5 | $CH_3$ | $(CH_2)_2CH_3$ | C5 | $CH_3$ | $CH_2$cHex | C5 |

In the table, C4-C9 represents straight-chain alkyl.

Further, in the table, "$CH_2$cHex" represents cyclohexylmethyl, and "$CH_2$cPro" represents cyclopropylmethyl.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ in formula (1) are combinations indicated in compound Nos. 1 to 34, 50, 54 to 56, 61 to 65 and 67 to 69 in Table 1 may be used as compounds in the first embodiment.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are combinations indicated in compound Nos. 47, 48, and 53 in Table 1 may be used as compounds in the second embodiment.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are combinations indicated in compound Nos. 35 to 43 in Table 1 may be used as compounds in the third embodiment.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are combinations indicated in compound Nos. 44 to 46 and 51 in Table 1 may be used as compounds in the fourth embodiment.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are combinations indicated in compound Nos. 49 and 57 to 60 in Table 1 may be used as compounds in the fifth embodiment.

For example, compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are combinations indicated in compound Nos. 52 and 66 in Table 1 may be used as compounds in the sixth embodiment.

In compounds of formula (I), various isomers exist. The present invention embraces both the isomers and mixtures thereof. The presence of isomers derived from other groups in formula (I) is also considered. These isomers and mixtures thereof are also embraced in formula (I).

Production of compounds of the present invention

Compounds of the present invention can be produced by a person having ordinary skill in the art by the method described in Nobuo Izumiya, Tetsuo Kato, Haruhiko Aoyanagi, and Michinori Waki, "Pepuchido Gosei No Kiso To Jikken (Basis and Experiments of Peptide Synthesis)," Maruzen Co., Ltd., 1985. The method will be described in detail.

Compounds of formula (1) can be produced by cyclizing compounds of formula (2) (straight-chain octadepsipeptide):

[Chemical formula 7]

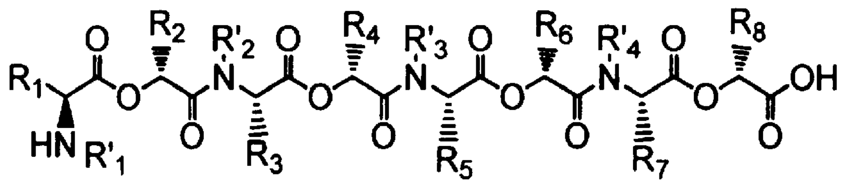

(2)

wherein $R_1$, $R_2$, $R_3$, $R_4$, $R_5$, $R_6$, $R_7$, $R_8$, $R'_1$, $R'_2$, $R'_3$, and $R'_4$ are as defined above. Specifically, compounds of formula (1) can be produced by reacting a compounds of formula (2) using a suitable condensing agent in the presence of a basic reaction assistant after the addition of a suitable inert solvent to give a diluted solution.

All of condensing agents suitable for the production of an amide bond may be mentioned as the condensing agent. Examples thereof include carbodiimide-based condensing agents such as dicyclohexylcarbodiimide (DCC), and 1-ethyl-3-(3-dimethylaminopropyl)carbodiimide hydrochloride (EDCI), imidazolinium-based condensing agents such as 1,1'-carbonyldiimidazole (CDI) and 2-chloro-1,3-dimethylimidazolinium hexafluorophosphate (CIP), and phosphonium salt-based condensing agents such as 1H-benzotriazole-1-yloxytripyrrolidinophosphonium hexafluorophosphate (PyBOP), and hexafluorophosphoric acid bromotris(pyrrolidino)phosphonium(PyBrop). Preferred are EDCI and PyBOP.

Basic reaction assistants include basic compounds such as amidine bases and quanidine bases, for example, 7-methyl-1,5,7-triazabicyclo(4.4.0)dec-5-ene (MTBD): diazabicyclo(4.3.0)nonene (DBN), diazabicyclo(2.2.2)-octane (BCO), 1,8-diaza-bicyclo(5.4.0)undecene (DBU), cyclohexyl-tetrabutylguanidine (CyTBG), cyclohexyltetramethylguanidine (CyTMG), N,N,N,N-tetramethyl-1,8-naphthalenediamine, pentamethylpiperidine, tertiary amines, triethylamine, trimethylamine, tribenzylamine, triisopropylamine, tributylamine, tribenzylamine, tricyclohexylamine, triamylamine, trihexylamine, N,N-dimethyl-aniline, N,N-dimethyl-toluidine, N,N-dimethyl-p-aminopyridine, N-methyl-pyrrolidine, N-methyl-piperidine, N-methyl-imidazole, N-methyl-pyrrole, N-methyl-morpholine, N-methyl- hexamethyleneimine, pyridine, 4-pyrrolidinopyridine, 4- dimethylamino-pyridine, quinoline, α-picoline, β-picoline, isoquinoline, pyrimidine, acridine, N,N,N',N'-tetramethylenediamine, N,N',N'-tetraethylenediamine, quinoxaline, N-propyl-diisopropylamine, N-ethyl- diisopropylamine, N,N'-dimethylcyclohexylamine, 2,6-lutidine, and 2,4-lutidine or triethylenediamine. Preferred are trialkylamines such as triethylamine, N,N-diisopropylethylamine, N-propyl-diisopropylamine, N,N'-dimethyl-cyclohexylamine, or N-methylmorpholine.

Any solvent may be used without limitation as long as dehydrocondensation is not inhibited. Examples of solvents include halogenated hydrocarbons such as tetrachloroethylene, tetrachloroethane, dichloropropane, methylene chloride, dichlorobutane, chloroform, carbon tetrachloride, trichloroethane, trichloroethylene, pentachloroethane, difluorobenzene, 1,2-dichloroethane, chlorobenzene, bromobenzene, dichlorobenzene, chlorotoluene, and trichlorobenzene, ethers such as ethyl propyl ether, methyl tert-butyl ether, n-butyl ether, anisole, phenetol, cyclohexyl methyl ether, dimethyl ether, diethyl ether, dipropyl ether, diisopropyl ether, di-n-butyl ether, diisobutyl ether, diisoamyl ether, ethylene glycol dimethyl ether, tetrahydrofuran, and dioxane, amines such as trimethyl-, triethyl-, tripropyl-, tributylamine, N-methylmorpholine, pyridine, and tetramethylenediamine, esters such as
methyl acetate, ethyl acetate, butyl acetate, isobutyl acetate, dimethyl carbonate, dibutyl carbonate, and ethylene carbonate, amides such as hexamethylene phosphoric triamide, formamide, N-methyl-formamide, N,N-dimethylformamide, N,N-dipropylformamide, N,N-dibutylformamide, N-methylpyrrolidine, N-methyl-caprolactam, 1,3-dimethyl-3,4,5,6-tetrahydro-2(1H)-pyrimidine, octylpyrrolidone, octylcaprolactam, 1,3-dimethyl-2-imidazolinedione, N-formyl-piperidine, and N,N'-1,4-diformylpiperazine, and ketones such as acetone, acetophenone, methyl ethyl ketone, and methyl butyl ketone. Preferrred are halogenated hydrocarbons such as dichloromethane. These reaction solvents may be used either solely or as a mixture thereof.

The amount of the condensing agent used is in the range of 1 to 20 times by weight, preferably 1 to 5 times by weight, the amount of the compound of formula (2).

The amount of the basic reaction assistant used is in the range of 0.01 to 50 times by weight, preferably in the range of 0.01 to 10 times by weight, the amount of the compound of formula (2).

The amount of the reaction solvent used is in the range of 50 to 1000 times by weight, preferably 100 to 500 times by weight, the amount of the compound of formula (2).

The reaction temperature is in the range of -10°C to 100°C, preferably in the range of -10°C to 40°C.

Compounds of formula (2) can be obtained by removing group X and group Y from compounds of formula (3):

[Chemical formula 8]

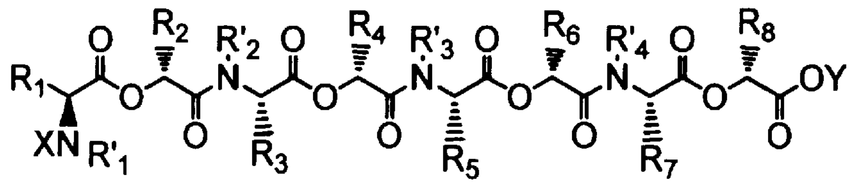

(3)

wherein $R_1$, $R_2$, $R_3$, $R_4$, $R_5$, $R_6$, $R_7$, $R_8$, $R'_1$, $R'_2$, $R'_3$, and $R'_4$ are as defined above; X represents a selectively removable N-terminal protecting group such as acetyl (Ac), allyloxycarbonyl (Alloc), benzyloxycarbonyl (CbZ), tert-butyloxycarbonyl (Boc), or 9-fluorenylmethyloxycarbonyl (Fmoc); and Y represents a support for a polymer with a selectively removable C-terminal protecting group such as benzyl (OBn), p-nitrobenzyl (ONb), tert-butyl ($OBu^t$) or a selectively removable fixing group, under suitable conditions.

The group X and the group Y can be removed, for example, by acid decomposition in the presence of protonic acid, β-elimination under mild basic conditions, or catalytic reductin with a suitable catalyst under a hydrogen atmosphere, depending upon the type of the group X and the group Y.

In the acid decomposition, a reactant prepared by introducing a hydrogen chloride gas into an organic solvent, such as HCl/ethyl acetate or HCl/dioxane, as such, trifluoracetic acid as such, solvents that do not inhibit the reaction, for example, halogenated hydrocarbons such as methylene chloride, chloroform, or carbon tetrachloride, or aromatic hydrocarbon-based solvents such as benzene or toluene may be used as solvents in the acid decomposition.

β-Elimination (for example, removal of Fmoc) may be carried out in the presence of secondary amines such as piperazine or diethylamine, for example, in aprotic polar solvents such as N,N-dimethylformamide and halogenated hydrocarbons such as methylene chloride, chloroform, and carbon tetrachloride.

The amount of the solvent used is in the range of 1 to 200 times by weight, preferably in the range of 2 to 50 times by weight, the amount of the compound of formula (3).

The reaction temperature is in the range of 0°C to 50°C, preferably in the range of 0°C to room temperature.

Catalytic reduction is carried out in a solvent using a catalyst in the presence or absence of an acid or an alkali under a hydrogen gas atmosphere.

Any solvent may be used as the solvent for the catalytic reduction as long as the solvent does not inhibit the reaction. Examples thereof include alcoholic solvents such as methanol, ethanol, n-propanol, isopropanol, and n-butanol and esters such as methyl acetate, ethyl acetate, and butyl acetate. These reaction solvents may also be used solely or as a mixture with water.

The amount of the solvent used is in the range of 1 to 200 times by weight, preferably in the range of 2 to 50 times by weight, that of the compound of formula (3).

Examples of catalysts usable in the catalytic reduction include metal-containing compounds such as 5% palladium carbon, 10% palladium carbon, and Raney nickel.

The amount of the catalyst used is in the range of 1% by weight to 100% by weight, preferably in the range of 1% by weight to 20% by weight, of the compound of formula (3).

Examples of the acid or the alkyl usable in the catalytic reduction include mineral acids such as hydrochloric acid, hydrobromic acid, hydroiodic acid, nitric acid, sulfuric acid, and phosphoric acid, organic acids such as acetic acid and methanesulfonic acid, and inorganic bases such as sodium hydroxide, potassium hydroxide, calcium hydroxide, and ammonia water.

The reaction is carried out at a temperature of room temperature to 200°C under a hydrogen atmosphere in the range of ordinary pressure to 150 atm.

Compounds of formula (3) can be synthesized by several methods. Three methods will be exemplified (processes A) to C)).

<u>Process A)</u>

Compounds of formula (3) can be produced by reacting hexadepsipeptide with didepsipeptide. Specifically, compounds of formula (3) can be produced by reacting a compound represented by formula (4) (hexadepsipeptide):

[Chemical formula 9]

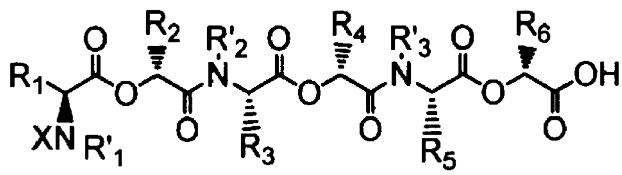

(4)

wherein $R_1$, $R_2$, $R_3$, $R_4$, $R_5$, $R_6$, $R'_1$, $R'_2$, $R'_3$, and X are as defined above,
with a compound represented by formula (5) (didepsipeptide):

[Chemical formula 10]

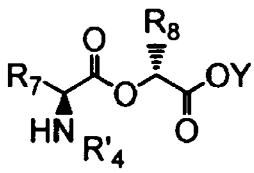

(5)

wherein $R_7$, $R_8$, $R'_4$, and Y are as defined above.

Specifically, compounds of formula (3) can be produced by reacting compounds of formula (4) with compounds of formula (5) using a suitable condensing agent in the presence of a basic reaction assistant after the addition of a suitable solvent.

Suitable condensing agents and basic reaction assistants may be as used above.

Solvents usable in the cyclization reaction are usable as the solvent.

The amount of the condensing agent used is in the range of 1 to 20 times by weight, preferably in the range of

1 to 5 times by weight, the amount of the compound of formula (4).

The amount of the basic reaction assistant is in the range of 0.01 to 50 times by weight, preferably in the range of 0.01 to 10 times by weight, the amount of the compound of formula (4).

The amount of the reaction solvent is in the range of 1 to 200 times by weight, preferably in the range of 2 to 50 times by weight, the amount of the compound of formula (4).

The reaction temperature is in the range of -10°C to 100°C, preferably in the range of -10°C to 40°C.

Process B)

Compounds of formula (3) can be provided by reacting two tetradepsipeptides which may be the same as or different from each other. Specifically, compounds of formula (3) can be produced by reacting a compound represented by formula (6) (tetradepsipeptide):

[Chemical formula 11]

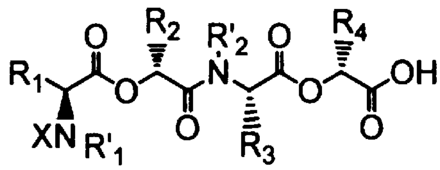

(6)

wherein $R_1$, $R_2$, $R_3$, $R_4$, $R'_1$, $R'_2$, and X are as defined above, with a compound represented by formula (7) (tetradepsipeptide):

[Chemical formula 12]

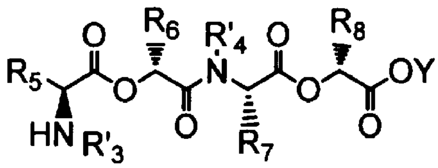

(7)

wherein $R_5$, $R_6$, $R_7$, $R_8$, $R'_3$, $R'_4$, and Y are as defined above.

Reaction conditions are the same as those in process A).

Process C)

Compounds of formula (3) can also be produced by a solid phase synthesis method using a support for a polymer with a selectively removable fixing group according to scheme 1.

[Chemical formula 13]

Scheme 1

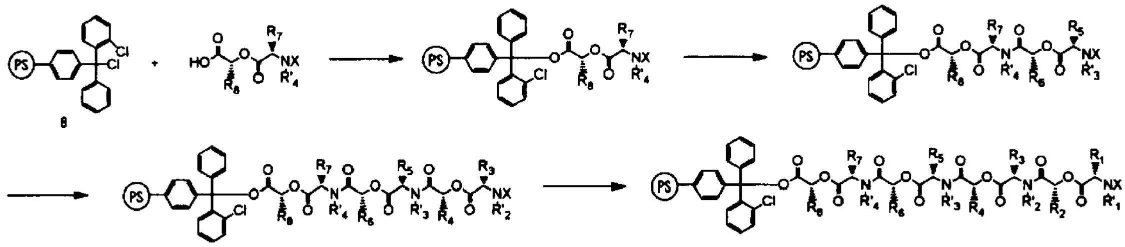

8

wherein $R_1$, $R_2$, $R_3$, $R_4$, $R_5$, $R_6$, $R'_1$, $R'_2$, $R'_3$, and X are as defined above; and PS represents a polystyrene resin.

The support for a polymer with a selectively removable fixing group includes a resin resistant to organic solvents or reagents (for example, polystyrene or polyethylene glycol-polystyrene crosslinked with divinylbenzene or the like) and a fixing group supported on the resin, for connecting the resin to the compound (for example, 2-chlorotrityl, hydroxymethylphenol), and examples thereof include 2-chlorotritylchlororesins.

Compounds of formula (4) (hexadepsipeptide) can also be obtained by providing a compound represented by formula (10):

[Chemical formula 15]

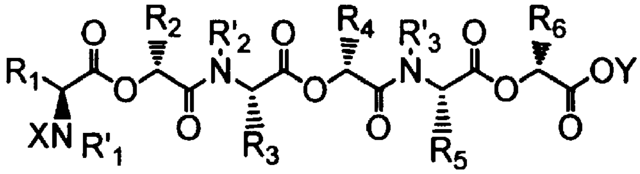

(1 0)

wherein $R_1$, $R_2$, $R_3$, $R_4$, $R_5$, $R_6$, $R'_1$, $R'_2$, $R'_3$, X, and Y are as defined above,
that can be obtained by reacting a compound of formula (6) (tetradepsipeptide) with a compound represented by formula (9) (didepsipeptide):

[Chemical formula 14]

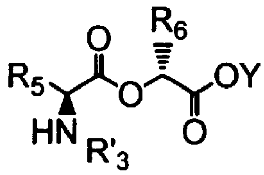

(9)

wherein $R_5$, $R_6$, $R'_3$, and Y are as defined above,
under the same conditions as used in process A), and
removing group Y from the compound of formula (10) under the same conditions as used in the above process that synthesizes the compound of formula (2) from the compound of formula (3).

Compounds of formula (6) (tetradepsipeptide) can also be produced by providing a compound represented by formula (13):

[Chemical formula 18]

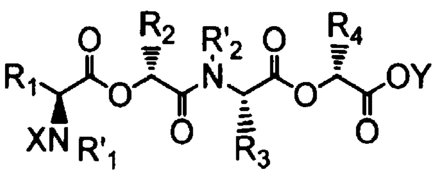

(1 3)

wherein $R_1$, $R_2$, $R_3$, $R_4$, $R'_1$, $R'_2$, X, and Y are as defined above,
that can be produced by reacting a compound of formula (11) (didepsipeptide):

[Chemical formula 16]

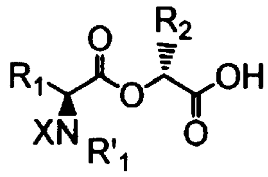

(1 1)

wherein $R_1$, $R_2$, $R'_1$, and X are as defined above, with a compound of formula (12) (didepsipeptide):

[Chemical formula 17]

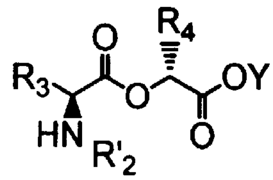

(1 2)

wherein $R_3$, $R_4$, $R'_2$, and Y are as defined above,
under the same conditions as used in process A), and
removing group Y from the compound of formula (13) under the same reaction conditions as used in the process that synthesizes the compound of formula (2) from the compound of formula (3).

Likewise, compounds of formula (7) (tetradepsipeptide) can also be produced by providing a compound of formula (15):

[Chemical formula 20]

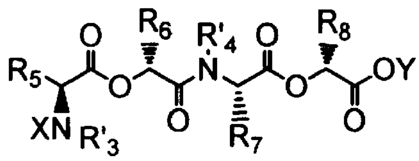

(1 5)

wherein $R_5$, $R_6$, $R_7$, $R_8$, $R'_3$, $R'_4$, X, and Y are as defined above,
that is a compound (tetradepsipeptide) produced by reacting a corresponding compound of formula (14) (didepsipeptide):

[Chemical formula 19]

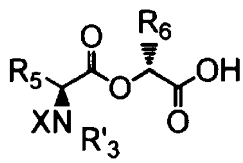

(1 4)

wherein $R_5$, $R_6$, $R'_3$, and X are as defined above,
with a compound represented by formula (5) (didepsipeptide), and
removing group X from the compound of formula (15) under the same reaction conditions as used in the process that synthesizes compound (2) from the compound of formula (3).

Didepsipeptides of formulae (9), (11), (12), and (14) can be synthesized by removing N-terminal protecting

group or C-terminal protecting group in didepsipeptide having protected N terminal and C terminal that can be synthesized by condensing α-amino acid with a protected amino group in which a corresponding amino group has been substituted by $R'_1$ to $R'_4$, with an α-hydroxycarboxylic acid ester using a suitable condensing agent.

The N-substituted and N-protected α-amino acid that is a starting material for a didepsipeptide may be a commercially available product as such, or alternatively may be a commercially available α-amino acid that is protected by a protecting group such as Boc or CbZ and has been alkylated with an alkylating agent such as an alkali halide. Further, a product obtained by protecting an amino group in a free amino acid by a protecting group such as Boc or CbZ and then alkylating the protected compound with an alkylating agent such as an alkyl halide may be used.

The α-hydroxycarboxylic acid that is a starting material for a didepsipeptide can be produced by a known process using a commercially available α-amino acid as represented by the following scheme:

[Chemical formula 21]

Scheme 2

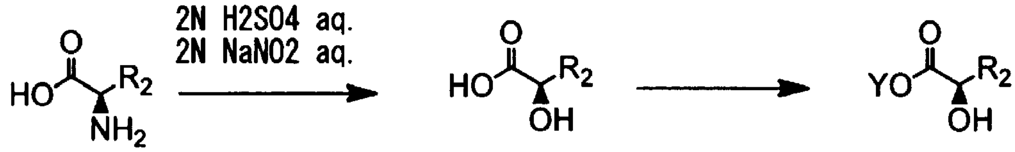


wherein $R_2$ and Y are as defined above (Brewster, P., et al. Nature 1950, 166, 179-180. Lerchen, H. G. et al. Tetrahedron Lett. 1985, 26, 5257-5260),
a process represented by the following scheme:

[Chemical formula 22]

Scheme 3

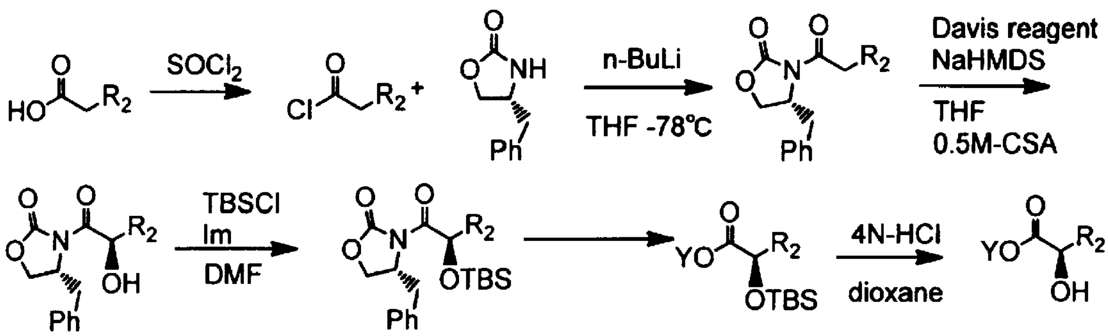


wherein $R_2$ and Y are as defined above,
using a Davis reagent (Souichi Monma et al. Org. Lett., Vol. 8, No. 24, 2006), or
a synthesis process represented by the following scheme:

[Chemical formula 23]

Scheme 4

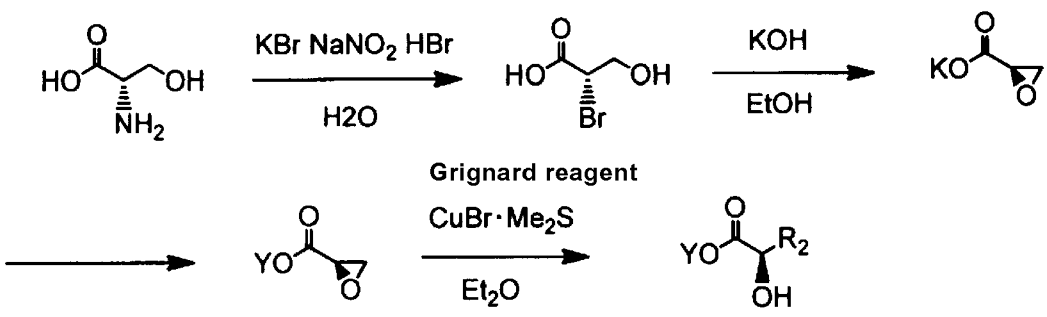

wherein $R_2$ and Y are as defined above,
using L-serine as a starting material.

For example, when compounds represented by formula (1) are compounds in which the didepsipeptide chain constituting the ring is of -A-A-A-B- type (compounds in the first embodiment),
compounds of formula (1) can be produced by reacting a straight-chain octadepsipeptide represented by formula (2a):

[Chemical formula 24]

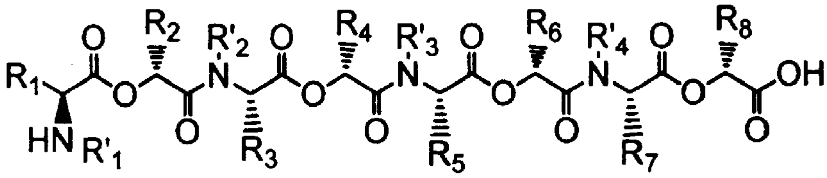

(2a)

wherein
$R_1$, $R_3$, $R_5$, and $R_7$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_1$, $R_3$, and $R_5$ are the same as each other while $R_7$ is different from $R_1$, $R_3$, and $R_5$; $R_2$, $R_4$, $R_6$, and $R_8$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_2$, $R_4$, and $R_6$ are the same as each other while $R_8$ may be the same as or different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other, or
$R_1$, $R_3$, $R_5$, and $R_7$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_1$, $R_3$, and $R_5$ are the same as each other while $R_7$ may be the same as or different from $R_1$, $R_3$, and $R_5$; $R_2$, $R_4$, $R_6$, and $R_8$ each represent a hydrogen atom, optionally substituted straight-chain, branched, or cyclic alkyl having 1 to 9 carbon atoms, or optionally substituted alkoxyalkyl having 2 to 9 carbon atoms, provided that $R_2$, $R_4$, and $R_6$ are the same as each other while $R_8$ is different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other, using a suitable condensing agent in the presence of a basic reaction assistant after the addition of a suitable solvent to give a dilute solution.

The suitable condensing agents and basic reaction assistants may be those described above. Solvents described above for use in the cyclization reaction may be used as the solvent.

Straight-chain octadepsipeptides of formula (2a) can be produced by providing a compound represented by formula (3a):

[Chemical formula 27]

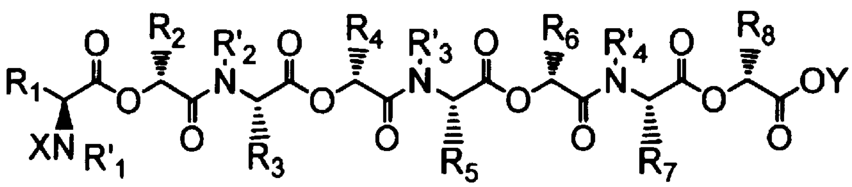

(3a)

wherein $R_1$, $R_2$, $R_3$, $R_4$, $R_5$, $R_6$, $R_7$, $R_8$, $R'_1$, $R'_2$, $R'_3$, and $R'_4$ are as defined in formula (2a); and X and Y are as defined above,
that is produced by reacting a hexadepsipeptide represented by formula (4a):

[Chemical formula 25]

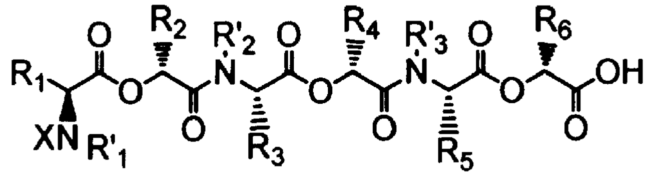

(4a)

wherein $R_1$, $R_2$, $R_3$, $R_4$, $R_5$, $R_6$, $R'_1$, $R'_2$, and $R'_3$ are as defined in formula (2a); and X is as defined above, with a didepsipeptide represented by formula (5a):

[Chemical formula 26]

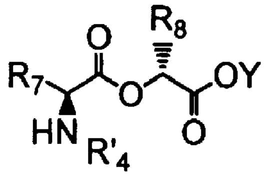

(5a)

wherein $R_7$, $R_8$, and $R'_4$ are as defined in formula (2a); and Y is as defined above, using a suitable condensing agent in the presence of a basic reaction assistant after the addition of a suitable solvent, and
removing N-terminal protecting group X and C-terminal protecting group Y from the compound represented by formula (3a) under suitable deprotection conditions.

Hexadepsipeptides represented by formula (4a) may be synthesized by the above processes, or alternatively may be produced by reacting didepsipeptides represented by formulae (11), (12), (9), (5) or the like under suitable conditions. Further, hexadepsipeptides represented by formula (4a) may be produced, for example, using verticilide-producing microorganisms, verticilide analogue-producing microorganisms, or microorganisms obtained by introducing a part or the whole of biosynthesized genes obtained from these producing microorganisms.

Didepsipeptides represented by formula (5a) can be synthesized by the above process.

Harmful organism control agents

Compounds of formula (1) can be used as the harmful organism control agent.

The term "harmful organism (pest) control agent" as used herein means medicaments that exert control effect against harmful organisms and embraces medicaments that kill harmful organisms, as well as medicaments that inhibit propagation of harmful organisms.

Examples of insect pest species against which harmful organism control agents containing at least one of compounds represented by formula (1) exert control effect are as follows.

Agricultural and horticultural insect pest species include, for example, Lepidopteran insect pests, for example, Spodoptera litura, Mamestra brassicae, Pseudaletia separata, green caterpillar, Plutella xylostella, Spodoptera exigua, Chilo suppressalis, Cnaphalocrocis medinalis, Tortricidae, Carposinidae, Lyonetiidae, Lymantriidae; insect pests belonging to the genus Agrotis spp., insect pests belonging to the genus Helicoverpa spp.; insect pests belonging to the genus Heliothis spp. and the like; Hemipteran insect pests, for example, Aphididae, Adelgidae or Phylloxeridae, for example, Myzus persicae, Aphis gossypii Glover, or Rhopalosiphum padi, Deltocephalidae such as Nephotettix cincticeps and Tea green leafhopper, Delphacidae such as Laodelphax striatellus, Nilaparvata lugens, or Sogatella furcifera, Pentatomidae such as Eysarcoris ventralis, Nezara viridula, or Trigonotylus caelestialium, Aleyrodidae such as Bemisia argentifolii, Bemisia tabaci or Trialeurodes vaporariorum, Coccoidea such as Pseudococcus comstocki, Planococcus citri Risso, Pseudaulacaspis prunicola, or Aonidiella aurantii (for example, Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae); Coleoptera insect pests, for example, Lissorhoptrus oryzophilus, Callosobruchus chinensis, Tenebrio molitor, Diabrotica virgifera virgifera, Diabrotica undecimpunctata howardi, Anomala cuprea, Anomala

rufocuprea, Phyllotreta striolata, Aulacophora femoralis, Leptinotarsa decemlineata, Oulema oryzae, Grapholita molesta, or Cerambycidae; Acari, for example, Tetranychidae such as Tetranychus urticae or Tetranychus kanzawai Kishida belonging to the genus Tenranychus, Panonychus citri McGregor or Panonychus ulmi belonging to the genus Panonychus, rust mites such as Aculops lycopersici or Aculops pelekassi, and Tarsonemidae such as Polyphagotarsonemus latus; Hymenopteran insect pests, for example, Tenthredinoidae; Orthopteran insect pests, for example, Acrididae; Dipteran insect pests, for example, Agromyzidae; Thysanopteran insect pests, for example, Thrips palmi KARNY, Frankliniella occidentalis, or Scirtothrips dorsalis; and Plant Parasitic Nematodes, for example, Meloidogyne hapla, Pratylenchus, Aphelenchoides besseyi, or Bursaphelenchus xylophilus. Preferred are Lepidopteran insect pests, Hemipteran insect pests, Thysanopteran insect pests, and Acarina insect pests. More preferred are Hemipteran insect pests, Thysanopteran insect pests, and Acarina insect pests.

Examples of zoobiotic insect pests include ticks, for example, Lone star tick (Amblyomma americanum), Gulf coast tick (Amblyomma maculatum), Tropical cattle tick (Boophilus microplus), Rocky Mountain wood tick (Dermacentor andersoni), Pacific Coast tick (Dermacentor occidentalis), American dog tick (Dermacentor variabilis), Haemaphysalis campanulata, Haemaphysalis flava, Bush tick (Haemaphysalis longicornis), Haemaphysalis megaspinosa, Ixodes nipponensis, Ixodes ovatus, Western black-legged tick (Ixodes pacifcus), Taiga tick (Ixodes persulcatus), Castor bean tick (Ixodes ricinus), Black-legged tick (Ixodes scapularis), Soft tick (Ornithodoros moubata), and Brown dog tick (Rhipicephalus sanguineus); Cheyletidae, for example, Cheyletiella blakei and Cheyletiella yasguri; Demodex folliculorum, for example, Dog follicle mite (Demodex canis), and Cat follicle mite (Demodex cati); Psoroptidae for example, Psoroptic mite (Psoroptes communis); Sarcoptes spp., for example, Chorioptic mange mite (Chorioptes bovis), and Dog ear mite (Otodectes cynotis); Dermanyssidae, for example, Northern fowl mite (Ornithonyssus sylviarum); Dermanyssus spp.; Analgidae, for example, Feather mite (Megninia cubitalis), and Feather mite (Pterolichus obtusus); Leptotrombidium, for example, Trombiculid mite (Helenicula miyagawai), Leptotrombidium akamushi; Siphonaptera, for example, Cat flea (Ctenocephalides felis), Pulex irritans, Oriental rat flea (Xenopsylla cheopis) and Xenopsylla; biting lice (Mallophaga), for example, Dog biting louse (Trichodectes canis) and Chicken shaft louse (Menopon gallinae); sucking lice (Anoplura), for example, Large pig louse (Haematopinus suis), Dog sucking louse (Linognathus setosus), Pediculus humanus corporis, Pediculus humanus humanus, Phthirus pubis, and Cimex lectularius; Muscidae; warble botfly (Hypoderma spp.); Stomoxys calcitrans; Gastrophilus spp.; Psychodidae, for example, Sergentomyia squamirostri, tsetse fly (Glossina spp.), Aphididae; Aedes, for example, Stegomyia albopicta and Yellow fever mosquito (Aedes aegypti); Culex, for example, House mosquito (Culex pipiens pallens); Anopheles; Ceratopogonidae; Simuliidae; reduvid; Pharaoh ant; Nematoda, for example, Strongyloides, hookworms, Strongylida, for example, Haemonchus spp, and Nippostrongylus brasiliensis, Trichostrongyloidea, Metastrongyloidea, for example, Metastrongylus apr, Anriostrongylus cantonesis, and Aelurostrongylsabstrusus), Oxyuroidea, Haterakoidea, for example, Ascaridiidae galli, Ascaridoidea, for example, Anisakis simplex, Ascaris lumbricoides suum, Parascaris equorum, Toxocara canis, and Toxocara cati, Spirurida, for example, Subuluroidea, Gnathostoma spinigerum, Physaloptea praeputialis, Ascarops strongylina, Draschia megastoma, Ascaria hamulosa, and Guinea worm (Dracunculus medinensis), Filariida, for example, Dog heartworm (Dirofilaria immitis), Wuchereriabancrofti, Onchocerca volvulus, and African eye worm (Loa loa), Dioctophymatoidea, Trichinelloidea, for example, Trichuris vulpis and Trichinella spiralis; Trematoda, for example, Schistosoma japonicum and Fasciola spp.; Acanthocephala; Cestoda, for example, pseudo leaves (Pseudophyllidea), for example, Spirometra erinaceieuropaei, Cyclophyllidea, for example, Dipylidium caninum; and Protozoa.

Examples of sanitary insect pests, offensive insect pests, stored grain insect pests, stored product insect pests, and house insect pests include mosquitoes, for example, Asian tiger mosquito (Aedes albopictus), and House mosquito (Culex pipiens pallens); cockroaches (Blattaria), for example, Smoky- brown cockroach (Periplaneta fuliginosa), Japanese cockroach (Periplaneta japonica), German cockroach (Blattella germanica); Acaridae, for example, Cheese mite (Tyrophagus putrescentiae); Diptera, for example, Housefly (Musca domestica), Sarcophaga spp., Psychodidae, Drosophilidae, and Chironomidae; Simuliidae; Ceratopogonidae, Hymenoptera, for example, Simuliidae such as Japanese carpenter ant (Camponotus japonicus) and Fire ant (Solenopsis spp.) and Hymenoptera such as Asian giant hornet (Vespa mandarina); arthropod belonging to Isopoda, for example, Common rough woodlouse (Porcellio scaber), Ligia exotica, and Armadillidium vulgare; Hemiptera insects, for example, Bed bug (Cimex lectularius); arthropod belonging to Myriapoda, for example, centipede, Scutigeromorpha, and Diplopoda (millipedes); arthropod belonging to Araneae, for example, Heteropoda venatoria; Coleoptera insects, for example, Anisodactylus signatus; arthropod belonging to Collembola, for example, Onychiurus folsomi; Dermaptera insects, for example, Labidura riparia; Orthroptera insects, for example, Stenopelmatidae; Coleoptera insects, for example, Callosobruchus chinensis, Sitophilus zeamais, Tenebroides mauritanicus, Tribolium castaneum, Ptinidae, Anobiidae, Limnoria tripunctata, Dermestidae, and Chlorophorus diadema (Motschulsky), Lepidoptera insects, for example, Pyralidae, and tineo; Hemipeplidae; Isoptera insects, for example, Coptotermes formosanus, Incisitermes minor (Hagen), and Odontotermes formosanus; and Thysanura, for example, Ctenolepisma villosa.

Insect pest species to which the harmful organism control agent of the present invention are applied are preferably agricultural and horticultural insect pests and zoobiotic insect pests, more preferably agricultural and horticultural

insect pests selected from Lepidoptera insect pests, Hemiptera insect pests, Order Thysanoptera insect pests, and Acari insect pests, or zoobiotic insect pests selected from zoobiotic nematode and zoobiotic ascarid, still more preferably Lepidoptera insect pests, Hemiptera insect pests, Order Thysanoptera insect pests, or Acari insect pests, specifically Plutella xylostella, Spodoptera litura, Aphis gossypii, Myzus persicae, Rhopalosiphum padi, Trialeurodes vaporariorum, Nephotettix cincticeps, Frankliniella occidentalis, Trigonotylus caelestialium, Tetranychus urticae Koch, Tetranychus kanzawai Kishida, Panonychus citri McGregor, or Aculops lycopersici, even more preferably Hemiptera insect pests, Order Thysanoptera insect pests, or Acari insect pests, specifically Aphis gossypii Glover, Rhopalosiphum padi, Trialeurodes vaporariorum, Trigonotylus caelestialium, Frankliniella occidentalis, Tetranychus urticae Koch, Tetranychus kanzawai Kishida, Panonychus citri McGregor, and Aculops lycopersici.

Examples of harmful organism control agents provided by the present invention include agricultural and horticultural harmful organism control agents, for example, agricultural and horticultural insecticides, veterinary parasite control agents, for example, veterinary endoparasitic control agents and veterinary ectoparasitic control agents, sanitary insect pest control agents, offensive insect pest control agents, tored grain and stored food insect pest control agents, and house insect pest control agents. Preferred are agricultural and horticultural insecticides.

The harmful organism control agent according to the present invention can also be prepared using compounds represented by formula (1) and carriers depending upon use thereof.

When the harmful organism control agent according to the present invention is an agricultural and horticultural harmful organism control agent, the above compounds are generally mixed with suitable solid carriers, liquid carriers, gaseous carriers, surfactants, dispersants, or other adjuvants for preparations and formulated into any suitable dosage forms, for example, emulsifiable concentrates, liquid formulations, suspensions, wettable powders, floables, dusts, granules, tablets, oils, aerosols or smoking agents.

Solid carriers include, for example, talc, bentonite, clay, kaolin, diatomaceous earth, vermiculite, white carbon, and calcium carbonate.

Liquid carriers include, for example, alcohols such as methanol, n-hexanol, and ethylene glycol; ketones such as acetone, methyl ethyl ketone and cyclohexanone; aliphatic hydrocarbons such as n-hexane, kerosine and kerosene; aromatic hydrocarbons such as toluene, xylene and methylnaphthalene; ethers such as diethyl ether, dioxane and tetrahydrofuran; esters such as ethyl acetate; nitriles such as acetonitrile and isobutyronitrile; acid amides such as dimethylformamide and dimethylacetamide; vegetable oils such as soy bean oil and cotton seed oil; dimethylsulfoxide; and water.

Gaseous carriers include, for example, LPG, air, nitrogen, carbon dioxide, and dimethyl ether.

Surfactants or dispersants usable, for example, for emulsifying, dispersing, or spreading include, for example, alkylsulfuric esters, alkyl(aryl)sulfonic acid salts, polyoxyalkylene alkyl(aryl) ethers, polyhydric alcohol esters, and lignin sulfonic acid salts.

Adjuvants usable for improving the properties of preparations include, for example, carboxymethylcellulose, gum arabic, polyethylene glycol and calcium stearate.

The above carriers, surfactants, dispersants and adjuvants may be used either solely or in combination according to need.

The content of the active ingredient in the preparation is not particularly limited, but is generally 1 to 75% by weight for emulsifiable concentrate, 0.3 to 25% by weight for dust, 1 to 90% by weight for wettable powder and 0.5 to 10% by weight for granules.

Compounds represented by formula (1), preparations containing them, and admixtures thereof with other harmful organism control agents can be applied to harmful insects, plants, and plant propagation materials, for example, seeds, plant foliages, roots, germinated plants, and embryo plants, soil, nutrient solutions in nutriculture, solid media in nutriculture, and room where the entry of pests should be prevented.

The application thereof may be carried out before and after the entry of insect pests.

In particular, compounds represented by formula (1), preparations containing them, and admixtures thereof with other harmful organism control agents can control harmful organisms by applying an effective amount thereof to an object selected from the group consisting of seeds, roots, tubers, bulbs, and rhizomes of plants, germinated plants, embryo plants, soil, nutrient solutions in nutricultures, solid media in nutricultures, and systematically translocating the compound of formula (1) into the plant.

When the object is a seed, root, tuber, bulb, or rhizome of a plant, any application method that does not inhibit systemic translocation can be adopted without particular limitation, and examples of suitable application methods include dipping, dust coating, smearing, spraying, pelleting, or coating.

When the object is a seed, application methods usable herein include dipping, dust coating, smearing, spraying, pelleting, coating, and fumigating. The dipping is a method in which seeds are dipped in a chemical solution. The dust coating is classified into two types, i.e., a dry dust coating method in which a powdery chemical is adhered onto dry seeds, and a wet dust coating method in which a powdery chemical is adhered onto seeds which have been lightly soaked in water. The smearing is a method in which a suspended chemical is coated on the surface of seeds within a

mixer. The spraying is a method in which a suspended chemical is sprayed onto the surface of seeds. The pelleting is a method in which a chemical is mixed with a filler when seeds, together with the filler, are pelleted to form pellets having given size and shape. The coating is a method in which a chemical-containing film is coated onto seeds. The fumigating is a method in which seeds are sterilized with a chemical which has been gasified within a hermetically sealed vessel.

Compounds represented by formula (1), preparations containing them, and admixtures thereof with other harmful organism control agents can also be applied to, in addition to seeds, germinated plants which are transplanted after germination or after budding from soil, and embryo plants. These plants can be protected by the treatment of the whole or a part thereof by dipping before transplantation.

In application, for example, to seeds, roots, tubers, bulbs, or rhizomes, the seeds, roots, tubers, bulbes, or rhizomes may be planted or dipped for a period of time enough to allow the drug to be systematically translocate. The time and temperature in dipping may be properly determined by a person having ordinary skill in the art depending, for example, upon the object to be applied and the type and amount of the chemical. The systemic translocation time is not particularly limited and may be, for example, one hr or longer. The temperature in the systemic translocation is, for example, 5 to 45°C.

An example of the method for application to soil is a method in which granules of compounds of the present invention, preparations containing them, and admixtures thereof with other harmful organism control agents are applied into soil or on soil. Preferred methods for application to soil include spreading, stripe application, groove application, and planting hole application. The spreading includes surface treatment over the whole area to be treated, and mechanical introduction into soil following the surface treatment.

Drenching of soil with a solution prepared by emulsifying or dissolving derivatives of the present invention, preparations containing them, and admixtures thereof with other harmful organism control agents in water is also an advantageous soil application method.

In application to a nutrient solution in nutrient solution culture systems such as water culture and solid medium culture, for example, sand culture, NFT (nutrient film technique), or rock wool culture, for the production of vegetables and flowering plants, compounds of the present invention, preparations containing them, and admixtures thereof with other harmful organism control agents can be applied directly to artificial culture soil containing vermiculite and a solid medium including an artificial mat for raising seedling.

In the application step, the effective amount of the compound of formula (1) or salt thereof is preferably an amount large enough to allow the compound of formula (1) to be systemically translocated into the plant in the subsequent systemic translocation step.

The effective amount can be properly determined by taking into consideration, for example, the properties of compounds, the type and amount of the application object, the length of the subsequent systemic translocation step, and the temperature. For example, in the case of seeds, the compound of formula (1) or salt thereof is applied in an amount of preferably 1 g to 10 kg, more preferably 10 g to 1 kg, per 10 kg of seeds. On the other hand, in application to soil, the compound of formula (1) or salt thereof is applied in an amount of preferably 0.1 g to 10 kg, more preferably 1 g to 1 kg, per 10 ares of cultivated land. In treatment of foliage of plants, the compound of formula (1) or salt thereof is applied in an amount of preferably 0.1 g to 10 kg, more preferably 1 g to 1 kg, per 10 ares of cultivated land.

When the harmful organism control agent of the present invention is a zoobiotic insect pest control agent, the zoobiotic insect pest control agent is provided, for example, in liquid formulations, emulsifiable concentrates, liquefied drops, sprays, foam formulations, tablets, granules, fine subtilaes, dust, capsules, tablets, chewable formulations, injections, suppositories, creams, shampoos, rinses, resin agents, fumigants, and poison baits. Among them, liquid formulations and liquefied drops are particularly preferred.

Adjuvants for preparations such as conventional emulsifiers, dispersants, spreaders, wetting agents, suspending agents, preserving agents, and propellants and the like may be further incorporated in the liquid formulation. Further, conventional coating film forming agents may be incorporated. Surfactants for emulsification, dispersion, spreading or the like include, for example, soaps, polyoxyalkylene alkyl (aryl) ethers, polyoxyethylene alkyl allyl ethers, polyoxyethylene fatty acid esters, higher alcohols, and alkylarylsulfonates. Dispersants include, for example, casein, gelatin, polysaccharides, lignin derivatives, saccharides, and synthetic water soluble polymers. Spreaders/wetting agents include, for example, glycerin and polyethylene glycol. Suspending agents include, for example, casein, gelatin, hydroxypropylcellulose, and gum arabic. Stabilizers include, for example, phenolic antioxidants such as BHT and BHA, amine antioxidants such as diphenylamine, and organic sulfur antioxidants. Preserving agents include, for example, methyl p-oxybenzoate, ethyl p-oxybenzoate, propyl p-oxybenzoate, and butyl p-oxybenzoate. The carriers, surfactants, dispersants, and adjuvants may if necessary be used solely or in a combination of two or more. Perfumes, synergists and the like may also be incorporated. The content of the active ingredients in the harmful organism control agent of the present invention is generally 1 to 75% by weight for the liquid formulation.

Carriers usable for the preparation of creams include, for example, nonvolatiel hydrocarbons such as liquid paraffin, lanolin hydrogenated fats and oils, higher fatty acids, fatty acid esters, animal and vegetable oils, silicone oils, and water. The emulsifiers, humectant, antioxidants, perfumes, borax, and ultraviolet absorbers may if necessary be

used solely or in a combination of two or more. Emulsifiers include, for example, fatty acid sorbitan, polyoxyethylene alkyl ethers, and fatty acid polyoxyethylene. The content of the active ingredients in the composition for controlling ectoparasiticides according to the present invention is generally 0.5 to 70% by weight for the cream.

The capsules, pills or tablets may be used in such a manner that the active ingredients in the composition according to the present invention are divided into suitable small portions, the small portion is mixed with a diluting solution or a carrier such as starch, lactose, or talc, a disintegrator such as magnesium stearate and/or a binder is further added thereto, and, if necessary, the mixture is tabletted.

Injections should be prepared as an aseptic solution. The injection may contain other substances, for example, a salt or glucose in an amount enough to be isotonicated with blood. Carriers usable for injection preparation include organic solvents, for example, esters such as glycerides, benzyl benzoate, isopropyl myristate, and fatty acid derivatives of propylene glycol, N-methylpyrrolidone, and glycerol formal. The content of the active ingredients in the harmful organism control agent according to the present invention is generally 0.01 to 10% by weight for the injection.

Carriers usable for the preparation of resin agents include, for example, vinyl chloride polymers, and polyurethane. Plasticizers such as phthalic acid esters, adipic acid esters, and stearic acid may if necessary be added to these bases. After kneading of the active ingredients according to the present invention into the base, the kneaded product is molded, for example, by injection molding, extrusion, or press molding. The molded product may also be further properly subjected to molding, cutting or the like to obtain an ear tag for animals or insecticidal collar for animals.

Carriers usable for toxic baits include bait substances and attractants, for example, farina such as wheat flour and corn flour, starch such as corn starch and potato starch, saccharides such as granulated sugar, malt sugar, and honey, food flavors such as glycerin, onion flavor, and milk flavor, animal powders such as pupal powder and fish powder, and various pheromones. The content of the active ingredients in the composition for controlling ectoparasiticides according to the present invention is generally 0.0001 to 90% by weight for the toxic bait.

The harmful organism control agent according to the present invention can control harmful organisms, for example, by oral or injection administration into the body of an object animal, administration onto the whole or a part of the body surface of an object animal, and covering of a place where invasion, parasitism, and migration of harmful organisms are expected.

The harmful organism control agent according to the present invention as such may be used, or alternatively may be diluted with water, a liquid carrier, a commercially available shampoo, a rinse, a feed, an underlayment for animal-rearing houses or the like.

Further, the harmful organism control agent according to the present invention may also be used as admixtures with other bactericides, insecticides, miticides or tickicides, herbicides, plant growth-regulating agents, and fertilizers.

Such agents usable as admixtures include those described in The Pesticide Manual, 13th edition, published by The British Crop Protection Council; and SHIBUYA INDEX, the 13th edition, 2008, published by SHIBUYA INDEX RESEARCH GROUP.

More specific examples of insecticides, miticides or tickicides, or nematicides include: organic phosphoric acid ester-based compounds such as acephate, dichlorvos, EPN, fenitothion, fenamifos, prothiofos, profenofos, pyraclofos, chlorpyrifos-methyl, diazinon, fosthiazate, and imicyafos; carbamate compounds such as methomyl, thiodicarb, aldicarb, oxamyl, propoxur, carbaryl, fenobucarb, ethiofencarb, fenothiocarb, pirimicarb, carbofuran, and benfuracarb; nereistoxin derivatives such as cartap, and thiocyclam; organic chloro compounds such as dicofol, and tetradifon; pyrethroid-based compounds such as permethrin, tefluthrin, cypermethrin, deltamethrin, cyhalothrin, fenvalerate, fluvalinate, ethofenprox, and silafluofen; benzoyl urea-based compounds such as diflubenzuron, teflubenzuron, flufenoxuron, and chlorfluazuron; juvenile hormone-like compounds such as methoprene; and molting hormone-like compounds such as chromafenozide. Other compounds include buprofezin, hexythiazox, amitraz, chlordimeform, pyridaben, fenpyroxymate, pyrimidifen, tebufenpyrad, tolfenpyrad, fluacrypyrim, acequinocyl, cyflumetofen, flubendizmide, ethiprole, fipronil, ethoxazole, imidacloprid, clothianidin, thiamethoxam, acetamiprid, nitenpyram, thiacloprid, dinotefuran, pymetrozine, bifenazate, spirodiclofen, spiromesifen, flonicamid, chlorfenapyr, pyriproxyfen, indoxacarb, pyridalyl, or spinosad, avermectin, milbemycin, cyenopyrafen, spinetoram, pyrifluquinazon, chlorantraniliprole, cyantraniliprole, cyclaniliprole, spirotetramat, lepimectin, metaflumizone, pyrafluprole, pyriprole, hydramethylnon, triazamate, sulfoxaflor, flupyradifurone, flometoquin, afidopyropen, pyflbumide, organic metal-based compounds, dinitro-based compounds, organic sulfur comounds, urea-based comounds, triazine-based comounds, hydrazine-based comounds, amine derivatives represented by the following formula or acid addition salts thereof:

[Chemical formula 28]

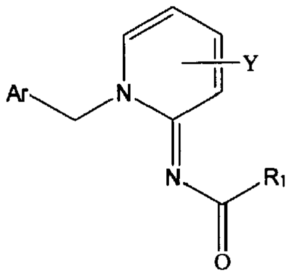

wherein
Ar represents a halogen atom, hydroxyl or $C_{1-6}$ alkyl optionally substituted by a halogen atom, $C_{1-6}$ alkyloxy optionally substituted by a halogen atom, pyridyl optionally substituted by cyano or nitro; or a halogen atom, $C_{1-4}$ alkyl optionally substituted by a halogen atom, $C_{1-4}$ alkyloxy optionally substituted by a halogen atom, pyrimidyl optionally substituted by hydroxyl, cyano, or nitro,
Y represents a hydrogen atom; a halogen atom; hydroxyl; $C_{1-6}$ alkyl optionally substituted by a halogen atom; $C_{1-6}$ alkyloxy optionally substituted by a halogen atom; cyano; or nitro, and $R_1$ represents $C_{1-6}$ alkyl substituted by a halogen atom, and
amine derivatives represented by the following formula and acid addition salts tthereof

[Chemical formula 29]

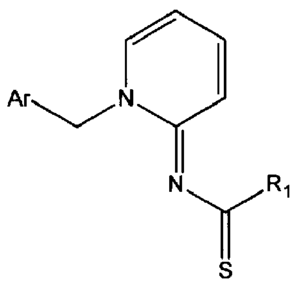

wherein
Ar represents a halogen atom, hydroxyl or $C_{1-6}$ alkyl optionally substituted by a halogen atom, $C_{1-6}$ alkyloxy optionally substituted by a halogen atom, or pyridyl optionally substituted by cyano or nitro; or a halogen atom, $C_{1-4}$ alkyl optionally substituted by a halogen atom, $C_{1-4}$ alkyloxy optionally substituted by a halogen atom, or pyrimidyl optionally substituted by hydroxyl, cyano, or nitro, and
$R_1$ represents $C_{1-6}$ alkyl substituted by a halogen atom.

The harmful organism control agent according to the present invention may also be used as admixtures or in combination with microbial pesticides such as BT formulations and insect pathological viral agents.

Examples of bactericides usable as admixtures or in combination include: strobilurin-based compounds such as azoxystrobin, kresoxym-methyl, trifloxystrobin, metominostrobin, and orysastrobin; anilinopyrimidine-based compounds such as mepanipyrim, pyrimethanil, and cyprodinil; azole-based compounds such as triadimefon, bitertanol, triflumizole, metoconazole, propiconazole, penconazole, flusilazole, myclobutanil, cyproconazole, tebuconazole, hexaconazole, prochloraz, and simeconazole; quinoxaline-based compounds such as quinomethionate; dithiocarbamate-based compounds such as maneb, zineb, mancozeb, polycarbamate, and propineb; phenylcarbamate-based compounds such as diethofencarb; organic chloro compounds such as chlorothalonil and quintozene; benzimidazole-based compounds such as benomyl, thiophanate-methyl, and carbendazole; phenylamido-based compounds such as metalaxyl, oxadixyl, ofurase, benalaxyl, furalaxyl, and cyprofuram; sulfenic acid-based compounds such as dichlofluanid; copper-based compounds such as copper hydroxide and oxine-copper; isoxazole-based compounds such as hydroxyisoxazole; organic phosphorus compounds such as fosetyl-aluminium and tolclofos-methyl; N-halogenothioalkyl-based compounds such as captan, captafol, and folpet; dicarboxyimide-based compounds such as procymidone, iprodione, and vinchlozolin; caboxyanilide-based compounds such as flutolanil, mepronil, furamepyr, thifluzamide, boscalid, and penthiopyrad; morpholine-based compounds such as fenpropimorph, and dimethomorph; organic tin compounds such as fenthin hydroxide, and fenthin acetate; cyanopyrrole-based compounds such as fludioxonil and fenpiclonil; and other

compounds such as tricyclazole, pyroquilon, carpropamid, diclocymet, fenoxanil, fthalide, fluazinam, cymoxanil, triforine, pyrifenox, fenarimol, fenpropidin, pencycuron, ferimzone, cyazofamid, iprovalicarb, benthiavalicarb-isopropyl, iminoctadin-albesilate, cyflufenamid, kasugamycin, validamycin, streptomycin, oxolinic-acid, tebufloquin, probenazole, tiadinil, isotianil, and tolprocarb.

In a preferred embodiment of the present invention, compounds represented by formula (1) can be used as Hemipteran insect pest control agents (preferably Aphididae control agents). In this case, preferred compounds of formula (1) are compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are those indicated by compound Nos. 2, 17, 20, 32, 33, 34, 36, 48, 49, 51, 52, 53, 54, 57, 58, 60, 61, 62, 64, 66, and 67 in Table 1.

In a preferred embodiment of the present invention, compounds represented by formula (1) can be used as Acarina insect pest control agents (preferably Tetranychidae (more preferably Tetranychidae belonging to the genus Tetranycus and Tetranychidae belonging to the genus Panonychus) or rust mite control agents). Acarina insect pests can be effectively controlled even when they are resistant to drugs. In this case, preferred compounds of formula (1) are compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are those indicated by compound Nos. 53, 55, 56, 57, 58, 59, 60, and 68 in Table 1.

In a preferred embodiment of the present invention, compounds represented by formula (1) can be used as veterinary parasite control agents. In this case, preferred compounds of formula (1) are compounds of formula (1) wherein combinations of $R_1$, $R_2$, $R_3$, $R_4$, $R_5$, $R_6$, $R_7$, $R_8$, $R'_1$, $R'_2$, $R'_3$, and $R'_4$ are those indicated by compound Nos. 1, 4, 5, 10, 21, 27, 54, 57, 58, and 61 in Table 1.

According to the present invention, there is provided a method for controlling agricultural and horticultural harmful organisms, the method comprising applying an effective amount of a compound represented by formula (1) to agricultural and horticultural harmful organisms or a habitat thereof. According to the method for controlling agricultural and horticultural harmful organisms, an effective amount of a compound represented by formula (1) can be applied to agricultural and horticultural harmful organisms, plants, and plant propagation materials, for example, seeds, plant foliages, roots, germinated plants, and embryo, soil, nutrient solutions in nutriculture, solid media in nutriculture, and room where the entry of pests should be prevented. In a preferred embodiment of the present invention, the control method is a non-therapeutic method.

According to the present invention, there is provided a method for controlling a veterinary parasite, the method comprising applying an effective amount of a compound represented by formula (1) to a veterinary parasite or a habitat thereof. According to the present invention, there is provided a method for controlling a veterinary parasite, the method comprising administering an effective amount of a compound represented by formula (1) into the body of an object animal through oral administration or injection, or administering an effective amount of a compound represented by formula (1) onto the whole or a part of the body surface of an object animal. According to the present invention, there is provided a method for controlling a veterinary parasite, the method comprising administering an effective amount of a compound represented by formula (1) to a place where invasion, parasitism, and migration of harmful organisms are expected. In a preferred embodiment of the present invention, the control method is a non-therapeutic method.

According to the present invention, there is provided use of a compound represented by formula (1), for the production of agricultural and horticultural harmful organism control agents.

According to the present invention, there is provided use of a compound represented by formula (1), for the production of veterinary parasite control agents.

According to the present invention, there is provided a compound represented by formula (1) for use as an agricultural and horticultural harmful organism control agent.

According to the present invention, there is provided a compound represented by formula (1) for use as a veterinary parasite control agent.

According to the present invention, there are also provided the following inventions.

(1) A compound represented by formula (1) or a stereoisomer thereof:

[Chemical formula 30]

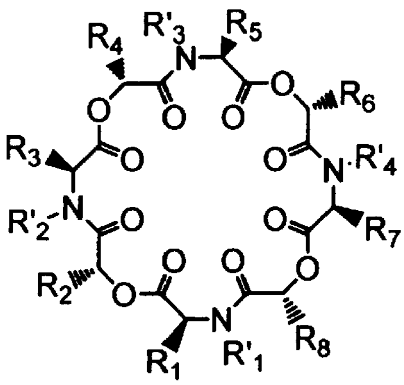

(1)

wherein

$R_1$, $R_2$, $R_3$, $R_4$, $R_5$, $R_6$, $R_7$, and $R_8$, which may be the same as or different from each other, represent a hydrogen atom, optionally substituted straight-chain, branched, or cyclic alkyl having 1 to 9 carbon atoms, or optionally substituted alkoxyalkyl having 2 to 9 carbon atoms and

$R'_1$, $R'_2$, $R'_3$, and $R'_4$, which may be the same as or different from each other, represent a hydrogen atom, methyl, or ethyl, excluding compounds wherein $R_1$, $R_3$, $R_5$, and $R_7$ represent isopropyl or isobutyl when all of $R_2$, $R_4$, $R_6$, and $R_8$ represent isopropyl; compounds wherein all of $R_1$, $R_3$, $R_5$, and $R_7$ represent methyl when all of $R_2$, $R_4$, $R_6$, and $R_8$ represent n-pentyl; and compounds wherein all of $R_1$, $R_2$, $R_3$, $R_4$, $R_5$, $R_6$, $R_7$, and $R_8$ represent methyl.

(2) The compound according to (1) or a stereoisomer thereof, wherein $R_1$, $R_3$, $R_5$, and $R_7$, which may be the same as or different from each other, represent a hydrogen atom, straight-chain or branched alkyl having 1 to 4 carbon atoms and $R_2$, $R_4$, $R_6$, and $R_8$, which may be the same as or different from each other, represent straight-chain alkyl having 1 to 9 carbon atoms.

(3) The compound according to (1) or a stereoisomer thereof, wherein $R_1$, $R_3$, $R_5$, and $R_7$, which may be the same as or different from each other, represent a hydrogen atom or straight-chain or branched alkyl having 1 to 4 carbon atoms and $R_2$, $R_4$, $R_6$, and $R_8$, which may be the same as or different from each other, represent straight-chain alkyl having 4 to 8 carbon atoms.

(4) The compound according to (1) or a stereoisomer thereof, wherein $R_1$, $R_3$, $R_5$, and $R_7$ each represent a hydrogen atom or straight-chain or branched alkyl having 1 to 4 carbon atoms, provided that $R_1$, $R_3$, and $R_5$ are the same as each other while $R_7$ is different from $R_1$, $R_3$, and $R_5$; $R_2$, $R_4$, $R_6$, and $R_8$ each represent straight-chain alkyl having 4 to 8 carbon atoms, provided that $R_2$, $R_4$, and $R_6$ are the same as each other while $R_8$ may be the same as or different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other, or

$R_1$, $R_3$, $R_5$, and $R_7$ each represent a hydrogen atom or straight-chain or branched alkyl having 1 to 4 carbon atoms, provided that $R_1$, $R_3$, and $R_5$ are the same as each other while $R_7$ may be the same as or different from $R_1$, $R_3$, and $R_5$; $R_2$, $R_4$, $R_6$, and $R_8$ each represent straight-chain alkyl having 4 to 8 carbon atoms, provided that $R_2$, $R_4$, and $R_6$ are the same as each other while $R_8$ is different from $R_2$, $R_4$, and $R_6$; and $R'_1$, $R'_2$, $R'_3$, and $R'_4$ each represent a hydrogen atom, methyl, or ethyl, provided that $R'_1$, $R'_2$, $R'_3$, and $R'_4$ are the same as each other.

(5) The compound according to (1) or a stereoisomer thereof, wherein $R_1$, $R_3$, $R_5$, and $R_7$ each represent a hydrogen atom or straight-chain or branched alkyl having 1 to 4 carbon atoms, provided that $R_1$ and $R_3$ are the same as each other and $R_5$ and $R_7$ are the same as each other while $R_1$ and $R_3$ are different from $R_5$ and $R_7$; $R_2$, $R_4$, $R_6$, and $R_8$ each represent straight-chain alkyl having 4 to 8 carbon atoms, provided that $R_2$ and $R_4$ are the same as each other and $R_6$ and $R_8$ are the same as each other while $R_2$ and $R_4$ may be the same as or different from $R_6$ and $R_8$; and $R'_1$, $R'_2$, $R'_3$, and $R'_4$ each represent a hydrogen atom, methyl, or ethyl, provided that $R'_1$ and $R'_2$ are the same as each other and $R'_3$ and $R'_4$ are the same as each other while $R'_1$ and $R'_2$ may be the same as or different from $R'_3$ and $R'_4$,

$R_1$, $R_3$, $R_5$, and $R_7$ each represent a hydrogen atom or straight-chain or branched alkyl having 1 to 4 carbon atoms, provided that $R_1$ and $R_3$ are the same as each other and $R_5$ and $R_7$ are the same as each other while $R_1$ and $R_3$ may be the same as or different from $R_5$ and $R_7$; $R_2$, $R_4$, $R_6$, and $R_8$ each represent straight-chain alkyl having 4 to

8 carbon atoms, provided that $R_2$ and $R_4$ are the same as each other and $R_6$ and $R_8$ are the same as each other while $R_2$ and $R_4$ are different from $R_6$ and $R_8$; and $R'_1$, $R'_2$, $R'_3$, and $R'_4$ each represent a hydrogen atom, methyl, or ethyl, provided that $R'_1$ and $R'_2$ are the same as each other and $R'_3$ and $R'_4$ are the same as each other while $R'_1$ and $R'_2$ may be the same as or different from $R'_3$ and $R'_4$, or

$R_1$, $R_3$, $R_5$, and $R_7$ each represent a hydrogen atom or straight-chain or branched alkyl having 1 to 4 carbon atoms, provided that $R_1$ and $R_3$ are the same as each other and $R_5$ and $R_7$ are the same as each other while $R_1$ and $R_3$ may be the same as or different from $R_5$ and $R_7$; $R_2$, $R_4$, $R_6$, and $R_8$ each represent straight-chain alkyl having 4 to 8 carbon atoms, provided that $R_2$ and $R_4$ are the same as each other and $R_6$ and $R_8$ are the same as each other while $R_2$ and $R_4$ may be the same as or different from $R_6$ and $R_8$; and $R'_1$, $R'_2$, $R'_3$, and $R'_4$ each represent a hydrogen atom, methyl, or ethyl, provided that $R'_1$ and $R'_2$ are the same as each other and $R'_3$ and $R'_4$ are the same as each other while $R'_1$ and $R'_2$ are different from $R'_3$ and $R'_4$.

(6) The compound according to (1) or a stereoisomer thereof, wherein $R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ are different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ may be the same as or different from $R_4$ and $R_8$; and $R'_1$, $R'_2$, $R'_3$, and $R'_4$ each represent a hydrogen atom, methyl, or ethyl, provided that $R'_1$ and $R'_3$ are the same as each other and $R'_2$ and $R'_4$ are the same as each other while $R'_1$ and $R'_3$ may be the same as or different from $R'_2$ and $R'_4$,

$R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ are different from $R_4$ and $R_8$; and $R'_1$, $R'_2$, $R'_3$, and $R'_4$ each represent a hydrogen atom, methyl, or ethyl, provided that $R'_1$ and $R'_3$ are the same as each other and $R'_2$ and $R'_4$ are the same as each other while $R'_1$ and $R'_3$ may be the same as or different from $R'_2$ and $R'_4$, or

$R_1$, $R_3$, $R_5$, and $R_7$ each represent a hydrogen atom or straight-chain or branched alkyl having 1 to 4 carbon atoms, provided that $R_1$ and $R_5$ are the same as each other and $R_3$ and $R_7$ are the same as each other while $R_1$ and $R_5$ may be the same as or different from $R_3$ and $R_7$; $R_2$, $R_4$, $R_6$, and $R_8$ each represent straight-chain alkyl having 4 to 8 carbon atoms, provided that $R_2$ and $R_6$ are the same as each other and $R_4$ and $R_8$ are the same as each other while $R_2$ and $R_6$ may be the same as or different from $R_4$ and $R_8$; and $R'_1$, $R'_2$, $R'_3$, and $R'_4$ each represent a hydrogen atom, methyl, or ethyl, provided that $R'_1$ and $R'_3$ are the same as each other and $R'_2$ and $R'_4$ are the same as each other while $R'_1$ and $R'_3$ are different from $R'_2$ and $R'_4$.

(7) The compound according to (1) or a stereoisomer thereof, wherein $R_1$, $R_3$, $R_5$, and $R_7$, which are the same as each other, represent a hydrogen atom or straight-chain or branched alkyl having 1 to 4 carbon atoms; $R_2$, $R_4$, $R_6$ and $R_8$, which are the same as each other, represent straight-chain alkyl having 1 to 9 carbon atoms; and $R'_1$, $R'_2$, $R'_3$, and $R'_4$, which are the same as each other, represent a hydrogen atom, methyl, or ethyl.

(8) An agricultural and horticultural harmful organism control agent, comprising at least one of compounds according to any one of (1) to (7) or stereoisomers thereof.

(9) A veterinary parasite control agent comprising at least one of compounds according to (4) or a stereoisomer thereof.

(10) A process for producing a compound represented by formula (1):

[Chemical formula 31]

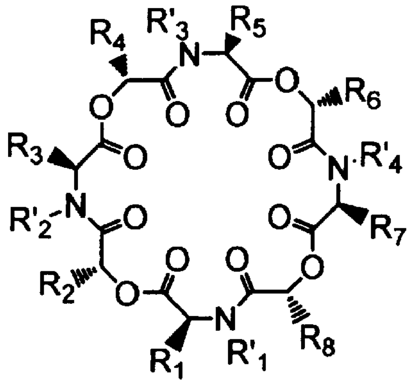

(1)

wherein $R_1$, $R_2$, $R_3$, $R_4$, $R_5$, $R_6$, $R_7$, $R_8$, $R'_1$, $R'_2$, $R'_3$, and $R'_4$ are as defined in claim 1, the process comprising: cyclizing a compound represented by formula (2):

[Chemical formula 32]

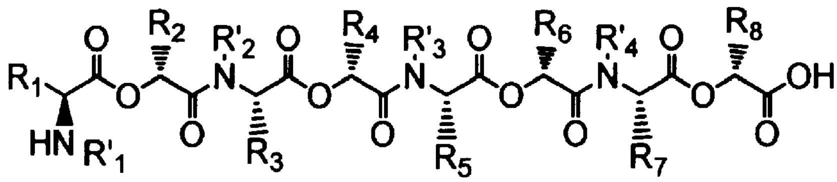

(2)

wherein $R_1$, $R_2$, $R_3$, $R_4$, $R_5$, $R_6$, $R_7$, $R_8$, $R'_1$, $R'_2$, $R'_3$, and $R'_4$ are as defined in (1).

(11) The process according to (10), which further comprises obtaining the compound of formula (2) by removing group X and group Y of a compound represented by formula (3):

[Chemical formula 33]

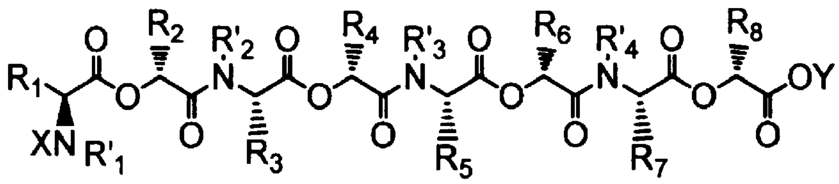

(3)

wherein $R_1$, $R_2$, $R_3$, $R_4$, $R_5$, $R_6$, $R_7$, $R_8$, $R'_1$, $R'_2$, $R'_3$, and $R'_4$ are as defined in (1); X represents an N-terminal protecting group selected from the group consisting of acetyl (Ac), allyloxycarbonyl (Alloc), benzyloxycarbonyl (CbZ), tert-butyloxycarbonyl (Boc), and 9-fluorenylmethyloxycarbonyl (Fmoc); and Y represents a C-terminal protecting group selected from the group consisting of benzyl (OBn), p-nitrobenzyl (ONb), and tert-butyl ($OBu^t$), or a support of a polymer with a selectively removable fixing group.

(12) The process according to (11), which further comprises obtaining a compound of formula (3) by reacting a

compound represented by formula (4):

[Chemical formula 34]

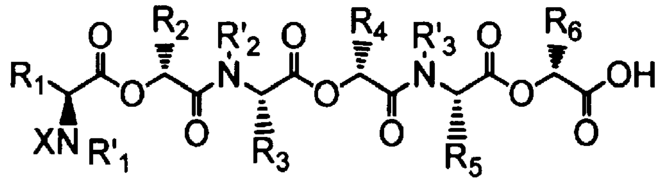

(4)

wherein $R_1$, $R_2$, $R_3$, $R_4$, $R_5$, $R_6$, $R'_1$, $R'_2$, $R'_3$, and X are as defined in claim 11, with a compound represented by formula (5):

[Chemical formula 35]

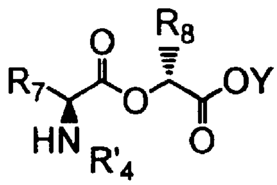

(5)

wherein $R_7$, $R_8$, $R'_4$, and Y are as defined in (11).

EXAMPLES

The present invention is further illustrated by the following Examples that are not intended as a limitation of the invention.

[Production Examples]

Production Example 1: Synthesis of (R)-(+)-2,3-epoxypropanoic acid benzyl ester

L-serine (100 g) and 340 g of potassium bromide were dissolved at 0°C in 600 ml of water, and an aqueous 47% hydrogen bromide solution was added dropwise thereto. When the reaction solution has become homogeneous, 200 ml of a 5N sodium nitrite solution was added dropwise. The mixture was stirred at 0°C for 30 min and was then stirred at room temperature for 24 hr. Diethyl ether was added to the reaction solution, and the mixture was extracted five times. A diethyl ether phase was dried over sodium sulfate and was then concentrated. The residue was dissolved in ethanol, and a 9.5N ethanol solution of potassium hydroxide was added dropwise at -40°C. The mixture was stirred at room temperature for 24 hr, and the reaction solution was concentrated. The resultant white solid was suspended in ethanol, and the suspension was stirred at 60°C for one hr and was then filtered through Kiriyama Rohto to collect a white solid. The collected white solid was washed twice with ethanol at 60°C. The white solid (50 g) was dissolved in 264 ml of dichloromethane, 90 g of benzyltrimethylammonium chloride and 52 ml of benzyl bromide were added thereto, and the mixture was heated under reflux for 8 hr. The reaction solution was returned to room temperature before diethyl ether was added. The organic phase was washed with water and saturated brine, was dried over sodium sulfate, and was concentrated. The resultant crude product was purified by flash column chromatography (n-hexane : ethyl acetate = 15 : 1 to 4 : 1) to obtain 20 g of a target compound, (R)-(+)-2,3-epoxypropanoic acid benzyl ester.
Yield: 28%
$[\alpha]_D^{24}$: +18.3° (c=0.82, $CHCl_3$) 1H-NMR (400MHz, $CDCl_3$): δ:7.43-7.25 (m, 5H), 5.20 (dd, 2H), 3.46 (dd, 1H), 2.94 (dd, 2H)
HR-MS (FAB, NBA matrix):m/z [M+H] 178.1

Production Example 2: Synthesis of (R)-2-hydroxyheptanoic acid benzyl ester ((R)-Hep(OBn))

Under a nitrogen atmosphere, 10.5 g of a copper(I) bromide dimethyl sulfide complex was suspended at -78°C to anhydrous diethyl ether, 107 ml of a tetrahydrofuran solution of 0.91 Mn-butyl magnesium chloride was added dropwise to the suspension, and the mixture was stirred at -78°C for 30 min. The compound of Production Example 1 (8.3 g) was dissolved in 15 ml of anhydrous diethyl ether, the solution was added dropwise to the reaction solution at -78°C, and the mixture was stirred at -78°C for 30 min and was then stirred at -5°C for one hr. An aqueous saturated ammonium chloride solution was added to the reaction solution, and the mixture was returned to room temperature before diethyl ether was added thereto. The organic phase was separated and was washed thrice with an aqueous saturated ammonium chloride solution. The organic phase was dried over anhydrous sodium sulfate and was then concentrated. The resultant crude product was purified by flash column chromatography (n-hexane : ethyl acetate = 20 : 1 to 15 : 1) to obtain 11.1 g of a target compound, (R)-2-hydroxyheptanoic acid benzyl ester.
Yield: quant.
$[\alpha]_D^{24}$: +18.0° (c=0.93, $CHCl_3$) 1H-NMR (400MHz, $CDCl_3$): δ: 7.45-7.28 (m, 5H), 5.24 (d, J=12.0Hz, 1H), 5.19 (d, J=12.0Hz, 1H), 4.23 (dd, J=3.75, 7.20Hz, 1H), 1.88-1.56 (m, 2H), 1.52-1.18 (m, 6H), 0.87 (t, J=6.75Hz, 3H)
HR-MS (FAB, NBA matrix): m/z [M+H] 236.1

Production Example 3: Synthesis of (R)-2-hydroxynonanoic acid benzyl ester (Bn-(R)-Nna)

(R)-2-aminononanoic acid (2 g) was dissolved in 160 ml of 2N sulfuric acid, 160 ml of a 2N aqueous sodium nitrite solution was added dropwise with stirring at room temperature, and the mixture was stirred at room temperature for 2 hr. Diethyl ether was added to the reaction solution, and the mixture was extracted thrice. Anhydrous sodium sulfate was added to the organic phase for drying, and the dried organic phase was then concentrated.

The resultant crude product as such was dissolved in 50 ml of methanol, and 5 ml of water was added to the solution. A 20% aqueous cesium carbonate solution was added thereto to pH 7.0, followed by concentration of the solution. The mixture was dissolved in 5 ml of N,N-dimethylformamide, and 12.6 ml of benzyl bromide was added dropwise to the solution. The mixture was stirred at room temperature for 17hr, water was then added thereto, and the mixture was extracted thrice with ethyl acetate. The organic phase was dried over anhydrous sodium sulfate and was then concentrated. The resultant crude product was purified by flash column chromatography (n-hexane : ethyl acetate = 30 : 1) to obtain 1.8 g of a target compound, (R)-2-hydroxynonanoic acid benzyl ester.
Yield: 59%
1H-NMR (400MHz, $CDCl_3$): δ: 7.34-7.33 (m, 5H), 5.22 (dd, J=12.2Hz, 16.1Hz, 2H), 4.25-4.20 (m, 1H), 2.69 (d, 5.8Hz, 1H), 1.82-1.75 (m, 1H), 1.67-1.60 (m,1H), 1.42-1.41 (m, 1H), 1.28-1.25 (m, 9H), 0.87 (t, J=6.9Hz, 3H)
MS (FAB, NBA matrix): m/z [M+H] 264

Production Example 4: Synthesis of N-Boc-N-Me-L-Ala-(R)-Hep(OBn)

Under a nitrogen atmosphere, 1.2 g of (R)-2-hydroxyheptanoic acid benzyl ester and 1.55 g of Boc-N-methyl-L-alanine were dissolved in 50 ml of dichloromethane at 0°C, 1.46 g of 1-ethyl-3-(3-dimethylaminopropyl)carbodiimide hydrochloride and 32 mg of dimethylaminopyridine were added thereto, and the mixture was stirred at 0°C for 30 min and was then stirred at room temperature for one hr. Water was added to the reaction solution, and the mixture was extracted twice with dichloromethane. The organic phase was washed with a saturated aqueous sodium hydrogencarbonate solution and saturated brine, was then dried over anhydrous sodium sulfate, and was concentrated. The resultant crude product was purified by flash column chromatography (n-hexane : ethyl acetate = 10 : 1 to 9 : 1) to obtain 2.3 g of a target compound, N-Boc-N-Me-L-Ala-(R)-Hep(OBn).
Yield: 96%
$[\alpha]_D^{27}$: -13.1° (c=1.66, $CHCl_3$)
1H-NMR (400MHz, $CDCl_3$): δ: 7.62-7.30 (m, 5H), 5.22 (d, J=12.2Hz, 1H), 5.11 (d, J=12.4Hz, 1H), 5.03 (t, J=6.08Hz, 1H), 4.87-4.57 (m, 1H), 3.00 (bra, 3H), 2.00-1.88 (m, 2H), 1.56 (brs, 9H), 1.48 (d, J=7.50Hz, 3H), 1.32-1.16 (m, 6H), 0.86 (t, J=6.00Hz, 3H)
HR-MS (FAB, NBA matrix+Nal):m/z [M+Na] 444.2

Production Example 5: Synthesis of N-Boc-N-Me-L-Ala-(R)-Hep-N-Me-L-Ala-(R)-Hep(OBn)

N-Boc-N-Me-L-Ala-(R)-Hep(OBn) (4.3 g) was dissolved in 50 ml of ethyl acetate, 430 mg of 10% palladium carbon was added to the solution, and the mixture was stirred at room temperature under a hydrogen atmosphere for one hr. The reaction solution was filtered through Celite, followed by washing with ethyl acetate. The filtrate was concentrated to give crude N-Boc-N-Me-L-Ala-(R)-Hep.

On the other hand, 20 ml of a 4 M dioxane solution of hydrochloric acid was added to 4.3 g of N-Boc-N-Me-L-Ala-(R)-Hep(OBn), the mixture was stirred at room temperature for one hr, and toluene was then added to the reaction solution. The mixture was subjected to azeotropic treatment thrice to give crude N-Me-L-Ala-(R)-Hep(OBn).

The crude N-Boc-N-Me-L-Ala-(R)-Hep and the crude N-Me-L-Ala-(R)-Hep(OBn) were dissolved in 50 ml of dichloromethane. Diisopropylethylamine (5.27 ml) and 7.06 g of PyBrop were added, and the mixture was stirred for one hr. 10% Citric acid was added to the reaction solution, and the mixture was extracted twice with chloroform. The organic phase was washed with a saturated aqueous sodium hydrogencaronate solution and saturated brine, was dried over anhydrous sodium sulfate, and was concentrated. The resultant crude product was purified by flash column chromatography (n-hexane : ethyl acetate = 10 : 1 to 3 : 1) to give 6.3 g of a target compound, N-Boc-N-Me-L-Ala-(R)-Hep-N-Me-L-Ala-(R)-Hep(OBn).
Yield: 96%
$[\alpha]_D^{27}$: -27.0° (c=1.01, $CHCl_3$)
1H-NMR (400MHz, $CDCl_3$): $\delta$: 7.38-7.26 (m, 5H), 5.18 (d, J=12.3Hz, 1H), 5.08 (d, J=12.3Hz, 1H), 5.00 (t, J=5.70Hz, 2H), {5.04-4.84, 4.80-4.60 (m, each1H)}, 3.02-2.72 (m, 6H), 1.90-1.56 (m, 4H), 1.43 (brs, 9H), 1.56-1.10 (m, 6H), 1.80-1.10 (m, 12H), 0.90-0.78 (m, 6H)
HR-MS (FAB, NBA matrix+Nal):m/z [M+Na] 657.4

Production Example 6: Synthesis of N-Boc-N-Me-L-Ala-(R)-Hep-N-Me-L-Ala-(R)-Hep-N-Me-L-Ala-(R)-Hep(O Bn)

N-Boc-N-Me-L-Ala-(R)-Hep-N-Me-L-Ala-(R)-Hep(OBn) (14.6 g) was dissolved in 110 ml of ethyl acetate, 1.45 g of 10% palladium carbon was added to the solution, and the mixture was stirred under a hydrogen atmosphere at room temperature for 3 hr. The reaction solution was filtered through Celite, followed by washing with ethyl acetate. The filtrate was concentrated to give crude N-Boc-N-Me-L-Ala-(R)-Hep-N-Me-L-Ala-(R)-Hep. On the other hand, 52 ml of a 4 M dioxane solution of hydrochloric acid was added to 9.7 g of N-Boc-N-Me-L-Ala-(R)-Hep(OBn), and the mixture was stirred at room temperature for 5 hr. Toluene was then added to the reaction solution, and the mixture was subjected to azeotropic treatment thrice to give crude N-Me-L-Ala-(R)-Hep(OBn).

The crude N-Boc-N-Me-L-Ala-(R)-Hep-N-Me-L-Ala-(R)-Hep and the crude N-Me-L-Ala-(R)-Hep(OBn) were dissolved in 230 ml of dichloromethane, 9.0 g of diisopropylethylamine and 16.3 g of PyBrop were added to the solution, and the mixture was stirred at 0°C for 30 min and at room temperature for 2.5 hr. 10% citric acid was added to the reaction solution, and the mixture was extracted twice with chloroform. The organic phase was washed with a saturated aqueous sodium hydrogencaronate solution and saturated brine, was dried over anhydrous sodium sulfate, and was concentrated. The resultant crude product was purified by flash column chromatography (n-hexane : ethyl acetate =10 : 1 to 3 : 1) to give 16.19 g of a target compound, N-Boc-N-Me-L-Ala-(R)-Hep-N-Me-L-Ala-(R)-Hep-N-Me-L-Ala-(R)-Hep(O Bn).
Yield: 83%
1H-NMR (60MHz, $CDCl_3$) $\delta$:7.20 (bra, 5H), 5.30-4.80 (m, 8H), 3.05 (bra, 3H), 2.97-2.63 (m, 6H), 1.40 (bra, 43H), 1.00-0.70 (m, 8H)

Production Example 7: Synthesis of N-Boc-N-Me-L-Nle-(R)-Nna(OBn)

The compound of Production Example 3 (0.1 g) and 0.09 g of N-Boc-N-methyl-L-norleucine were dissolved in 5 ml of dichloromethane under a nitrogen atmosphere. Thereafter, a synthesis was carried out in the same manner as in Production Example 4 to give 0.16 g of a target compound, N-Boc-N-Me-L-Leu-(R)-Nna(OBn).
Yield: 86%
1H-NMR (400MHz, $CDCl_3$) $\delta$:7.38-7.32 (m, 5H), 5.21-5.18 (d, J=12.2Hz, 1H), 5.13-5.10 (d, J=12.0, 1H), 5.03 (t, J=6.2Hz, 1H), 4.91-4.90, 4.68-4.66 (m, 1H), 2.75 (d, J=14.2Hz, 3H), 1.83-1.82 (m, 2H), 1.46-1.44 (m, 8H), 1.34-1.21 (m, 17H), 0.94-0.86 (m, 6H)
MS (FAB, NBA matrix+Nal):m/z [M+H] 492

Production Example 8: Synthesis of N-Boc-N-Me-L-Nle-(R)-Nna-N-Me-L-Ala-(R)-Hep-N-Me-L-Ala-(R)-Hep-N-Me-L-Ala-(R)-Hep(OBn)

The compound of Production Example 6 (0.17 g) was dissolved in 1 ml of a 4 M dioxane solution of hydrochloric acid, and the mixture was stirred at room temperature for one hr. The reaction solution was concentrated to give crude N-Me-L-Ala-(R)-Hep-N-Me-L-Ala-(R)-Hep-N-Me-L-Ala-(R)-Hep(OBn).

Next, 0.1 g of the compound of Production Example 7 was dissolved in methanol, 1 mg of 10% palladium carbon was added to the solution, and the mixture was stirred at room temperature under a hydrogen atmosphere for one hr. The reaction solution was filtered through Celite, followed by washing with methanol. The organic phases were

combined and concentrated to give crude N-Boc-N-Me-L-Nle-(R)-Nna. The crude N-Me-L-Ala-(R)-Hep-N-Me-L-Ala-(R)-Hep-N-Me-L-Ala-(R)-Hep(OBn) and the crude N-Boc-N-Me-L-Nle-(R)-Nna were dissolved in dichloromethane, 0.11 ml of diisopropylethylamine and 0.14 g of PyBrop were added to the solution, and the mixture was was stirred at room temperature for 17. 10% citric acid was added to the reaction solution, and the mixture was extracted twice with chloroform. The organic phase was washed with a saturated aqueous sodium hydrogencaronate solution and saturated brine, was dried over anhydrous sodium sulfate, and was concentrated. The resultant crude product was purified by flash column chromatography (n-hexane : ethyl acetate = 20 : 1) to give 0.2 g of a target compound, N-Boc-N-Me-L-Nle-(R)-Nna-N-Me-L-Ala-(R)-Hep-N-Me-L-Ala-(R)-Hep-N -Me-L-Ala-(R)-Hep(OBn).
Yield: 91%
1H-NMR (400MHz, $CDCl_3$) δ:7.37-7.33 (5H,m), 5.23-5.19 (6H, m), 5.14 (1H, d, J=2.9Hz), 5.03 (1H, t, J=6.3), 4.88-4.82 (1H, m), 4.79-4.71 (1H, m), 4.70-4.60 (1H, m), 3.10 (5H, d, J=6.0Hz), 2.98 (4H, d, J=7.6Hz), 2.91 (2H, d, J-4.4Hz), 2.84 (5H, d, J=10.7Hz), 2.05 (4H, s)1.85-1.79 (4H, m), 1.78-1.65 (4H, m), 1.65-1.50 (8H, m), 1.46 (8H, d, J=9.3Hz), 1.41-1.33 (8H, m), 1.32-1.24 (14H, m), 0.89-0.86 (20H, m)
MS (EI): m/z [M+H]1131

Production Example 9: Synthesis of N-Boc-N-Me-L-Gly-(R)-Hep-N-Me-L-Gly-(R)-Hep-N-Me-L-Leu-(R)-Hep-N-Me-L-Leu-(R)-Hep(OBn)

N-Boc-N-Me-L-Gly-(R)-Hep-N-Me-L-Gly-(R)-Hep(OBn) (100 mg) synthesized in the same manner as in Production Example 5 except for use of N-Boc-N-Me-L-Gly-(R)-Hep(OBn) instead of N-Boc-N-Me-L-Ala-(R)-Hep(OBn) was dissolved in 5 ml of ethyl acetate, and 20 mg of 10% palladium carbon was added to the solution, and the mixture was stirred at room temperature under a hydrogen atmosphere for 2.5 hr. The reaction solution was filtered through Celite, followed by washing with ethyl acetate. The filtrate was concentrated to give crude N-Boc-N-Me-L-Gly-(R)-Hep-N-Me-L-Gly-(R)-Hep. On the other hand, 100 mg of N-Boc-N-Me-L-Leu-(R)-Hep-N-Me-L-Leu-(R)-Hep(OBn) synthesized in the same manner as in Production Example 5 except for use of N-Boc-N-Me-L-Leu-(R)-Hep(OBn) instead of N-Boc-N-Me-L-Ala-(R)-Hep(OBn) was dissolved in 1 ml of a 4 M dioxane solution of hydrochloric acid and the mixture was stirred at room temperature for 1.5 hr. The reaction solution was concentrated to give crude N-Me-L-Leu-(R)-Hep-N-Me-L-Leu-(R)-Hep(OBn). The crude N-Boc-N-Me-L-Gly-(R)-Hep-N-Me-L-Gly-(R)-Hep and the crude N-Me-L-Leu-(R)-Hep-N-Me-L-Leu-(R)-Hep(OBn) were dissolved in dichloromethane, 0.07 ml of diisopropylethylamine and 0.09 g of PyBrop were added to the solution, and the mixture was stirred at room temperature for 17 hr. 10% citric acid was added to the reaction solution, and the mixture was extracted twice with chloroform. The organic phase was washed with a saturated aqueous sodium hydrogencarbonate solution and saturated brine, was dried over anhydrous sodium sulfate, and was concentrated. The resultant crude product was purified by flash column chromatography (n-hexane : ethyl acetate = 5 : 1 to 3 : 1) to give 0.13 g of a target compound, N-Boc-N-Me-L-Gly-(R)-Hep-N-Me-L-Gly-(R)-Hep-N-Me-L-Leu-(R)-Hep-N-Me-L-Leu-(R)-Hep(OBn).
Yield: 71%
LC/MS (SenshuPak PEGASIL ODS 2ϕ×50mm, 10% acetonitrile
(0.1 %TFA)/water (0.05%TFA) → 100% acetonitrile (0.1%TFA), 8 min, flow rate: 0.3 mL/min) R.T 9.7 min, m/z [M+Na] 1139.3

Production Example 10: Synthesis of Cycro(-N-Me-L-Nle-(R)-Nna-N-Me-L-Ala-(R)-Hep-N-Me-L-Ala-(R)-Hep-N-Me-L-Ala-(R)-Hep-) (compound 16)

The compound of Production Example 8 (0.06 g) was dissolved in 1 ml of a 4 M dioxane solution of hydrochloric acid, and the mixture was stirred at room temperature for one hr. The reaction solution was concentrated, the residue was dissolved in methanol, 1 mg of 10% palladium carbon was added to the solution, and the mixture was stirred at room temperature under a hydrogen atmosphere for one hr. The reaction solution was filtered through Celite, followed by washing with methanol. The organic phases were combined and concentrated to give crude N-Me-L-Nle-(R)-Nna-N-Me-L-Ala-(R)-Hep-N-Me-L-Ala-(R)-Hep-N-Me-L-Ala-(R)-Hep. The resultant crude product was purified by flash column chromatography (chloroform : methanol = 50 : 1 to 10 : 1). The purification product was dissolved in 5 ml of dichloromethane, 0.01 ml of diisopropylethylamine and 0.02 g of PyBrop were added to the solution, and the mixture was stirred at room temperature for 4 hr. 10% citric acid was added to the reaction solution, and the mixture was then extracted twice with chloroform. The organic phase was washed with a saturated aqueous sodium hydrogencarbonate solution and saturated brine, was dried over anhydrous sodium sulfate, and was concentrated. The resultant crude product was purified by flash column chromatography (chloroform : methanol = 200 : 1) to give 0.01 g of a target compound, Cycro(-N-Me-L-Nle-(R)-Nna-N-Me-L-Ala-(R)-Hep-N-Me-L-Ala-(R)-Hep-N-Me-L-Ala-(R)-Hep-).

Production Example 11: Synthesis of Cycro(-N-Me-L-Gly-(R)-Hep-N-Me-L-Gly-(R)-Hep-N-Me-L-Leu-(R)-Hep-N -Me-L-Leu-(R)-Hep-) (compound 47)

The compound of Production Example 9 (0.13 g) was dissolved in ethyl acetate, 30 mg of 10% palladium carbon was added to the solution, and the mixture was stirred at room temperature under a hydrogen atmosphere for 1.5 hr. The reaction solution was filtered through Celite, followed by washing with ethyl acetate. The organic phases were combined and concentrated. The residue was dissolved in 1 ml of a 4 M dioxane solution of hydrochloric acid, and the solution was stirred at room temperature for 1.5 hr. The reaction solution was concentrated to give crude N-Me-L-Gly-(R)-Hep-N-Me-L-Gly-(R)-Hep-N-Me-L-Leu-(R)-Hep-N-Me-L-Leu-(R)-Hep. The resultant crude product was dissolved in 18 ml of dichloromethane, 0.05 ml of diisopropylethylamine and 0.07 g of PyBrop were added to the solution, and the mixture was stirred at room temperature for 17 hr. The reaction solution was washed with a saturated aqueous sodium hydrogencarbonate solution, 10% sodium hydrogen sulfate, and saturated brine, was dried over anhydrous sodium sulfate, and was concentrated. The resultant crude product was purified by flash column chromatography (chloroform : methanol =200:1) to give 0.07 g of a target compound, Cycro(-N-Me-L-Gly-(R)-Hep-N-Me-L-Gly-(R)-Hep-N-Me-L-Leu-(R)-Hep-N -Me-L-Leu-(R)-Hep-).
Yield: 67%

Production Example 12: Synthesis of Cycro(-N-Me-L-Gly-(R)-Oct-N-Me-L-Ala-(R)-Hep-N-Me-L-Ala-(R)-Hep-N-Me-L-Ala-(R)-Hep-) (compound 2)

N-Boc-N-Me-L-Gly-(R)-Oct-N-Me-L-Ala-(R)-Hep-N-Me-L-Ala-(R)-Hep-N-Me-L-Ala-(R)-Hep(OBn) (0.1 g) synthesized in the same manner as in Production Example 8 except for use of Boc-N-Me-L-Gly-(R)-Oct(OBn) instead of Boc-N-Me-L-Leu-(R)-Nna(OBn) was dissolved in methanol, 20 mg of 10% palladium carbon was added to the solution, and the mixture was stirred at room temperature under a hydrogen atmosphere for one hr. The reaction solution was filtered through Celite, followed by washing with ethyl acetate. The organic phases were combined and were concentrated. The residue was dissolved in 1 ml of a 4 M dioxane solution of hydrochloric acid, and the solution was stirred at room temperature for one hr. The reaction solution was concentrated to give crude N-Me-L-Gly-(R)-Oct-N-Me-L-Ala-(R)-Hep-N-Me-L-Ala-(R)-Hep-N-Me-L-Al a-(R)-Hep. The resultant crude product was dissolved in 19 ml of dichloromethane, 0.05 ml of diisopropylethylamine and 0.07 g of PyBOP were added to the solution, and the mixture was stirred at room temperature for 17 hr. The reaction solution was washed with a saturated aqueous sodium hydrogencarbonate solution, 10% sodium hydrogen sulfate, and saturated brine, was dried over anhydrous sodium sulfate, and was concentrated. The resultant crude product was purified by flash column chromatography (chloroform : methanol = 200 : 1) to give 0.06 g of a target compound, Cycro(-N-Me-L-Gly-(R)-Oct-N-Me-L-Ala-(R)-Hep-N-Me-L-Ala-(R)-Hep-N -Me-L-Ala-(R)-Hep-).
Yield: 75%

Compounds produced according to processes described in Production Example 1 to 12 and property values thereof are shown in Table 2.

[Table 2]

| Compound No. | NMR | MS | LC | αD | Production process |
|---|---|---|---|---|---|
| 1 | δ: 5.54-5.24 (m, 7H), 4.93 (d, J=17.2Hz, 1H), 3.78 (d, J=17.6, 1H), 3.17 (s, 3H), 3.05 (s, 9H), 1.87-1.74 (m, 8H), 1.52-1.32 (m, 33H), 0.90-0.87 (m, 12H) | [(M+Na)$^+$]: 861.6 | 7.71 | | Produced in the same manner as in Compound 16 |
| 2 | δ: 5.45-5.29 (8H, m), 4.96 (1H, d, J = 17.6 Hz), 3.77 (1H, d, J = 17.6 Hz), 3.18 (3H, s), 3.06 (10H, t, J = 6.7 Hz), 1.90-1.75 (10H,m), 1.58-1.46 (14H, m), 1.42-1.30 (12H, m), 0.96-0.89 (17H,m). | [(M+H)$^+$]: 853.9 | | | Production Example 12 |
| 3 | δ: 5.34-5.28 (m, 7H), 3.76 (d, J=17.6, 1H), 3.15 (s, 3H), 3.07 (s, 3H), 3.05 (s, 3H), 3.05 (m, 1H), 3.03 (s, 3H), 1.82-1.81 (m, 8H), 1,52-1.31 (m, 37H), 0.94-0.88 (m, 12H) | [(M+Na)$^+$]: 889.5 | 8.4 | | Produced in the same manner as in Compound 16 |
| 4 | | [(M+H)$^+$]: 881.6 | | | Produced in the same manner as in Compound 16 |
| 5 | δ: 5.34-5.28 (m, 7H), 3.76 (d, J=17.6, 1H), 3.15 (s, 3H), 3.05 (m, 1H), 3.06 (s, 3H), 3.05 (s, 3H), 3.03 (s, 3H), 1.84-1.79 (m, 8H), 1.48-1.30 (m, 41H), 0.94-0.87 (m, 12H) | [(M+Na)$^+$]: 889.6 | 8.85 | | Produced in the same manner as in Compound 16 |
| 6 | | [(M+H)$^+$]: 839.4 | | | Produced in the same manner as in Compound 16 |
| 7 | δ: 5.35-5.28 (m, 8H), 3.04 (s, 12H), 1.85-1.79 (m, 8H), 1,53-1.33 (m, 38H), 0.94-0.88 (m, 12H) | [(M+Na)$^+$]: 917.6 | 8.3 | | Produced in the same manner as in Compound 16 |
| 8 | δ: 5.34-5.29 (m, 8H), 3.05 (s, 12H), 1.83-1.80 (m, 8H), 1,53-1.30 (m, 42H), 0.94-0.88 (m, 12H) | [(M+Na)$^+$]: 917.6 | 8.75 | | Produced in the same manner as in Compound 16 |

| | | | | | |
|---|---|---|---|---|---|
| 9 | δ: 5.36-5.28 (m, 8H), 3.05 (s, 12H), 1.86-1.78 (m, 8H), 1,53-1.30 (m, 44H), 0.94-0.88 (m, 12H) | [(M+Na)$^+$]: 931.6 | 8.88 | | Produced in the same manner as in Compound 16 |
| 10 | δ: 5.38-5.32 (m, 8H), 3.06 (s, 9H), 3.03 (s, 3H), 2.16 (m, 1H), 1.82-1.81 (m, 9H), 1,50-1.36 (m, 33H), 0.93-0.87 (m, 15H) | [(M+Na)$^+$]: 889.6 | 8.11 | | Produced in the same manner as in Compound 16 |
| 11 | δ: 5.37-5.30 (m, 8H), 3.06 (s, 9H), 3.03 (s, 3H), 2.16 (m, 1H), 1.84-1.81 (m, 9H), 1,50-1.37 (m, 35H), 0.95-0.88 (m, 15H) | [(M+Na)$^+$]: 903.6 | 8.3 | | Produced in the same manner as in Compound 16 |
| 12 | δ: 5.37-5.31 (m, 8H), 3.06 (s, 9H), 3.04 (s, 3H), 2.20 (m, 1H), 1.83-1.81 (m, 9H), 1,51-1.32 (m, 37H), 0.95-0.88 (m, 15H) | [(M+Na)$^+$]: 917.6 | 8.55 | | Produced in the same manner as in Compound 16 |
| 13 | δ: 5.38-5.30 (m, 8H), 3.06 (s, 9H), 3.04 (s, 3H), 2.20 (m, 1H), 1.84-1.81 (m, 9H), 1,51-1.32 (m, 39H), 0.95-0.88 (m, 15H) | [(M+Na)$^+$]: 931.6 | 8.81 | | Produced in the same manner as in Compound 16 |
| 14 | δ: 5.37-5.31 (m, 8H), 3.06 (s, 9H), 3.05 (s, 3H), 2.20 (m, 1H), 1.83-1.82 (m, 9H), 1,51-1.31 (m, 41H), 0.95-0.88 (m, 15H) | [(M+Na)$^+$]: 945.6 | 8.46 | | Produced in the same manner as in Compound 16 |
| 15 | | [(M+H)$^+$]:895.3 | | | Produced in the same manner as in Compound 16 |
| 16 | δ: 5.42-5.28 (8H, m), 3.05 (9H,s), 3.01(3H,s),1.88-1.78 (10H, m), 1.50 (24H, dd, J = 7.1, 1.2 Hz), 1.42-1.33 (16H, m), 0.94 (16H, t, J = 7.0 Hz) | [(M+H)$^+$]: 923.4 | | | Production Example 10 |

| | | | | | |
|---|---|---|---|---|---|
| 17 | δ: 5.38-5.29 (m, 8H), 3.06 (s, 9H), 3.03 (s, 3H), 1.90-1.77 (m, 10H),1.49(ddJ=10.3,7.4Hz, 16H), 1.42-1.26(m,26H),0.91 (m, 16H) | [(M+H)+]: 937.8 | 5.23 | | Produced in the same manner as in Compound 16 |
| 18 | δ: 5.35-5.30 (m, 8H), 3.06 (s, 9H), 3.03 (s, 3H), 2.16 (m, 1H), 1.84-1.81 (m, 9H), 1,50-1.24 (m, 43H), 0.95-0.89 (m, 18H) | [(M+Na)$^+$]: 973.6 | 9.29 | | Produced in the same manner as in Compound 16 |
| 19 | | [(M+H)$^+$]:881.6 | | | Produced in the same manner as in Compound 16 |
| 20 | δ: 5.45-5.29 (8H, m), 3.06 (9H, s), 3.03 (3H, s), 1.87-1.76 (12H, m), 1.49 (14H,dd,J=9.5Hz,7.3Hz), 1.42-1.30 (18H, m), 0.92 (18H, m) | [(M+H)$^+$] : 895.5 | 4.72 | | Produced in the same manner as in Compound 16 |
| 21 | δ: 5.36-5.28 (8H, m), 3.05 (9H, s), 3.02 (3H, s), 1.87-1.70 (9H, d, *J* = 8.8 Hz), 1.49 (18H, dd, *J* = 7.2, 3.1 Hz), 1.36-1.23 (19H, m), 0.98-0.89 (18H, m). | [(M+H)$^+$] : 909.6 | 6.69 | | Produced in the same manner as in Compound 16 |
| 22 | δ: 5.37-5.29 (m, 8H), 3.05 (s, 9H), 3.02 (s, 3H), 2.06 (m, 1H), 1.83-1.79 (m, 9H), 1,50-1.24 (m, 39H), 0.97-0.88 (m, 17H) | [(M+Na)$^+$]: 945.6 | 9.13 | | Produced in the same manner as in Compound 16 |
| 23 | δ: 5.45-5.28 (8H, m), 3.05 (9H, s,), 3.03(3H,s),1.86-1.78 (9H,m), 1.49 (18H, dd, *J* = 9.7, 7.3 Hz), 1.30-1.28(25H,m),0.98-0.88 (16H, m) | [(M+H)$^+$]: 937.8 | 5.05 | | Produced in the same manner as in Compound 16 |
| 24 | δ: 5.37-5.25 (m, 6H), 5.12 (m, 1H), 4.61 (m, 1H), 3.15 (s, 3H), 3.12 (s, 3H), 3.09 (s, 3H), 3.07 (s, 3H), 2.34 (m, 1H), 1.84-1.80 (m, 8H), 1.51-1.36 (m, 35H), 1.04 (d, *J*=6.55, 3H), 0.93-0.89 (m, 15H) | [(M+Na)$^+$]: 903.6 | 8.13 | | Produced in the same manner as in Compound 16 |

| | | | | | |
|---|---|---|---|---|---|
| 25 | δ: 5.36-5.25 (m, 6H), 5.09 (m, 1H), 4.55 (m, 1H), 3.15 (s, 3H), 3.13 (s, 3H), 3.09 (s, 3H), 3.07 (s, 3H), 2.32 (m, 1H), 1.91-1.76 (m, 8H), 1.51-1.31 (m, 39H), 1.04 (d, J=6.50, 3H), 0.94-0.88 (m, 15H) | [(M+Na)$^+$]: 931.6 | 8.92 | | Produced in the same manner as in Compound 16 |
| 26 | δ : 5.43-5.33 (m, 6H), 5.15 (broad s, 5.15, 1H), 4.62 (m, 1H), 3.22 (s, 3H), 3.18 (s, 3H), 3.15 (s, 3H), 3.13 (s, 3H), 2.38 (m, 1H), 1.94-1.85 (m, 8H), 1.57-1.35 (m, 43H), 1.10 (d, J=6.50, 3H), 1.00-0.93 (m, 15H) | [(M+Na)$^+$]: 959.6 | 9.38 | | Produced in the same manner as in Compound 16 |
| 27 | | [(M+H)$^+$]:895.3 | 6.49 | | Produced in the same manner as in Compound 16 |
| 28 | δ: 5.45-5.28 (m, 8H), 3.04 (s × 2, 9H), 3.01 (s, 3H), 1.83-1.80 (m, 10H), 1.48-1.31 (m, 40H), 0.99 (d, J=6.51, 3H), 0.93-0.89 (m, 15H) | [(M+Na)$^+$]: 945.6 | 9.06 | | Produced in the same manner as in Compound 16 |
| 29 | | [(M+H)$^+$]:937.6 | | | Produced in the same manner as in Compound 16 |
| 30 | δ: 5.46-5.28 (m, 8H), 3.04 (s×2, 9H), 3.01 (s, 3H), 1.89-1.80 (m, 10H), 1.48-1.31 (m, 44H), 0.99 (d, J=6.51, 3H), 0.93-0.89 (m, 15H) | [(M+Na)$^+$]: 973.6 | 9.49 | | Produced in the same manner as in Compound 16 |
| 31 | | [(M+H+)]: 841.5 | | $[a]_D^{25}$ - 45.6 (c 0.51, CHCl3) | Produced in the same manner as in Compound 16 |
| 32 | δ: 5.40-5.23 (m, 5H), 4.86 (d, 1H, J=17.5), 3.79-3.64 (m, 3H), 3.12-2.83 (m, 3H), 3.08 (s, 3H), 3.06 (s, 3H), 3.05 (s, 3H), 2.93 (s, 3H), 1.79-1.69 (m, 10H), 1.42-1.23 (m, 29H), 0.92-0.79 (m, 18H) | [(M+Na)$^+$]: 903.5 | 8.38 | | Produced in the same manner as in Compound 47 |

| | | | | | |
|---|---|---|---|---|---|
| 33 | δ: 5.36-5.24 (m, 7H), 4.86-4.71 (m, 3H), 3.68-3.64 (d, J=17.5, 1H), 3.10-2.84 (m, 1H), 3.04 (s, 3H), 2.99-2.84 (m, 1H), 2.93 (s, 3H), 2.92 (s, 3H), 2.90 (s, 3H), 1.80-1.72 (m, 14H), 1.42-1.26 (m, 27H), 0.91-0.82 (m, 30H) | [(M+Na) $^+$]: 987.7 | 9.08 | | Produced in the same manner as in Compound 47 |
| 34 | δ: 5.40-5.23 (m, 5H), 4.60-, 4.48 (m, 1H), 3.79-3.64 (m, 3H), 3.12-2.84 (m, 3H), 3.07 (s, 3H), 3.05 (s, 6H), 3.05 (s, 3H), 2.93 (s, 3H), 1.79-1.69 (m, 10H), 1.42-1.23 (m, 25H), 0.92-0.79 (m, 18H) | [(M+Na) +]: 875.5 | 7.95 | | Produced in the same manner as in Compound 16 |
| 36 | δ:5.52-5.4(m, 2H), 5.36(m, 4H), 3.30(s, 12H), 3.18(s, 2H), 3.00(s, 2H), 1.88-1.76(m, 12H), 1.59-1.49(m, 2H), 1.40-1.30(m, 24H), 0.95-0.87(m, 24H) | [(M+H)$^+$]:909.6 | 9.52 | | Produced in the same manner as in Compound 47 |
| 37 | δ(ppm) 5.46–5.40 (m, 2H), 5.40–5.29 (m, 4H), 3.88–3.70 (m, 4H), 3.28–2.80 (m, 16H), 1.82 (s, 8H ), 1.48 (d, J = 7.0 Hz, 12H), 1.64–1.22 (s, 24H), 0.93 (s, 12H) | [(M+H)$^+$]:847.5 | 8.81 | | Produced according to step c) (PS: 2-chloro-tritylchloro-resin) |
| 39 | δ: 5.34-5.28 (m, 8H), 3.02 (s, 6H), 3.00 (s, 6H), 2.12-2.09 (m, 2H), 1.90-1.76 (m, 8H), 1.46-1.14 (m, 46H), 0.90-0.87 (m, 18H) | [(M+Na)$^+$ ]: 959.6 | 10.65 | | Produced according to step c) (PS: 2-chloro-tritylchloro-resin) |
| 40 | δ: 5.49-5.36 (m, 8H), 3.01 (s, 6H), 3.00 (s, 6H), 1.89-1.78 (m, 12H), 1.49-1.35 (m, 22H), 0.98 (d, 12H, J=6.7Hz), 0.99-0.90 (m, 18H) | [(M+Na) $^+$]: 931.6 | 8.19 | | Produced according to step c) (PS: 2-chloro-tritylchloro-resin) |
| 41 | δ: 5.49-5.29 (m, 8H), 3.01 (s, 12H), 2.10-2.08 (m, 2H), 1.91-1.79 (m, 12H), 1.53-1.21 (m, 34H), 1,00-0.91 (m, 30H) | [(M+Na)$^+$]:1015.7 | 10.42 | | Produced according to step c) (PS: 2-chloro-tritylchloro-resin) |
| 43 | δ(ppm)5.19-5.18 (m, 6H), 4.21 (s, 2H), 3.63-3.59 (m, 2H), 3.58-3.41 (m, 2H), 3.16 (s, 6H), 1.92-1.28 (m, 54H), 0.95-0.92 (m, 12H) | [(M+Na)$^+$]: 903.6 | 9.54 | | Produced in the same manner as in Compound 47 |

| | | | | | |
|---|---|---|---|---|---|
| 44 | δ(ppm) 5.00-4.70 (m, 4H ), 4.50-4.20 (m, 4H ), 2.00-1.60 (m, 8H), 1.60-1.10 (m, 12H), 1.60-1.10 (m, 24H ), 1.00-0.70 (m, 12H) | [(M+H)+]: 819.5 | | $[a]_D^{24}$ - 37.9 (c 1.34, MeOH) | Produced according to step c) (PS: 2-chloro-tritylchloro-resin) |
| 46 | δ(ppm) 5.58–5.20 (m, 8H ), 3.02 (s, 12H), 2.00–1.70 (m, 20H ), 1.64-1.22 (m, 24H), 1.07–0.82 (m, 36H ) | [(M+H)+]:1021.7 | 10.86 | | Produced according to step c) (PS: 2-chloro-tritylchloro-resin) |
| 47 | δ: 5.51-5.32 (m, 6H), 4.88-4.67 (m, 1H), 4.57-4.56 (m, 1H), 3.82 (dd, 2H, J=17.6, 14.5), 3.85-2.92 (m, 2H), 3.15(s, 3H), 3.14 (s, 3H), 3.05 (s, 3H), 3.02 (s, 3H), 1.86-1.78 (m, 12H), 1.52-1.36 (m, 26H), 1.01-0.92 (m, 24H) | [(M+Na) +]: 931.6 | 8.45 | | Production Example 11 |
| 48 | δ: 5.41-5.31 (m, 6H), 4.94-4.77 (m, 2H), 3.77 (dd, 2H, J=17.6, 14.5), 3.21-2.91 (m, 2H), 3.15(s, 3H), 3.14 (s, 3H), 3.05 (s, 3H), 3.01 (s, 3H), 1.88-1.80 (m, 12H), 1.50-1.30 (m, 34H), 1.00-0.89 (m, 24H) | [(M+Na) +]: 987.6 | | | Produced in the same manner as in Compound 47 |
| 49 | δ: 5.45-5.34 (m, 5H), 4.94 (d, 1H, J=17.2), 3.89-3.73 (m, 3H), 3.21-2.92 (m, 3H), 3.17 (s, 3H), 3.14 (s, 6H), 3.02 (s, 3H), 1.86-1.78 (m, 10H), 1.50-1.31 (m, 29H), 0.98 (d, 3H, J=6.6), 0.95-0.79 (m, 15H) | [(M+Na) +]: 903.6 | 8.35 | | Produced in the same manner as in Compound 47 |

| | | | | | |
|---|---|---|---|---|---|
| 51 | δ (ppm) 5.38 (m, 4H), 5.31 (t, J = 6.33 Hz, 4H), 3.06 (s, 12H), 1.83 (m, 8H), 1.60-1.45(m,12H), 1.45-1.25 (m, 40H), 0.90 (t, J = 6.83 Hz, 12H) | [(M+Na)+]: 987.7 | | | Produced in the same manner as in Compound 47 |
| 52 | δ (ppm) 5.6-5.4 (m, 3H), 5.35 (m, 4H), 3.75 (d, J = 16.0 Hz, 2H), 3.2-2.9 ( s, 12H), 1.95-1.75 (m, 16H), 1.45-1.25 (m, 28H), 1.05-0.85 (m, 27H) | [(M+Na)+]: 973.6 | | | Produced in the same manner as in Compound 47 |
| 53 | δ (ppm) 5.48 (m, 2H), 5.40 (m, 2H), 5.36 (m, 4H), 3.01 (s, 12H), 2.07 (m, 2H), 1.95-1.75 (m, 16H), 1.45-1.15 (m, 28H), 1.1-0.8 (m, 30H) | [(M+Na)+]: 1015.6 | | | Produced in the same manner as in Compound 47 |
| 54 | δ(ppm) 5.26-5.44 (m, 1H) ; 4.83 (s, 15H); 3.30 (d t, J=1.7 & 3.2 Hz, 18H); 3.15 (s,1H); 3.06 ( d, J=4.9 Hz, 1H); 3.03 (s, 1H); 1.75-1.87 (m, 1H); 1.44-1.55 (m, 3H); 1.29-1.42 (m, 2H); 0.97-1.05 (m, 1H); 0.87-0.96 (m, 2H) | [(M+H)+]: 825.6 | | | Produced in the same manner as in Compound 16 |
| 55 | δ (ppm) 5.48 (m, 1H), 5.41 (m, 3H), 5.36 (m, 4H), 3.01 (s, 12H), 2.15-2.04(m, 16H), 1.99-1.70 (m, 1H), 1.46-1.17 (m, 30H), 1.10-0.80 (m, 27H) | [(M+H)+]: 979.7 | | | Produced in the same manner as in Compound 16 |
| 56 | δ (ppm) 5.50 (m, 3H), 5.41 (m, 1H), 5.36 (m, 4H), 3.01 (s, 12H), 2.13-2.03(m, 16H) 1.98-1.63 (m, 3H), 1.46-1.16 (m, 26H), 1.10-0.75 (m, 33H) | [(M+H)+]: 1007.7 | | | Produced in the same manner as in Compound 16 |

| | | | | | |
|---|---|---|---|---|---|
| 57 | δ (ppm) 5.56-5.35 (m, 3H), 5.38 (m, 4H), 4.98 (d, J = 17.7 Hz, 1H), 3.78 (d, J = 17.8 Hz, 1H), 3.25-2.75 (m, 12H), 2.12-1.66 (m, 16H), 1.45-1.18 (m, 26H), 1.08-0.75 (m, 27H) | $[(M+H)^+]$: 951.5 | | | Produced in the same manner as in Compound 47 |
| 58 | δ (ppm) 5.65-5.25 (m, 7H), 4.98 (d, J = 17.2 Hz, 1H), 3.78 (d, J = 17.7 Hz, 1H), 3.20-2.75 (s, 12H), 2.18-1.65 (m, 16H), 1.45-1.15 (m, 26H), 1.10-0.70 (m, 27H) | $[(M+H)^+]$: 937.6 | | | Produced in the same manner as in Compound 47 |
| 59 | δ (ppm) 5.5 (m, 1H), 5.45-5.30 (m, 6H), 4.99 (d, J = 17.8 Hz, 1H), 4.01 3.77 ( d, J = 17.2 Hz, 1H), 3.20-2.78 (s, 12H), 2.15-1.65 (m, 15H), 1.45-1.15 (m, 28H), 1.08-0.75 m, 24H) | $[(M+H)^+]$: 937.5 | | | Produced in the same manner as in Compound 47 |
| 60 | δ (ppm) 5.55-5.20 (m, 7H), 4.98 (d, J = 17.2 Hz, 1H), 3.77 ( d, J = 17.8 Hz, 1H), 3.25-2.80 (s, 12H), 2.12-1.66 (m, 15H), 1.45-1.15 (m, 28H), 1.05-0.75 (-m, 24H) | $[(M+H)^+]$: 937.5 | | | Produced in the same manner as in Compound 47 |
| 61 | δ(ppm) 5.35-5.44 (m, 1H) ,5.28-5.35 (m, 1H,), 4.85 (7H, s), 3.18-3.39 (m, 8H), 3.01-3.10 (m, 1H), 1.77-1.89 (m, 1H), 1.45-1.56 (m, 2H), 1.28-1.42 (m, 2H), 0.98-1.07 (m, 1H), 0.93 (s, 1H) | $[(M+H)^+]$: 839.2 | | | Produced in the same manner as in Compound 16 |
| 62 | δ(ppm) 5.35-5.43 (m, 1H) , 5.25-5.35 (m, 1H), 4.85 (s, 11H), 3.22-3.39 (m, 16H), 2.99-3.12 (m, 4H), 1.71-1.96 (m, 4H), 1.44-1.60 (m, 6H), 1.37 (s, 4H), 1.25 (s, 1H), 0.97-1.06 (m, 3H), 0.93 (s, 4H) | $[(M+H)^+]$: 881.3 | | | Produced in the same manner as in Compound 16 |
| 63 | δ(ppm) 5.37-5.48 (m, 1H) ,5.34 (d, J=5.9 Hz, 1H), 4.85 (s, 10H), 3.31 (dt, J=1.7 & 3.2 Hz, 13H), 3.17 (s, 1H), 3.02-3.12 (m, 2H), 1.83 (d, J=7.1 Hz, 2H), 1.42-1.66 (m, 4H), 1.37 (d, J=2.4 Hz, 3H), 0.86-0.98 (m, 2H) | $[(M+H)^+]$: 823 | | | Produced in the same manner as in Compound 16 |

| | | | | | |
|---|---|---|---|---|---|
| 64 | δ(ppm) 5.35-5.46 (m, 1H) ,5.27-5.35 (m, 1H), 4.85 (s, 3H), 3.31 (dt, J=1.7 & 3.2 Hz, 4H), 2.97-3.13 (m, 1H), 1.74-1.89 (m, 1H), 1.49 (dt, J=2.0 & 7.3 Hz, 1H), 1.36 (s, 1H), 0.99 (d, J=6.6 Hz, 1H), 0.89-0.95 (m, 1H) | [(M+H)$^+$]: 873.9 | | | Produced in the same manner as in Compound 16 |
| 65 | δ(ppm) 5.36-5.48 (m, 1H), 5.26-5.36 (m, 1H), 4.84 (s, 7H), 3.31 (dt, J=1.4 & 2.7 Hz, 11H), 2.98-3.17 (m, 1H), 1.72-1.94 (m, 1H), 1.42-1.58 (m, 2H), 1.30-1.42 (m, 1H), 0.81-1.01 (m, 1H) | [(M+H)$^+$]: 837.2 | | | Produced in the same manner as in Compound 16 |
| 66 | δ (ppm) 5.60-5.25 (m, 7H), 4.98 (d, J = 17.8 Hz, 1H), 3.76 (d, J = 17.7 Hz, 1H), 3.25-2.75 (s, 12H), 2.15-1.62 (m, 14H), 1.48-1.15 (m, 28H), 1.10-0.75 (m, 24H) | [(M+H)$^+$]: 937.4 | | | Produced in the same manner as in Compound 47 |
| 67 | δ (ppm) 5.55-5.25 (m, 7H), 4.98 (d, J = 17.7 Hz, 1H), 3.76 (d, J = 17.8 Hz, 1H), 3.25-2.75 (s, 12H), 2.18-1.75 (m, 14H), 1.45-1.15 (m, 30H), 1.10-0.75 (m, 21H) | [(M+H)$^+$]: 923.4 | | | Produced in the same manner as in Compound 16 |
| 68 | δ(ppm) 5.47 (m, 1H), 5.41 (m, 3H), 5.37 (m, 4H), 3.20-2.70 (s, 12H), 2.15-1.85(m, 10H), 1.85-1.70 (m, 6H), 1.60-1.15 (m, 30H), 1.10-0.80 (m, 30H) | [(M+Na)$^+$]: 1015.7 | | | Produced in the same manner as in Compound 16 |
| 69 | δ(ppm) 5.52 (m, 1H), 5.41-5.30 (m, 7H), 3.20-2.60 (s, 12H), 2.15-2.00(m, 16H), 2.00-1.60 (m, 11H), 1.60-1.45 (m, 14H), 1.45-1.10 (m, 14H), 1.10-0.80 (m, 23H) | [(M+Na)$^+$]: 1041.7 | | | Produced in the same manner as in Compound 16 |

It was confirmed that compound 35, compound 38, compound 42, compound 45, and compound 50 were produced in the same manner as in compound 47.

[Preparation Examples]

Preparation Example 1 [Granules]

| | | |
|---|---|---|
| Compound 47 | 5% | by weight |
| Bentonite | 40% | by weight |
| Talc | 10% | by weight |

(continued)

| | | |
|---|---|---|
| Clay | 43% | by weight |
| Calcium lignin sulfonate | 2% | by weight |

The above ingredients were homogeneously ground and mixed together, water was added thereto, and the mixture was kneaded. The kneaded product was granulated and dried to give granules.

Preparation Example 2 [Wettable powder]

| | | |
|---|---|---|
| Compound 36 | 30% | by weight |
| Clay | 50% | by weight |
| White carbon | 2% | by weight |
| Diatomaceous earth | 13% | by weight |
| Calcium lignin sulfonate | 4% | by weight |
| Sodium lauryl sulfate | 1% | by weight |

The above ingredients were homogeneously were mixed together, and the mixture was ground to give a wettable powder.

Preparation Example 3 [Granular wettable powder]

| | | |
|---|---|---|
| Compound 47 | 30% | by weight |
| Clay | 60% | by weight |
| Dextrin | 5% | by weight |
| Alkyl maletic acid copolym er | 4% | by weight |
| Sodium lauryl sulfate | 1% | by weight |

The above ingredients were homogeneously ground and mixed together, water was added thereto, and the mixture was kneaded. The kneaded product was granulated and dried to give a granular wettable powder.

Preparation Example 4 [Floables]

| | | |
|---|---|---|
| Compound 33 | 25% | by weight |
| POE polystyryl phenyl ether sulfate | 5% | by weight |
| Propylene glycol | 6% | by weight |
| bentonite | 1% | by weight |
| 1% aqueous solution of xanthan gum | 3% | by weight |
| PRONAL EX-300 (Toho Chemical Industry Co., Ltd.) | 0.05% | by weight |
| ADDAC 827(K.I. Chemical Industry Co., Ltd.) | 0.02% | by weight |
| Water | Added to total weight of 100% | by weight |

All the above ingredients except for the 1% aqueous solution of xanthan gum and a suitable amount of water were premixed and ground with a wet grinding mill. Thereafter, the 1% aqueous solution of xanthan gum and the remaining water were added to bring the total weight to 100% by weight and thus to give floables.

Preparation Example 5 [Emulsion]

| | |
|---|---|
| Compound 47 | 15% by weight |
| N,N-dimethylformamide | 20% by weight |
| Solvesso 150 (Exxon Mobil) | 55% by weight |
| Polyoxyethylene alkyl aryl ether | 10% by weight |

The above ingredients were homogeneously mixed with and dissolved in each other to give an emulsion.

Preparation Example 6 [Dust]

| | | |
|---|---|---|
| Compound 33 | 2% | by weight |
| Clay | 60% | by weight |
| Talc | 37% | by weight |
| Calcium stearate | 1% | by weight |

The above ingredients were homogeneously mixed together to give dust.

Preparation Example 7 [PL dust]

| | | |
|---|---|---|
| Compound 41 | 2% | by weight |
| DL clay | 94.5% | by weight |
| White carbon | 2% | by weight |
| Calcium stearate | 1% | by weight |
| Light liquid paraffin | 0.5% | by weight |

The above ingredients were homogeneously mixed together to give DL dust.

Preparation Example 8 [Micro granules F]

| | | |
|---|---|---|
| Compound 40 | 2% | by weight |
| Carrier | 94% | by weight |
| White carbon | 2% | by weight |
| Hisol SAS-296 | 2% | by weight |

The above ingredients were homogeneously mixed together to give micro granules F.

Preparation Example 9 [Liquefied drops]

| | | |
|---|---|---|
| Compound 27 | 10% | by weight |
| Benzyl alcohol | 74.9% | by weight |
| Propylene carbonate | 15% | by weight |
| BHT | 0.1% | by weight |

The above ingredients were homogeneously stirred and dissolved in each other to give liquefied drops.

Preparation Example 10 [Liquefied drops]

| | | |
|---|---|---|
| Compound 27 | 48% | by weight |
| Ethanol | 52% | by weight |

The above ingredients were homogeneously mixed together to give liquefied drops.

[Test Examples]

Test Example 1: Pesticidal effect against Plutella xylostella

A leaf disk having a diameter of 5.0 cm was cut out from a cabbage grown in a pot. A chemical solution adjusted to a 50% aqueous acetone solution (0.05% Tween 20 added) containing a compound of the present invention at a predetermined concentration was applied to the leaf disk. The leaf disk was then air-dried. Five larvae at the second instar born of Plutella xylostella were released, followed by standing in a thermostatic chamber (light period 16 hr - dark period 8 hr) (25°C). Three days after the start of standing, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As a result, spreading treatment of 100 ppm showed a high insecticidal activity of not less than 80% in terms of death rate for compounds 2, 3, 32, 34, 40, and 47.

Test Example 2: Pesticidal effect against Spodoptera litura

A leaf disk having a diameter of 5.0 cm was cut out from a cabbage grown in a pot. A chemical solution adjusted to a 50% aqueous acetone solution (0.05% Tween 20 added) containing a compound of the present invention at a predetermined concentration was applied to the leaf disk. The leaf disk was then air-dried. Five larvae at the third instar born of Spodoptera litura were released, followed by standing in a thermostatic chamber (light period 16 hr - dark period 8 hr) (25°C). Three days after the start of standing, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As a result, spreading treatment of 500 ppm showed a high insecticidal activity of not less than 80% in terms of death rate for compound 40.

Test Example 3: Pesticidal effect against Aphis gossypii

A leaf disk having a diameter of 2.0 cm was cut out from a cucumber grown in a pot. A chemical solution adjusted to a 50% aqueous acetone solution (0.05% Tween 20 added) containing a compound of the present invention at a predetermined concentration was applied to the leaf disk. The leaf disk was then air-dried. Ten larvae at the first instar born of Aphis gossypii were released, followed by standing in a thermostatic chamber (light period 16 hr - dark period 8 hr) (25°C). Three days after the start of standing, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As a result, foliage treatment of 20 ppm showed a high insecticidal activity of not less than 80% in terms of death rate for compounds 1, 3, 4, 5, 6, 7, 8, 9, 10, 11, 12, 13, 14, 15, 16, 18, 19, 21, 22, 23, 24, 25, 27, 28, 29, 36, 41, 47, 53, 54, 55, 56, 59, 63, 65, 67, and 68.

Further, spreading treatment of 5 ppm showed a high insecticidal activity of not less than 80% in terms of death rate for compounds 2, 17, 20, 32, 33, 34, 36, 49, 51, 52, 53, 54, 57, 58, 60, 61, 62, 64, 66, and 67.

Further, spreading treatment of 0.313 ppm showed a high insecticidal activity of not less than 80% in terms of death rate for compounds 48.

Test Example 4: Pesticidal effect against Rhopalosiphum padi

The root of wheat 96 hr after seeding was dipped in a chemical solution adjusted to a 50% aqueous acetone solution containing a compound of the present invention at a predetermined concentration. The root was then air-dried. Ten larvae (for each test compound) at the second instar born of Rhopalosiphum padi were released, followed by standing in a thermostatic chamber (light period 16 hr - dark period 8 hr) (25°C). Three days after the start of standing, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As a result, dipping treatment of 100 ppm showed a high insecticidal activity of not less than 80% in terms of death rate for compounds 31, 36, 38, 39, 40, and 41.

Test Example 5: Pesticidal effect against Frankliniella occidentalis

A leaf disk having a diameter of 2.8 cm was cut out from a kidney bean grown in a pot. A chemical solution adjusted to a 50% aqueous acetone solution (0.05% Tween 20 added) containing a compound of the present invention at a predetermined concentration was applied to the leaf disk. The leaf disk was then air-dried. Ten larvae at the first instar born of Frankliniella occidentalis were released, followed by standing in a thermostatic chamber (light period 16 hr - dark period 8 hr) (25°C). Six days after the start of standing, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As a result, foliage treatment of 100 ppm showed a high insecticidal activity of not less than 80% in terms of death rate for compounds 1, 2, 3, 6, 7, 8, 10, 11, 12, 13, 14, 15, 16, 19, 21, 24, 25, 27, 28, 35, 36, 38, 45, 47, and 66.

Spreading treatment of 5 ppm showed a high insecticidal activity of not less than 80% in terms of death rate for compounds 1, 2, 3, 11, 12, and 15.

Test Example 6: Pesticidal effect against Trialeurodes vaporariorum

Adult of Trialeurodes vaporariorum was released over cucumber grown in a pot, followed by laying for two days. After the completion of laying, a leaf disk having a diameter of 2.0 cm was cut out from the cucumber. After the confirmation of laying, a chemical solution adjusted to a 50% aqueous acetone solution (0.05% Tween 20 added) containing a compound of the present invention at a predetermined concentration was spread over the leaf disk. After the spreading, the leaf disk was allowed to stand in a thermostatic chamber (light period 16 hr - dark period 8 hr) (25°C). Fourteen days after the start of standing, the larvae were observed for survival or death, and the death rate of larvae was calculated by the following equation. The test was duplicated.

$$\text{Death rate (\%)} = \{(\text{number of laid eggs} - \text{number of survived larvae})/\text{number of laid eggs}\} \times 100$$

As a result, spreading treatment of 100 ppm showed a high insecticidal activity of not less than 80% in terms of death rate for compounds 1, 3, 5, 6, 7, 8, 9, 10, 15, 16, 18, 19, 21, 22, 23, 24, 25, 26, 28, 30, 57, 58, 66, 67, and 68.
Foliage treatment of 20 ppm showed a high insecticidal activity of not less than 80% in terms of death rate for compounds 36, 37, 38, 39, 41, and 45.
Foliage treatment of 10 ppm showed a high insecticidal activity of not less than 80% in terms of death rate for compounds 1, 2, 3, 7, 8, 10, 11, 12, 13, 15, 16, 17, 18, 19, 20, 21, 22, 23, 24, 28, 29, 30, 32, 34, 37, 38, 39, 41, 45, 47, 49, 52, 55, 56, 57, 58, 66, and 67.

Test Example 7: Pesticidal effect against Trigonotylus caelestialium

Foliage of seedling of wheat four days after seeding was dipped in a chemical solution adjusted to a 50% aqueous acetone solution (0.05% Tween 20 added) containing a compound of the present invention at a predetermined concentration. The foliage was then air-dried. The dried foliage was placed in a glass tube, and two larvae at the second instar born of Trigonotylus caelestialium were released in the same glass tube. After the release of Trigonotylus caelestialium, a lid is put on the tube, followed by standing in a thermostatic chamber (25°C). During the test, for the supply of water to the wheat, water was allowed to be absorbed into wheat from below the glass tube. Four days after the treatment, the larvae were observed for survival or death, and the death rate of larvae was calculated by the following equation.

$$\text{Death rate (\%)} = \{\text{number of dead larvae}/(\text{number of survived larvae} + \text{number of dead larvae})\} \times 100$$

As a result, dipping treatment of 50 ppm showed a high insecticidal activity of not less than 80% in terms of death rate for compounds 2, 7, 12, 16, 18, 20, 21, 25, 27, 28, 32, 33, 34, 36, 47, 52, 57, 58, 66, and 67.

Test Example 8: Miticidal effect against Tetranychus cinnabarinus

Small pieces of kidney bean leaves were inoculated with four adult female of Tetranychus cinnabarinus, followed by laying in a thermostatic chamber (25°C). After the elapse of 24 hr, female adults were removed from the small species, and the small pieces were dipped in a chemical solution adjusted to a 50% aqueous acetone solution (0.05% Tween 20 added) containing a compound of the present invention at a predetermined concentration. The small species were then air-dried, was then placed on agar, and was allowed to stand in a thermostatic chamber (light period 16 hr - dark period 8 hr) (25°C). Six days after the start of the chemical solution treatment, the number of remaining eggs and the number of dead insects were counted, and ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, dipping treatment of 100 ppm showed a high insecticidal activity of not less than 80% in terms of ovicidal/miticidal rate (%) for compounds 41, 45, and 53.

<Miticidal effect against two-spotted spider mite (susceptible strain)>

Test Example 9: Miticidal effect against adult of Tetranychus uruticae

A leaf disk having a diameter of 2.8 cm was cut out from a kidney bean grown in a pot. The leaf disk was inoculated with ten adult of Tetranychus uruticae. Foliage was treated with a chemical solution adjusted to a 50% aqueous acetone solution (0.05% Tween 20 added) containing a compound of the present invention at a predetermined concentration. The leaf disk was then air-dried and was allowed to stand in a thermostatic chamber (light period 16 hr - dark period 8 hr) (25°C). Four days after the chemical solution treatment, the number of dead mites was counted, and the miticidal rate was calculated according to the following equation.

$$\text{Miticidal rate (\%)} = \{\text{number of dead mites}/(\text{number of survived mites} + \text{number of dead mites})\} \times 100$$

As a result, foliage treatment of 10 ppm showed a high miticidal activity of not less than 80% in terms of miticidal rate (%) for compounds 53, 57, 58, 59, 60, 64, 67, 68, and 69. Foliage treatment of 1.35 ppm showed a high miticidal activity of not less than 80% in terms of miticidal rate (%).

Test Example 10: Ovicidal effect against egg of Tetranychus uruticae

A leaf disk having a diameter of 2.0 cm was cut out from a kidney bean grown in a pot. The leaf disk was inoculated with seven female adult of Tetranychus uruticae, followed by laying in a thermostatic chamber (25°C). After the elapse of 24 hr, female adults were removed from the leaf disk. The foliage was treated with a chemical solution adjusted to a 50% aqueous acetone solution (0.05% Tween 20 added) containing a compound of the present invention at a predetermined concentration. The foliage was then air-dried and was allowed to stand in a thermostatic chamber (light period 16 hr - dark period 8 hr) (25°C). Seven days after the chemical solution treatment, the number of remaining eggs and the number of dead mites were counted, and the ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, dipping treatment of 10 ppm showed a high insecticidal activity of not less than 80% in terms of ovicidal/miticidal rate (%) for compounds 52, 53, 55, 56, 57, 58, 59, 60, 68, and 69.

<Miticidal effect against two-spotted spider mite (drug-resistant strain)>

Test Example 11: Miticidal effect against adult of drug-resistant Tetranychus uruticae

A leaf disk having a diameter of 2.8 cm was cut out from a kidney bean grown in a pot. The leaf disk was inoculated with ten adult of drug-resistant Tetranychus uruticae. Foliage was treated with a chemical solution adjusted to a 50% aqueous acetone solution (0.05% Tween 20 added) containing a compound of the present invention at a predetermined concentration. The leaf disk was then air-dried and was allowed to stand in a thermostatic chamber (light period 16 hr - dark period 8 hr) (25°C). Four days after the chemical solution treatment, the number of dead mites was counted, and the miticidal rate was calculated according to the following equation.

$$\text{Miticidal rate (\%)} = \{\text{number of dead mites}/(\text{number of survived mites} + \text{number of dead mites})\} \times 100$$

As a result, foliage treatment of 10 ppm showed a high miticidal activity of not less than 80% in terms of miticidal rate (%) for compounds 53, 55 and 56. Foliage treatment of 3.5 ppm showed a high miticidal activity of not less than 80% in terms of miticidal rate (%) for comounds58 and 59.

Test Example 12: Ovicidal effect against egg of drug-resistant Tetranychus uruticae

A leaf disk having a diameter of 2.0 cm was cut out from a kidney bean grown in a pot. The leaf disk was inoculated with seven female adult of drug-resistant Tetranychus uruticae, followed by laying in a thermostatic chamber (25°C). After the elapse of 24 hr, female adults were removed from the leaf disk. The foliage was treated with a chemical solution adjusted to a 50% aqueous acetone solution (0.05% Tween 20 added) containing a compound of the present

invention at a predetermined concentration. The foliage was then air-dried and was allowed to stand in a thermostatic chamber (light period 16 hr - dark period 8 hr) (25°C). Seven days after the chemical solution treatment, the number of remaining eggs and the number of dead mites were counted, and the ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, foliage treatment of 10 ppm showed a high insecticidal activity of not less than 80% in terms of ovicidal/miticidal rate (%) for compounds 55 and 56. Further, foliage treatment of 2.5 ppm showed a high insecticidal activity of not less than 80% in terms of ovicidal/miticidal rate (%) for compounds 58 and 59.

As a result of a test on foliage treatment of 10 ppm of acequinocyl that is a control agent, it was found that the ovicial/miticidal rate against egg of Tetranychus uruticae (susceptible strain) was 71% while the ovicial/miticidal rate against egg of Tetranychus uruticae (drug-resistant strain) was 11%.

From test results of Test Example 12, it was found that the compounds 53, 55, 56, 58, and 59 had a high insecticidal effect also against a strain having a lowered susceptibility to acequinocyl.

<Insecticidal effect against Tetranychus kanzawai >

Test Example 13: Miticidal effect against adult of Tetranychus kanzawai

A leaf disk having a diameter of 2.8 cm was cut out from a kidney bean grown in a pot. The leaf disk was inoculated with ten adults of Tetranychus kanzawai. Foliage was treated with a chemical solution adjusted to a 50% aqueous acetone solution (0.05% Tween 20 added) containing a compound of the present invention at a predetermined concentration. The leaf disk was then air-dried and was allowed to stand in a thermostatic chamber (light period 16 hr - dark period 8 hr) (25°C). Four days after the chemical solution treatment, the number of dead mites was counted, and the death rate was calculated according to the following equation.

$$\text{Death rate (\%)} = \{\text{number of dead mites}/(\text{number of survived mites} + \text{number of dead mites})\} \times 100$$

As a result, foliage treatment of 2.5 ppm showed a high miticidal activity of not less than 80% in terms of death rate (%) for compounds 53, 55, 58, 59, and 68.

Test Example 14: Ovicidal effect against egg of Tetranychus kanzawai

A leaf disk having a diameter of 2.0 cm was cut out from a kidney bean grown in a pot. The leaf disk was inoculated with seven female adults of Tetranychus kanzawai, followed by laying in a thermostatic chamber (25°C). After the elapse of 24 hr, female adults were removed from the leaf disk. The foliage was treated with a chemical solution adjusted to a 50% aqueous acetone solution (0.05% Tween 20 added) containing a compound of the present invention at a predetermined concentration. The foliage was then air-dried and was allowed to stand in a thermostatic chamber (light period 16 hr - dark period 8 hr) (25°C). Seven days after the chemical solution treatment, the number of remaining eggs and the number of dead mites were counted, and the ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, foliage treatment of 2.5 ppm showed a high insecticidal activity of not less than 80% in terms of ovicidal/miticidal rate (%) for compounds 53, 55, 56, 58, 59, and 68.

<Miticidal effect against Panonychus citri (strain harvested in Aichi prefecture)>

Test Example 15: Miticidal effect against adults of Panonychus citri harvested in Aichi prefecture

A leaf disk having a diameter of 2.8 cm was cut out from Citrus iyo grown in a pot. The leaf disk was inoculated with seven adults of Panonychus citri. Foliage was treated with a chemical solution adjusted to a 50% aqueous acetone solution (0.05% Tween 20 added) containing a compound of the present invention at a predetermined concentration. The leaf disk was then air-dried and was allowed to stand in a thermostatic chamber (light period 16 hr - dark period 8 hr) (25°C). Four days after the chemical solution treatment, the number of dead mites was counted, and the dath rate was calculated according to the following equation.

$$\text{Death rate (\%)} = \{\text{number of dead mites}/(\text{number of survived mites} + \text{number of dead mites})\} \times 100$$

As a result, foliage treatment of 10 ppm showed a high miticidal activity of not less than 80% in terms of death rate (%) for compounds 53 and 55.

Test Example 16: Ovicidal effect against egg of Panonychus citri harvested in Aichi prefecture

A leaf disk having a diameter of 2.0 cm was cut out from Citrus iyo grown in a pot. The leaf disk was inoculated with seven female adults of Panonychus citri, followed by laying in a thermostatic chamber (25°C). After the elapse of 24 hr, female adults were removed from the leaf disk. The foliage was treated with a chemical solution adjusted to a 50% aqueous acetone solution (0.05% Tween 20 added) containing a compound of the present invention at a predetermined concentration. The foliage was then air-dried and was allowed to stand in a thermostatic chamber (light period 16 hr - dark period 8 hr) (25°C). Eleven days after the chemical solution treatment, the number of remaining eggs and the number of dead mites were counted, and the ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, foliage treatment of 10 ppm showed a high miticidal activity of not less than 80% in terms of ovicidal/miticidal rate (%) for compounds 53 and 55.

<Miticidal activity against Panonychus citri (strain of Saga prefercture)>

Test Example 17: Ovicidal effect against Panonychus citri harvested in Saga prefecture

A leaf disk having a diameter of 2.0 cm was cut out from Citrus iyo grown in a pot. The leaf disk was inoculated with seven female adults of Panonychus citri, followed by laying in a thermostatic chamber (25°C). After the elapse of 24 hr, female adults were removed from the leaf disk. The foliage was treated with a chemical solution adjusted to a 50% aqueous acetone solution (0.05% Tween 20 added) containing a compound of the present invention at a predetermined concentration. The foliage was then air-dried and was allowed to stand in a thermostatic chamber (light period 16 hr - dark period 8 hr) (25°C). Eleven days after the chemical solution treatment, the number of remaining eggs and the number of dead mites were counted, and the ovicidal/miticidal rate was calculated by the following equation.

$$\text{Ovicidal/miticidal rate (\%)} = \{(\text{number of remaining eggs} + \text{number of dead mites})/(\text{number of remaining eggs} + \text{number of dead mites} + \text{number of survived insects})\} \times 100$$

As a result, foliage treatment of 12.5 ppm showed a high miticidal activity of not less than 80% in terms of ovicidal/miticidal rate (%) for compounds 58 and 68. Foliage treatment of 2.5 ppm showed a high miticidal activity of not

less than 80% in terms of ovicidal/miticidal rate (%) for compounds 55 and 59.

Test Example 18: Miticidal effect against Aculops lycopersici

Foliage of tomato that had previously been parasitized by Aculops lycopersici and grown in a pot was sprayed with a chemical solution adjusted to a 50% aqueous acetone solution (0.05% Tween 20 added) containing a compound of the present invention at a predetermined concentration. The foliage was then air-dried and was allowed to stand in a thermostatic chamber (light period 16 hr - dark period 8 hr) (25°C). Three days after spreading, one foliole was cut out, followed by observation under a microscope for survival or death of Aculops lycopersici. The death rate was calculated by the following equation.

$$\text{Death rate (\%)} = \{\text{number of dead insects}/(\text{number of survived insects} + \text{number of dead insects}\} \times 100$$

As a result, spreading treatment of 10 ppm showed a high miticidal activity of not less than 80% in terms of death rate for compounds 1, 3, 6, 11, 12, 15, 19, 32, 33, 34, 36, 41, 47, and 49.

Test Example 19: Control effect against Musca domestica

A chemical solution of the compound according to the present invention that had been adjusted with a 40% sucrose solution to a concentration of 50 ppm was adsorbed on a swab, and the swab was placed in a vial. Two adults of Musca domestica grown in a room were released, followed by standing in a room at a room temperature of 25°C. Four days after the release, the insects were observed for survival or death. When two insects were in a dead or anguished state, the test compound was determined to be effective.

As a result, the compounds 1, 4, 7, 8, 9, 11, 12, 13, 14, 15, 16, 17, 21, 22, 23, 25, 27, 28, 29, 32, 33, 47, 58, 59, and 60 had such a high insecticidal activity that the two insects were brought to a dead or anguished state.

Test Example 20: Control effect against larvae of Musca domestica

A compound of the present invention was applied to a very small amount of DMSO, followed by dissolution in deionized water to prepare a chemical solution. The chemical solution (2 ml) adjusted to a concentration of 12.5 ppm was added to and well mixed with 2 g of a powder prepared by mixing wheat bran, MF feed (ORIENTAL YEAST Co., Ltd.), and dry yeast at a wheat bran : MF feed : dry yeast of 25 : 5 : 1 to prepare a feed for larvae of Musca domestica. The feed containing the compound was lightly packed in a 3-ml vial, and eight eggs were released. The mouth of the vial was closed with a meshed lid, and the vial was left to stand at 25°C. The number of larvae was counted five days after the chemical treatment, and the death rate was calculated by the following equation. The test was carried out by replicating each treatment twice.

$$\text{Death rate (\%)} = \{\text{number of dead larvae}/(\text{number of survived larvae} + \text{number of dead larvae})\} \times 100$$

As a result, treatment of 12.5 ppm showed a death rate of 100% for compounds 57 and 58.

Test Example 21: Tickicidal effect against Haemaphysalis longicornis

A capsule having a diameter of 2 cm and a height of 2 cm was bonded to a surface of a back surface of a mouse. A compound of the present invention (9.5 $\mu$g) was dissolved in ethanol, and the solution was dropped on the surface of the body of the mouth within the capsule. After thorough drying, ten nymphal ticks of Haemaphysalis longicornis were released, and the upper portion of the capsule was lidded for hermetical closing. The mouse was grown in a cage at 25°C for 12 hr under light phase conditions. Five days after the release, the capsule was removed, and survival or death of the nymphal ticks and the number of blood sucked individuals were measured, and the death/anguish rate was calculated by the following equation.

$$\text{Death/anguish rate (\%)} = \{\text{number of dead/anguished insects}/(\text{number of survived ticks} + \text{number of dead/anguished insects})\} \times 100$$

As a result, treatment of 9.5 $\mu$g showed an insecticidal activity of not less than 70% in terms of death/anguish rate for compound 27.

Test Example 22: Anthelminticidal effect against adult of Ascaridiidae galli (in-vitro test)

The activity of compounds was evaluated based on a change in motility of adults of A. galli. Compounds of the present invention were dissolved in dimethyl sulfoxide, and the solution was added to a culture solution of A. galli that had been dissolved in a Ringer's solution to a compound concentration of 50 ppm. Six adults of A. galli were placed per culture solution, followed by culturing at 41°C. After the elapse of 24 hr, the motility of the adults of A. galli was observed for the evaluation of the activity of the compounds.

As a result, compound 1, 2, 6, 7, 10, 19, 54, and 61 of the present invention had a high anthelminticidal activity of four insects that had stopped automatic motility.

Test Example 23: Anthelminticidal effect against mature larvae of Nippostrongylus brasiliensis (insect combating test in mice)

Compounds of the present invention were suspended in a 0.05% carboxymethylcellulose (CMC) solution to give a concentration of 50 mg/kg, and the suspension was orally administered once with a stomach tube to a mouse artificially infected with N. brasiliensis. Three days after the administration, the mouse was autopsied to count the number of N. brasiliensis parasitic in small intestine, and the anthelmintic rate (%) was calculated by the following equation using infected mice without medication as control. One adiminstration lot consisted of three mice, and the anthelmintic rate was expressed in terms of average value per one administration lot. When a mouse was dead during the test period, the anthelmintic rate was determined based on mice excluding the dead mouse.

$$\text{Anthelmintic rate (\%)} = \{(\text{number of parasites in control mice} - \text{number of parasites in medicated infected mice})/\text{number of parasites in control mice}\} \times 100$$

As a result, compounds 4, 5, 10, and 21 had a high anthelmintic activity of not less than 70%.

Compounds according to the present invention had an excellent control activity, for example, against Plutella xylostella, Spodoptera litura, Aphis gossypii, Rhopalosiphum padi, Frankliniella occidentalis, Trialeurodes vaporariorum, Trigonotylus caelestialium, Tetranychus uruticae, Tetranychus kanzawai, Panonychus citri, and Aculops lycopersici. Accordingly, it was found that compounds of the present invention can be utilized as agricultural and horticultural harmful organism control agents, particularly as control agents against Lepidopteran insect pests, Hemipteran insect pests, Thysanopteran insect pests, and Acarina insect pests.

In particular, compounds according to the present invention exerted control effect against both Tetranychidae of genus Tetranychus and genus Panonychus in Acarina insect pests, exertedhigh activity against many strains having different drug sensitivity and resistant strains of existing drugs, and, further, have been found to have control effect attained by treatment of adult insects and eggs. Thus, it was found that compounds of the present invention can be utilized as control agents especially against Acarina insect pests (particularly Tetranychidae or rust mites).

Further, compounds of the present invention were also found to have an excellent control activity against Musca domestica, Haemaphysalis longicornis, Ascaridiidae galli, and Nippostrongylus brasiliensis. Thus, compounds of the present invention were found to be utilizable as control agents against hygiene pests, offensive insect pests, stored grain insect pests, stored food pests, house insect pests, and zooparasitic pests.

## Claims

1. A compound represented by formula (1) or a stereoisomer thereof:

[Chemical formula 1]

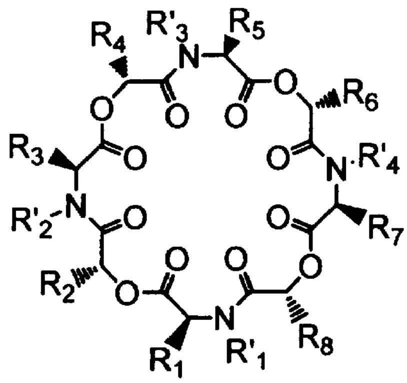

(1)

wherein $R_1$, $R_3$, $R_5$, and $R_7$ each represent a hydrogen atom or methyl, ethyl, n-propyl, isopropyl, n-butyl, and isobutyl, provided that $R_1$, $R_3$, and $R_5$ are the same as each other while $R_7$ is different from $R_1$, $R_3$, and $R_5$; $R_2$, $R_4$, $R_6$, and $R_8$ each represent straight-chain, branched, or cyclic alkyl having 4 to 9 carbon atoms, provided that $R_2$, $R_4$, and $R_6$ are the same as each other while $R_8$ may be the same as or different from $R_2$, $R_4$, and $R_6$; and $R'_1$, $R'_2$, $R'_3$, and $R'_4$ each represent a hydrogen atom, methyl or ethyl, provided that $R'_1$, $R'_2$, $R'_3$, and $R'_4$ are the same as each other.

**2.** An agricultural and horticultural harmful organism control agent, comprising at least one of compounds according to claim 1 or stereoisomers thereof.

**3.** A veterinary parasite control agent comprising at least one of compounds according to claim 1 or stereoisomers thereof.

**4.** A method for controlling an agricultural and horticultural harmful organism, the method comprising applying an effective amount of a compound according to claim 1 or a stereoisomer thereof to a harmful organism or a habitat thereof.

**5.** A method for controlling a veterinary parasite, the method comprising applying an effective amount of a compound according to claims 1 or a stereoisomer to a veterinary parasite or a habitat thereof.

**6.** Use of a compound according to claim 1 or a stereoisomer thereof, for producing an agricultural and horticultural harmful organism control agent.

**7.** Use of a compound according to claim 1 or a stereoisomer thereof, for producing a veterinary parasite control agent.

**8.** A compound according to claim 1 or a stereoisomer thereof, for use as an agricultural and horticultural harmful organism control agent.

**9.** A compound according to claim 1 or a stereoisomer thereof, for use as a veterinary parasite control agent.

**10.** A process for producing a compound represented by formula (1):

[Chemical formula 2]

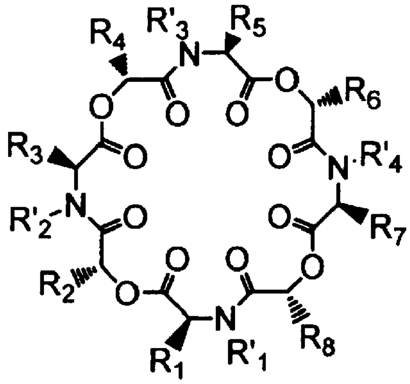

(1)

wherein $R_1$, $R_2$, $R_3$, $R_4$, $R_5$, $R_6$, $R_7$, $R_8$,$R'_1$, $R'_2$, $R'_3$, and $R'_4$ are as defined in claim 1, the process comprising: cyclizing a compound represented by formula (2):

[Chemical formula 3]

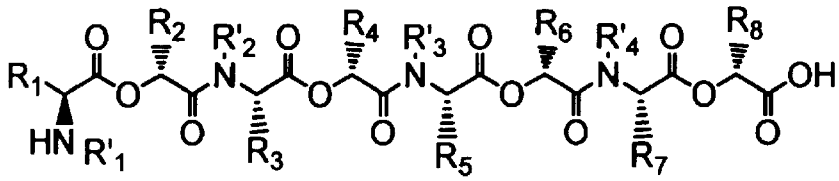

(2)

wherein $R_1$, $R_2$, $R_3$, $R_4$, $R_5$, $R_6$, $R_7$, $R_8$, $R'_1$, $R'_2$, $R'_3$, and $R'_4$ are as defined above.

**11.** The process according to claim 10, which further comprises obtaining the compound of formula (2) by removing group X and group Y of a compound represented by formula (3):

[Chemical formula 4]

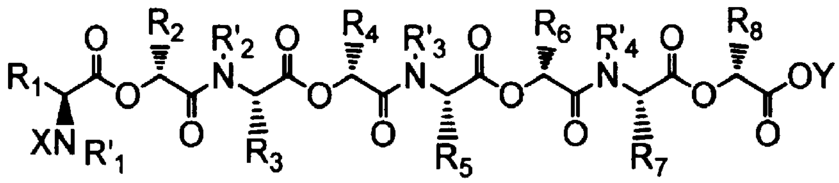

(3)

wherein $R_1$, $R_2$, $R_3$, $R_4$, $R_5$, $R_6$, $R_7$, $R_8$, $R'_1$, $R'_2$, $R'_3$, and $R'_4$ are as defined in claim 10; X represents an N-terminal protecting group selected from the group consisting of acetyl (Ac), allyloxycarbonyl (Alloc), benzyloxycarbonyl (CbZ), tert-butyloxycarbonyl (Boc), and 9-fluorenylmethyloxycarbonyl (Fmoc); and Y represents a C-terminal protecting group selected from the group consisting of benzyl (OBn), p-nitrobenzyl (ONb), and tert-butyl ($OBu^t$), or a support

of a polymer with a selectively removable fixing group.

**12.** The process according to claim 11, which further comprises obtaining a compound of formula (3) by reacting a compound represented by formula (4):

[Chemical formula 5]

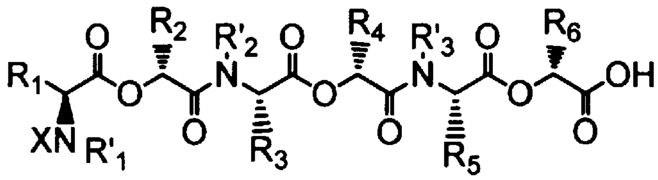

(4)

wherein $R_1$, $R_2$, $R_3$, $R_4$, $R_5$, $R_6$, $R'_1$, $R'_2$, $R'_3$, and X are as defined in claim 11, with a compound represented by formula (5):

[Chemical formula 6]

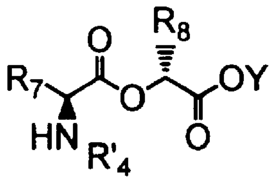

(5)

wherein $R_7$, $R_8$, $R'_4$, and Y are as defined in claim 11.

Europäisches Patentamt
European Patent Office
Office européen des brevets

# EUROPEAN SEARCH REPORT

Application Number
EP 18 19 4117

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TAICHI OHSHIRO ET AL: "New verticilides, inhibitors of acyl-CoA:cholesterol acyltransferase, produced by Verticillium sp. FKI-2679", THE JOURNAL OF ANTIBIOTICS, vol. 65, no. 5, 14 March 2012 (2012-03-14), pages 255-262, XP055214510, ISSN: 0021-8820, DOI: 10.1038/ja.2012.12 * vehicle A3; table 3 * ----- | 1-12 | INV. C07K11/02 A01N43/72 A01P5/00 A01P7/02 A01P7/04 C07C231/12 C07C235/12 C07C269/06 C07C271/22 C07D273/08 C07B51/00 C07B61/00 |
| Y | WO 2011/069995 A1 (BAYER ANIMAL HEALTH GMBH [DE]; HARDER ACHIM [DE]; KRIEGER KLEMENS [DE]) 16 June 2011 (2011-06-16) * compound XRB-C894; claims; table 1 * ----- | 1-12 | |
| Y | WO 2004/044214 A1 (KITASATO INST [JP]; OMURA SATOSHI [JP]; SHIOMI KAZURO [JP]; MASUMA ROK) 27 May 2004 (2004-05-27) * abstract * ----- | 1-12 | TECHNICAL FIELDS SEARCHED (IPC) |
| Y,P | WO 2012/077783 A1 (MEIJI SEIKA PHARMA CO LTD [JP]; SUZUKI EMIKO [JP]; YAMAGUCHI SYOICHI [) 14 June 2012 (2012-06-14) * the whole document * ----- | 1-12 | C07K |

1

The present search report has been drawn up for all claims

| Place of search | Date of completion of the search | Examiner |
|---|---|---|
| Munich | 18 October 2018 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 18 19 4117

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2018

| Patent document cited in search report | Publication date | Patent family member(s) | Publication date |
|---|---|---|---|
| WO 2011069995 A1 | 16-06-2011 | AR 079341 A1 | 18-01-2012 |
| | | AU 2010329998 A1 | 05-07-2012 |
| | | EP 2509967 A1 | 17-10-2012 |
| | | JP 5779787 B2 | 16-09-2015 |
| | | JP 2013513567 A | 22-04-2013 |
| | | TW 201138799 A | 16-11-2011 |
| | | US 2012302496 A1 | 29-11-2012 |
| | | UY 33095 A | 29-07-2011 |
| | | WO 2011069995 A1 | 16-06-2011 |
| WO 2004044214 A1 | 27-05-2004 | AT 419373 T | 15-01-2009 |
| | | AU 2002344494 A1 | 03-06-2004 |
| | | BR 0215937 A | 06-09-2005 |
| | | CA 2504309 A1 | 27-05-2004 |
| | | EP 1561820 A1 | 10-08-2005 |
| | | ES 2318053 T3 | 01-05-2009 |
| | | JP 4176077 B2 | 05-11-2008 |
| | | JP WO2004044214 A1 | 09-03-2006 |
| | | NZ 539986 A | 30-03-2007 |
| | | US 2006111280 A1 | 25-05-2006 |
| | | WO 2004044214 A1 | 27-05-2004 |
| WO 2012077783 A1 | 14-06-2012 | JP 2014037351 A | 27-02-2014 |
| | | TW 201306746 A | 16-02-2013 |
| | | WO 2012077783 A1 | 14-06-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012134304 A
- EP 0382173 A
- JP 5229997 A
- WO 9319053 A
- EP 0626376 A
- EP 0626375 A
- WO 2011069995 A
- WO 200444214 A
- JP 5271013 A


### Non-patent literature cited in the description


- *Tetrahedron Letters,* 1963, vol. 4 (6), 351
- *Tetrahedron Letters,* 1963, vol. 4 (14), 885
- *Tetrahedron Letters,* 1977, vol. 18 (25), 2167
- *Agricultural and Biological Chemistry,* 1978, vol. 42 (3), 629
- *Peptide Chemistry,* 1978, 165
- **NOBUO IZUMIYA ; TETSUO KATO ; HARUHIKO AOYANAGI ; MICHINORI WAKI.** Pepuchido Gosei No Kiso To Jikken (Basis and Experiments of Peptide Synthesis). Maruzen Co., Ltd, 1985
- **BREWSTER, P. et al.** *Nature,* 1950, vol. 166, 179-180
- **LERCHEN, H. G. et al.** *Tetrahedron Lett.,* 1985, vol. 26, 5257-5260
- **SOUICHI MONMA et al.** *Org. Lett.,* 2006, vol. 8 (24
- The Pesticide Manual. The British Crop Protection Council
- SHIBUYA INDEX. SHIBUYA INDEX RESEARCH GROUP, 2008